# EUROPEAN PATENT SPECIFICATION

(11) **EP 3 792 366 B1**
(45) Date of publication and mention of the grant of the patent: **30.09.2026**
(21) Application number: 20186222.4
(22) Date of filing: 03.04.2015
(51) Int. Cl.: C12Q 1/6827, C12Q 1/68

(54) **IMPROVED COMPOSITIONS AND METHODS FOR MOLECULAR INVERSION PROBE ASSAYS**
VERBESSERTE ZUSAMMENSETZUNGEN UND VERFAHREN FÜR MOLEKULARINVERSIONSPROBENASSAYS
COMPOSITIONS ET PROCÉDÉS AMÉLIORÉS POUR ANALYSES AU MOYEN DE SONDES D'INVERSION MOLÉCULAIRE

(30) Priority: 04.04.2014 US 201461975433 P
(43) Date of publication of application: 17.03.2021
(62) Document number(s) of the earlier application(s) in accordance with Art. 76 EPC: 15772997.1 / 3 126 524
(73) Proprietor: Affymetrix, Inc., Santa Clara, CA 95051 (US)
(72) Inventor: SHAPERO, Michael, H., Sunnyvale, CA 94085 (US); GREENFIELD, Lawrence, Santa Clara, CA 95054 (US); SAPOLSKY, Ronald, J., Mountain View, CA 94040 (US); JONES, Keith, Sunnyvale, CA 94087 (US); BELLON, Laurent, San Mateo, CA 94402 (US); LIPSHUTZ, Robert, J., Palo Alto, C 94301 (US); ESTABROOK, August, South San Francisco, CA 94080 (US); EFCAVITCH, John, San Carlos, CA 94070 (US)
(74) Representative: Thermo Fisher Scientific- Life Sciences Solutions Group

(56) References cited:
- US-A1- 2007 003 949
- US-A1- 2013 072 390
- HARDENBOL PAUL ET AL: "Multiplexed genotyping with sequence-tagged molecular inversion probes", NATURE BIOTECHNOLOGY, GALE GROUP INC., NEW YORK, US, vol. 21, no. 6, 1 June 2003 (2003-06-01), pages 673 - 678, XP002292164, ISSN: 1087-0156, DOI: 10.1038/NBT821
- MICHAEL S. AKHRAS ET AL: "PathogenMip Assay: A Multiplex Pathogen Detection Assay", PLOS ONE, vol. 2, no. 2, 21 February 2007 (2007-02-21), pages e223, XP055358376, DOI: 10.1371/journal.pone.0000223

## Description

### CROSS-REFERENCE TO RELATED APPLICATIONS

This application claims priority to and benefit of a prior U.S. Provisional Application number 61/973,433, Improved Compositions and Methods for Molecular Inversion Probes, by Michael Shapero, et al., filed April 4, 2014.

### FIELD OF THE INVENTION

The present inventions are in the field of molecular inversion probe (MIP) compositions and methods. The methods and compositions described herein enhance the efficiency and sensitivity of MIP assays. Methods include procedures for preventing and removing undesirable polymerase strand displacement over-extensions (flaps) that reduce MIP signal efficiency. The MIPs can be configured to facilitate combinations of techniques to enhance separation of hybridized and unhybridized probes, e.g., while providing unambiguous signals confirming the presence of targets of interest. Compositions can include MIPs adapted to the methods, e.g., including cleavage sites, informative tags, and regions adapted to next generation sequencing procedures.

### BACKGROUND OF THE INVENTION

Molecular inversion probes (MIPs) are, e.g., nucleic acid hybridization probes that hybridize to a target nucleic acid in a loop with the 5' and 3' ends abutting or separated in the target with a small gap. The MIPs are typically designed to interrogate a target nucleotide in the gap using the high specificity of the DNA polymerase reaction. If provided with the appropriate dNTP, the polymerase can fill the gap between the MIP 5' and 3' ends. For example, if the target nucleic acid has an adenine "A" in the gap, the polymerase can fill the gap if provided with a complementary dTTP. The polymerase will add a "T" and fill the gap in the so called gap-fill reaction. With the gap filled, a ligase can close the remaining nick and circularize the MIP. such MIPs are disclosed in e.g. US 2013/072390.

Because circularized single strand DNA is not a substrate for many nucleases, all other nucleic acids, including MIPs that did not hybridize and circularize, can be digested with a nuclease cocktail. However, some of these cocktails are not totally efficient and specific, so signal and background may be affected.

MIPs have been known to lose some efficiency when a polymerase writes through a gap during the gap-fill step and displaces the 5' end of the probe with an undesirable extension. With the MIP 5' end displaced, the hybridized MIP cannot act as a ligase substrate and is not circularized for detection. Thus, the strength of the assay signal is lost or reduced.

MIP reaction products are typically detected after an amplification step, such as PCR using primer binding sites within the MIPs or rolling circle amplification, on a capture array.

In view of the above, a need exists for MIP reactions not subject to polymerase strand displacement errors. It would be desirable to have additional options for separation of unreacted probe from circularized probe. Benefits could also be realized through additional means of detecting positive MIP reaction product signals. The present invention provides these and other features that will be apparent upon review of the following.

### SUMMARY OF THE INVENTION

The present disclosure teaches useful methods and compositions to enhance sensitivity, resolution, scope, and accuracy of molecular inversion probe (MIP) analyses. The methods and compositions are typically directed to techniques of avoiding polymerase strand displacement errors during MIP processing. Techniques can be used in complementary combinations with methods of removing unreacted probes and multiplexing MIP product detections.

The invention is set out in the appended set of claims. An MIP useful in the methods and compositions of the invention comprises: a first homology region (HR1) at a 5'end comprising a first sequence complementary to a target nucleic acid of interest, and a second homology region (HR2) at a 3'end comprising a second sequence complementary to the target nucleic acid of interest

In certain embodiments, the MIP can avoid the gap-fill reaction through provision of a stringently hybridizing oligomer insert probe to fill the gap between ends of the MIP. The oligomer probe can be designed to hybridize with the target in the gap with stringency adapted to not bind significantly in the presence of a mismatch. The MIP can be designed with the HR1 sequence at the 5' end and the HR2 sequence at the 3' end. The HR1 and HR2 complementary sequences on the target can be separated at least 5 bases from a target sequence complementary to HR2, e.g., with oligomer insert nucleic acid having a sequence at least 80% complementary to the at least 5 bases separating the HR1 and HR2 regions. A composition may comprise a molecular inversion probe (MIP) comprising a first homology region (HR1) sequence at a 5' end and a second homology region 2 (HR2) sequence at the 3' end, wherein the HR1 comprises a sequence complementary to a target nucleic acid and HR2 comprises a sequence complementary to the target nucleic acid, and wherein a target sequence complementary to HR1 is located 5' and separated at least 5 nucleotides from a target sequence complementary to HR2; and an oligomer insert nucleic acid having a sequence at least 80% complementary to the at least 5 nucleotides separating the HR1 and HR2 regions. In some embodiments, the oligomer insert is 100% complementary to a wild type sequence and the oligomer insert mismatches at least one nucleotide with a putative SNP of interest. In some embodiments, the oligomer insert ranges in length from 5 nucleotides to 15 nucleotides. In some embodiments, the oligomer insert mismatches with the target nucleic acid by from one to three nucleotides. In some embodiments, the MIP comprises additional segments from the group consisting of: a tag ID, a first cleavage site, a second cleavage site, a tag between HR1 or HR2 sequences, a forward (e.g. PCR) primer binding site, and a reverse (e.g. PCR) primer binding site.

The oligomer insert detection technique can be as follows. A MIP is provided comprising an HR1 sequence at a 5' end and a HR2 sequence at the 3' end. The oligomer insert nucleic acid is provided with a sequence complementary to the at least 5 bases separating the HR1 and HR2 regions along the target sequence of interest. The MIP and oligo are added to hybridize with sample nucleic acids. If the sample has the target nucleic acid comprising a sequence complementary to the HR1, and comprising a sequence complementary to the HR2, and wherein the sequence complementary to HR1 is located 5' and separated at least 5 bases from the target sequence complementary to HR2, the oligomer insert may hybridize in the gap between the MIP probe ends. The MIP and oligomer insert probe are hybridized to the putative target nucleic acid, which if present can align the MIP ends and oligomer so that there is no gap and the HR1 and HR2 ends each abut the oligomer, providing a ligase substrate at each end of the oligomer insert. The hybridized nucleic acids are contacted with a ligase and the MIP is circularized if the oligomer has hybridized to the target nucleic acid in the gap. The presence of the circularized MIP signals that the gap sequence was adequately complementary with the oligomer. The presence of the putative target sequence is confirmed if the MIP is determined to be ligated to the oligomer insert. In some embodiments, the method further comprises adapting or providing the oligomer insert to be 100% complementary to a wild type sequence, or adapting the oligomer insert to mismatch at least one nucleotide with a putative SNP of interest. In some embodiments, the oligomer insert ranges in length from 5 nucleotides to 15 nucleotides, and the oligomer insert mismatches with the putative target nucleic acid by from one to three nucleotides. In some embodiments, the method further comprises determining the MIP has not been ligated to the oligomer insert; thereby confirming the putative target sequence comprises at least one mismatch with the oligomer insert. In some embodiments, the method further comprises adopting a hybridization condition so that the oligomer insert does not hybridize to the target nucleic acid if there is a mismatch of at least one nucleotide. In some embodiments, determining ligation comprises cleavage of a circularized MIP between amplification (e.g. PCR) primer binding sites and amplification of the cleaved MIP by amplification (e.g. PCR). In some embodiments, the MIP comprises additional segments from the group consisting of: a tag ID, a first cleavage site, a second cleavage site, a forward (e.g. PCR) primer binding site, a tag between sequences of HR1 or HR2, and a reverse (e.g. PCR) primer binding site.

Disclosed but presently not claimed methods can avoid the problem of strand displacement by removing any over extension flap beyond the gap before attempting the ligation step. For example, a method of enhancing efficiency of MIP ligation efficiency can include: providing an MIP with a first homology region (HR1) at a 5' end and a second homology region (HR2) at a 3' end, wherein the HR1 and HR2 are configured to hybridize with complementary sequences on a target nucleic acid with the ends separated by a gap of one or more target sequence bases not hybridized to the MIP; contacting the HR1 region and HR2 region with a complementary target sequence under hybridizing conditions; adding one or more bases to the 3' end of the MIP by contacting the hybridized MIP/target with a polymerase and one or more nucleotide triphosphates (NTPs); and, chemically or enzymatically removing any of the bases beyond those required to fill the gap. For example, the NTPs added across the gap can be dNTPs, but NTPs for nucleotides that may be added beyond the gap can be specifically removable ribonucleotides. Such a method can include, e.g., enhancing MIP ligation efficiency by: providing an MIP comprising a first homology region (HR1) at a 5' end and a second homology region (HR2) at a 3' end, wherein the HR1 and HR2 are configured to hybridize with complementary sequences on a target nucleic acid with the ends separated by a gap of one or more (typically one) target sequence bases not hybridized to the MIP; contacting the HR1 region and HR2 region to a complementary target nucleic acid sequence under hybridizing conditions, effecting a gap-fill reaction (comprising a polymerase, the hybridized MIP, target, an interrogating deoxyribonucleotide triphosphate (dNTP) complementary to a putative target sequence base at a position of interest, and one or more ribonucleotide triphosphates (rNTPs)); contacting the gap-fill reaction product with a ribonuclease, thereby cleaving polymerase rNTP extensions 3' from any gap-fill incorporated dNTP and allowing hybridization of the 5' HR1 without interference from the rNTPs; and, contacting the gap-fill reaction product with a ligase, thereby circularizing the MIP probe if the target nucleic acid comprises the target sequence base at the position of interest. In some embodiments the gap is a single base gap. In some embodiments, the method further comprises adding one or more polymerase substrates from the group consisting of: a ribonucleotide, a mismatch base to a putative SNP, and a glycolase substrate. In some embodiments, the removing comprises a technique utilizing an enzyme or chemistry selected from the group consisting of: a endonuclease V, a 3' cutting ribonuclease, a 5' cutting ribonuclease, a celery mismatch endonuclease (CEL I), a glycosylase TDG, a glycosylase MutY, and AP endonuclease/lyase, a T4 endonuclease VII, a T7 endonuclease I, a deoxyinosine 3'-endonuclease, a mung bean nuclease, a resolvase, a flap endonucleases, a cleavase, a 3-methyladenine-DNA glycosylase, a thymine mismatch-DNA glycosylase, a uracil DNA glycosylase, a hypoxanthine DNA N-glycosylase, an endonuclease IV, an APE 1 AP endonuclease, an exonuclease III, an endonuclease IV (nfo), a 8-oxoguanine-DNA glycosylase, a 3-methyladenine-DNA glycosylase, DNA intercalator, a substrate for the hydroxylamine/permanganate/piperidine cleavage reaction, a substrate for the osmium tetroxide/piperidine cleavage reaction; and a thymine mismatch-DNA glycosylase. In some embodiments, the MIP comprises additional segments from the group consisting of: a tag ID, a first cleavage site, a second cleavage site, a forward (e.g. PCR) primer binding site, and a reverse (e.g. PCR) primer binding site. In some embodiments the polymerase enzyme is selected from the group consisting of: an RNA polymerase and poorly selective DNA polymerase. In some embodiments, a combination of rNTPs and dNTPs is selected from the group consisting of: dATP with rCTP, rUTP, or rGTP; dTTP with rATP, rCTP, or rGTP; dCTP with rATP, rUTP, or rGTP; and, dGTP with rATP, rCTP, or rUTP. In some embodiments, the gap is from 2 to 5 bases and each target nucleic acid in the gap has the same base.

Gap-fill reactions can avoid strand displacement by adding stop NTPs to the reaction so that only the NTP required to fill the gap can be added to the 3' end of the hybridized MIP. For example, a disclosed but presently not claimed method of enhancing efficiency of an MIP gap-fill reaction can include: providing an MIP nucleic acid with a first homology region (HR1) at a 5' end and a second homology region (HR2) at the 3' end; hybridizing the HR1 region and HR2 region to a complementary target sequence, wherein the HR1 and HR2 are hybridized to the target with the ends separated by a one base gap of target sequence not hybridized to the MIP; contacting the hybridized MIP/target with a polymerase and a modified nucleotide triphosphate (dNTP) that bears a polymerase inhibiting function at the C5 position (if the NTP is a pyrimidine) of the nucleobase or C7 position (if the NTP is a pyrimidine) of the nucleobase, thereby adding the modified nucleotide to the 3' end of the MIP; wherein the polymerase mediated gap-fill is limited to the incorporation only of the modified dNTP. The modified dNTP can have an unmodified 3' OH, allowing ligation to the 5' end of the MIP. In some embodiments, the modified NTP is selected from the group consisting of a pyrazolo [3,4-d] pyrimidines, a 5-methylcytosine (5-me-C), a 5-hydroxymethyl cytosine, a xanthine, a hypoxanthine, a 2-aminoadenine, a 6-methyl derivative of adenine, a 6-methyl derivative of guanine, a 2-propyl derivative of adenine and a 2-propyl derivative of guanine, a 2-thiouracil, a 2-thiothymine, a 2-thiocytosine, a 5-propynyl uracil, a 5-propynyl cytosine, a 6-azo uracil, a 6-azo cytosine, a 6-azo thymine, a 5-uracil (pseudouracil), a 4-thiouracil, an 8-halo adenine, an 8-amino adenine, an 8-thiol adenine, an 8-thioalkyl adenine, an 8-hydroxyl adenine, an 8-halo guanine, an 8-amino guanine, an 8-thiol guanine, an 8-thioalkyl guanine, an 8-hydroxyl guanine, a 5-halo uracil, a 5-trifuoromethyl uracil, a 5-halo cytosine, a 5-trifuoromethyl cytosine, a 7-methyl guanine, a 7-methyladenine, an 8-aza guanine, an 8-azaadenine, a deazaguanine, a 7-deazaguanine, a 3-deazaguanine, a deazaadenine a 7-deazaadenine, a 3-deazaadenine, a pyrazolo [3,4-d] pyrimidine, an imidazo [1,5-a] 1,3,5 triazinone NTP, a 9-deazapurine, an imidazo [4,5-d] pyrazine, a thiazolo [4,5 -d] pyrimidine, a pyrazin-2-one, a 1,2,4-triazine, pyridazine; and a 1,3,5 triazine NTP. In some embodiments, the gap is a single base gap. In some embodiments, the method further comprises ligating the 3' end of the modified NTP to the 5' end of the HR1. In some embodiments, the method further comprises processing a base of the added modified NTP to convert the base into a natural base. In some embodiments the MIP comprises additional segments from the group consisting of: a tag ID, a first cleavage site, a second cleavage site, a forward (e.g. PCR) primer binding site, and a reverse (e.g. PCR) primer binding site.

In disclosed but presently not claimed methods strand displacement errors can be avoided by using the specific substrate cutting activity of certain enzymes, chemical reactions, or photo-chemical reactions. The MIP can be configured to include an end sequence that only generates a cleaving or nicking enzyme substrate depending on the target nucleic acid sequence. For example, a method of enhancing efficiency of molecular inversion probe ligation can include: a) providing a MIP comprising a first homology region and a second homology region, wherein the second homology region comprises a cleavage substrate base and a removable nucleic acid region with at least a 1, 2, 3, or 4 nucleotide bases of a sequence (prepositioned "flap") not complementary to the target sequence; b) contacting the MIP with a target nucleic acid of interest, whereby the second homology region hybridizes to the target nucleic acid (e.g., but for the flap); c) contacting the hybridized MIP with an enzyme or chemical having an activity of cutting the MIP at the cleavage substrate base if the MIP is hybridized to the target, thus releasing the removable nucleic acid; and, d) hybridizing the first homology region to the target nucleic acid; whereby the first and second homology regions abut each other on the target nucleic acid without a gap. In the above method, the flap can be specifically cleaved from the homology region using an enzyme such as a flap cleavase. In some embodiments, the cleavage substrate base is selected from the group consisting of: a ribonucleotide, a mismatch base to a putative SNP, and a glycolase substrate. In some embodiments, the removable region comprises three or more bases not complementary to the target sequence of the HR1 region. In some embodiments, the enzyme or chemistry is selected from the group consisting of: a endonuclease V, a 3' cutting ribonuclease, a 5' cutting ribonuclease, a celery mismatch endonuclease (CEL I), a glycosylase TDG, a glycosylase MutY, and AP endonuclease/lyase, a T4 endonuclease VII, a T7 endonuclease I, a deoxyinosine 3'-endonuclease, a mung bean nuclease, a resolvase, a flap endonucleases, a cleavase, a 3-methyladenine-DNA glycosylase, a thymine mismatch-DNA glycosylase, a uracil DNA glycosylase, a hypoxanthine DNA N-glycosylase, an endonuclease IV, an APE 1 AP endonuclease, an exonuclease III, an endonuclease IV (nfo), a 8-oxoguanine-DNA glycosylase, a 3-methyladenine-DNA glycosylase, DNA intercalator, a substrate for the hydroxylamine/permanganate/piperidine cleavage reaction, a substrate for the osmium tetroxide/piperidine cleavage reaction; and a thymine mismatch-DNA glycosylase. In some embodiments, the cleavage substrate corresponds to a position of a putative SNP in the target nucleic acid. In some embodiments, the method further comprises ligating the abutted homology regions together. In some embodiments, the MIP comprises additional segments from the group consisting of: a tag ID, a first cleavage site, a second cleavage site, a forward (e.g. PCR) primer binding site, a segment with an attached affinity pair member, and a reverse (e.g. PCR) primer binding site.

Efficient removal of extraneous nucleic acids and unreacted MIPs from a completed MIP reaction can further enhance the signal to noise ratio during later detection steps. As an alternate to removal with a nuclease cocktail, affinity groups can be designed into MIPs in such a way that reacted probe is not captured for removal at later steps, while unreacted probes are captured. For example, a disclosed but presently not claimed method of separating an MIP that has hybridized to a target from MIP that has not hybridized to a target can include: a) providing a MIP comprising in order: a first homology region, a second homology region, and a first member of an affinity pair, wherein the second homology region comprises a cleavage substrate base and a removable nucleic acid region with at least 1, 2, 3, or 4 nucleotide bases of a same sequence as the first homology region; b) contacting the MIP with a target nucleic acid of interest, whereby the second homology region and removable nucleic acid region hybridize to the target nucleic acid; c) contacting the hybridized MIP with an enzyme or chemical having an activity of cutting the MIP at the cleavage substrate base if the MIP is hybridized to the target, thus releasing the removable nucleic acid and affinity pair member; and, d) capturing, by binding to a second member of the affinity pair, any MIP probe that is not cut in step c); thereby removing MIPs that were not hybridized at step b). In some embodiments, the removable nucleic acid region sequence of the same sequence as the first homology region ranges in length from 3 nucleotides to 15 nucleotides. In some embodiments, the affinity pairs are selected from the group consisting of: biotin/avidin, antibody/antigen, biotin/streptavidin, metal/chelator, ligand/receptor, nucleic acid and binding protein, and complementary nucleic acids. In some embodiments, the affinity pair is other than an affinity pair comprising a nucleic acid member. In some embodiments, pair members have an affinity Kd ranging from 10⁻⁵ M to 10⁻¹⁵ M for each other. In some embodiments, the cleavage substrate is selected from the group consisting of: a ribonucleotide, a flap endonuclease substrate, a mismatched nucleotide, and a cleavase substrate. In some embodiments, the removable region comprises three or more nucleotides. In some embodiments, the enzyme having an activity of cutting the MIP is selected from the group consisting of: an RNase, a flap endonuclease (FEN), a cleavase, endonuclease V, and ribonuclease H. In some embodiments, said capturing comprises capturing the first affinity pair member by the second affinity pair member on a solid support, bead, polymer, array, or chromatography media. In some embodiments, the method further comprises ligating the MIP hybridized to the target. In some embodiment, the MIP comprises additional segments from the group consisting of: a tag ID, a first cleavage site, a second cleavage site, a forward (e.g. PCR) primer binding site, and a reverse (e.g. PCR) primer binding site. A MIP, or composition comprising such a MIP, useful in the above technique can include in order a single stranded DNA homology region segment, linked to an RNA segment, linked to a first member of an affinity pair. In some embodiments, the MIP further comprises a second homology region in order before the first homology region. In some embodiments, the MIP further comprises a second DNA segment of the first homology region between the RNA segment and the first member of an affinity pair. In some embodiments, the second DNA segment comprises a four or more nucleotide sequence complementary to a same sequence as the second homology region. In some embodiments, the first member of the affinity pair is a member of a pair selected from the group consisting of: biotin/avidin, antibody/antigen, metal/chelator, ligand/receptor, nucleic acid and binding protein, and complementary nucleic acids. In some embodiments the MIP further comprises a segment selected from the group consisting of: a tag ID, a first cleavage site, a second cleavage site, a forward (e.g. PCR) primer binding site, and a reverse (e.g. PCR) primer binding site.

Alternately, the presently not claimed method of removing non-reacted MIPs can include: a) providing a MIP comprising in order: a first homology region, a second homology region, a non-hybridizing region not complementary to the target sequence, and a first member of an affinity pair; b) contacting the MIP with a target nucleic acid of interest, whereby the second homology region and first homology region hybridize to the target nucleic acid; c) contacting the hybridized MIP with a cleavase enzyme having an activity of excising single stranded nucleic acid originating at an abutment of homology regions hybridized to target without a gap, thereby releasing non-hybridizing region and affinity pair member if there is no gap between the first and second homology regions of the MIP; and, d) capturing, by binding to a second member of the affinity pair, any MIP probe that is not cut in step c); thereby removing MIPs that were not hybridized without a gap at step b). In some embodiments, the affinity pairs are selected from the group consisting of: biotin/avidin, antibody/antigen, metal/chelator, ligand/receptor, nucleic acid and binding protein, and complementary nucleic acids. In some embodiments, said capturing comprises capturing by the second affinity pair member on a solid support, bead, polymer, array, or chromatography media. In some embodiments, the MIP comprises additional segments from the group consisting of: a tag ID, a first cleavage site, a second cleavage site, a forward (e.g. PCR) primer binding site, and a reverse (e.g. PCR) primer binding site.

MIPs, or compositions including those MIPs, for the affinity removal of non-reacted probe can be configured, e.g., to include in order: a 5' first homology region, a second homology region, a non-hybridizing region, and a first member of an affinity pair; wherein the homology regions are adapted to hybridize to a target nucleic acid of interest with a gap or nicked sugar-phosphate backbone between them; and wherein the non-hybridizing region is adapted to not hybridize to the target nucleic acid. In some embodiments, the first affinity pair member is a member of an affinity pair selected from the group consisting of: biotin/avidin, antibody/antigen, biotin/streptavidin, metal/chelator, ligand/receptor, nucleic acid and binding protein, and complementary nucleic acids. In some embodiments, the MIP comprises additional nucleic acid regions selected from the group consisting of: a tag ID, a first cleavage site, a second cleavage site, a forward (e.g. PCR) primer binding site, and a reverse (e.g. PCR) primer binding site.

The above affinity member removal schemes can be especially effective in combination with flap cleavase interrogation technique described above. For example, the engineered flap at the end of the MIP homology region can include an affinity pair member so that if the nucleotide of interest exists at the position on the target the flap is specifically excised, abutting the MIP 3' and 5' ends for ligation and removing the affinity member.

In many MIP reaction processes, circularized product is eventually linearized by a cleavage step and/or a fragment is removed from the probe product in a cleavage step. Often Uracil N-Glycosylase (UGN) is used to cut between PCR primer binding sites on the circularized MIP, to create a proper PCR target. However, it can be useful to have alternate cleavage sites and associated enzymes, to enhance flexibility in cleavage timing and to avoid interference with downstream analysis methods. For example, an MIP can be configured to include a first homology region, a second homology region, and a cleavage site between the first and second homology regions (e.g., also often between a pair of PCR probe binding sites); wherein the cleavage site is subject to cleavage by an enzyme selected from the group consisting of: a DNA glycosylase with lyase activity, a DNA glycosylase without lyase activity, and an apurinic/apyrimidinic endonuclease. The enzyme may be other than a uracil-N-glycosidase (UNG). For example, the MIP can include a strategically located substrate for a 3-methyladenine-DNA glycosylase, a 3-methyladenine-DNA glycosylase II (AlkA), a 3-methyladenine-DNA glycosylase I, a 3-methyladenine-DNA glycosylase from mouse cells (Mouse MPG), a thymine mismatch-DNA glycosylase, a lymphoblast uracil DNA glycosylase, a hypoxanthine DNA N-glycosylase, a hypoxanthine-DNA glycosylase, an 8-oxoguanine-DNA glycosylase (OGG1), an endonuclease III (nth), a thymine glycol-DNA glycosylase, an endonuclease IV (nfo), an endonuclease V (deoxyinosine 3'-endonuclease) (nfi), an endonuclease VIII (nei), a formamidopyrimidine DNA glycosylase (Fpg) (mutM), a MutY (micA), a K142A mutant of Mut Y, a thymine hydrate DNA glycosylase (Escherichia coli), a pyrimidine dimer DNA glycosylase (M. luteus), an 8-hydroxyquanine endonuclease, a yeast endonuclease three-like glycosylase (NTG1) (yOgg2), a human endonuclease III (hNTH1), an APE 1 AP endonuclease, and an exonuclease III. The MIP may comprise additional segments from the group consisting of: a tag ID, a second cleavage site, a forward PCR primer binding site, a tag within an HR sequence, and a reverse PCR primer binding site. The first homology region may comprise a feature selected from the group consisting of: one or more ribonucleotides, a mismatch base, a cleavage substrate, a length longer than a second homology region (HR2) at the opposite end of the MIP, an attached first member of an affinity pair, and a flap with a sequence different from a sequence of the HR2. In light of these alternate cleavage schemes, the method of preparing a circularized probe for (e.g., PCR) amplification can include providing a circularized MIP comprising a cleavage site between a first (e.g., PCR) primer binding site and a second (e.g., PCR) primer binding site; wherein the cleavage site is other than a uracil-N-glycosidase (UNG) substrate; cleaving the circularized MIP at the cleavage site; and amplifying the cleaved MIP (e.g., in a PCR reaction). The amplification may substitute dUTP for dTTP and begin with a UNG treatment. In some embodiments, the MIP comprises additional segments from the group consisting of: a tag ID, a second cleavage site, a forward (e.g. PCR) primer binding site, and a reverse (e.g. PCR) primer binding site. The MIP may comprise a first homology region (HR1) comprising a feature selected from the group consisting of: one or more ribonucleotides, a mismatch base, a cleavage substrate, a length longer than a second homology region (HR2) at the opposite end of the MIP, an attached first member of an affinity pair, and a flap with a sequence different from a sequence of the HR2.

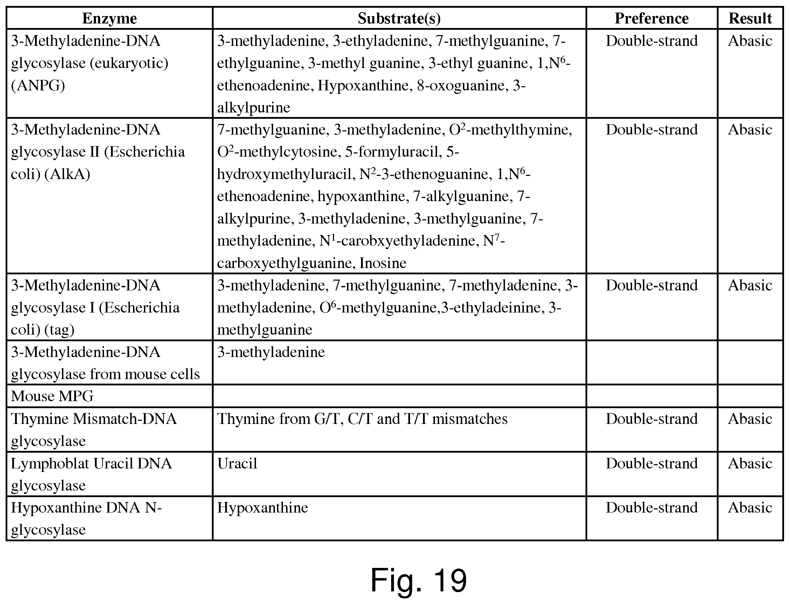

The cleavage site can be established by including certain modified nucleotides or bases at a desired location in the MIP sequence. For example, see the modified nucleotides listed in the Tables of Figures 19 to 21.

MIPs can be designed to facilitate multiplexed detections. For example, a disclosed but presently not claimed method of multiplex detection of SNP probe signals can include: providing a set of two or more different molecular inversion probes (MIPs) against different SNPs of interest, each different MIP comprising a different tag sequence and adapted to circularize in the presence of a putative target sequence comprising a different SNP of interest; probing a first sample with a first of the two or more MIPs; probing a second sample with a second of the two or more MIPs; pooling a MIP probe product of the first sample with a MIP probe product of the second sample; contacting the pooled probe products with an array comprising capture probes specific to the different tag sequences at different array locations; and, detecting the presence of captured probe products at one or more of the array locations, thereby identifying the presence of particular SNPs of interest in the particular samples. The method may further comprise amplifying the MIP probe products. Amplifying may comprise multiplex-PCR or quantitative PCR (qPCR). Tag sequences range in length from about 5 bases to 20 bases. The tag sequence may be a junction between a sample identification sequence and an allele identifying sequence. The MIP may comprise segments selected from the group consisting of: an HR1, an HR2, a first cleavage site, a forward (e.g. PCR) primer binding site, and a reverse (e.g. PCR) primer binding site. In some embodiments, the MIP comprises a first homology region (HR1) comprising a feature selected from the group consisting of: one or more ribonucleotides, a mismatch base, a cleavage substrate, a length longer than a second homology region (HR2) at the opposite end of the MIP, an attached first member of an affinity pair, and a flap with a sequence different from a sequence of the HR2. The probes may be captured at a solid support, bead, polymer, chromatography media, or capture probe of a next generation sequencing device. The capture probes may comprise a nucleic acid sequence selected from the group consisting of: a complement of a first MIP or second MIP homology region sequence, a SNP target sequence, a tag ID sequence complement, and a sequence overlapping a junction between a tag sequence and a homology region.

A useful MIP, or composition comprising the MIP, in the context of multiplexed detections can include one or more informative tags, such as a sample ID tag and/or an allele ID tag. For example, an MIP for multiplexed detection can include in order: a first homology region, a first (e.g., PCR) primer binding site, a first cleavage site, a second (e.g., PCR) primer binding site, a tag sequence, a second cleavage site, and a second homology region; wherein the MIP is adapted to hybridize to a target nucleic acid of interest with the first and second homology regions abutting each other, or separated by a gap of less than 3 bases; wherein the MIP is adapted so that, after circularization, cleavage at the first cleavage site linearizes the MIP into a substrate for (e.g., PCR) amplification using primers complementary to the first and second (e.g., PCR) primer binding sites; and wherein the MIP is adapted so that after (e.g., PCR) amplification cleavage at the second cleavage site separates the tag sequence from the homology regions. The composition may further comprise a (e.g. PCR) primer complementary to the second (e.g. PCR) binding site. The (e.g. PCR) primer may comprise a second tag sequence. The first tag sequence may code a sample identification and the second tag sequence codes an allele identification, the first tag sequence codes an allele identification and the second tag sequence codes a sample identification. The MIP may further comprise a first affinity pair member. HR1 or HR2 may comprise a feature selected from the group consisting of: one or more ribonucleotides, a mismatch base, a cleavage substrate, a length at least 5 nucleotides longer than the other HR, an attached first member of an affinity pair, and a flap with a sequence different from a sequence of the other HR.

One way of reading information from tags in an MIP is by detection of an informative sequence junction in the MIP reaction product. For example, a method of identifying a particular allele in a particular organism can include: providing a MIP comprising a first homology region site, a tag sequence, and a second homology region, wherein the tag sequence contacts one of the homology regions at a junction; hybridizing the MIP with a sample and ligating the MIP to form a circularized MIP if a target nucleic acid with an allele of interest is present in the sample; cleaving the circularized MIP and amplifying (such as by PCR), thus providing amplicons; providing a junction probe set comprising a series of oligomer probes with target sequences that overlap the junction between the tag and homology region in the amplicons; wherein the series of junction probes includes one or more members with predominantly tag sequence overlap and one or more members with predominantly homology sequence overlap; contacting the MIP to each of the junction probes under hybridizing conditions; and, detecting the extent to which each junction probe has hybridized to the amplicons; whereby a combination of tag sequence sample (organism) identity and homology region allele sequence identity is confirmed if all junction probes bind to the amplicons with similar stringency (e.g. under stringent hybridization conditions). For example, if both sides of the junction provides proper target sequences for the junction probes, the probes will each hybridize to their complementary sequence at least 50% to 150% as well as the other probes, e.g., with a melting temperatures within about 3°C of each other, 5°C, 7°C, 10°C, 15°C, or 20°C of each other. In some disclosed but presently not claimed methods, the junction probe set comprises three or more probes with different proportions of overlap on the two sides of the junction. The junction probe set has a range of melting temperatures (Tm) within 5°C of each other. The MIP may comprise additional segments selected from the group consisting of: a tag ID, a second cleavage site, a forward (e.g. PCR) primer binding site, and a reverse (e.g. PCR) primer binding site. HR1 or HR2 may comprise a feature selected from the group consisting of: one or more ribonucleotides, a mismatch base, a cleavage substrate, a length at least 5 nucleotides longer than the other HR, an attached first member of an affinity pair, and a flap with a sequence different from a sequence of the other HR.

Informative sequence junctions can be pre-established in MIPs or can arise, e.g., during cutting and ligating steps in an assay. For example, a presently not claimed method of identifying a target allele in a particular sample can include: providing a molecular inversion probe (MIP) comprising a target ID tag sequence adjacent to a first cleavage site, and a second cleavage site between a first PCR primer binding site and a second PCR primer binding site; probing a sample with the MIP, wherein the MIP is circularized if a target sequence of interest is present in the sample; cleaving the circularized MIP at the second cleavage site to linearize the MIP; amplifying the linearized MIP to produce amplicons using a PCR primer comprising a sequence complementary to the PCR primer binding site on same side of first cleavage site as the target ID tag, and the PCR primer comprising a 5' sequence encoding a sample ID; cleaving an amplicon at the first cleavage site, thus releasing a nucleic acid fragment comprising the target ID tag and sample ID sequence; circularizing the fragment, thus bringing the target ID tag into contact at a junction with the sample ID sequence; and, detecting the junction, thereby confirming the presence of the target allele in the particular sample. In some embodiments of said method, detecting the junction comprises a technique selected from the group consisting of: probing with a junction probe set, capture on an array with a capture probe specific to a sequence complementary to nucleotides on both sides of the junction, and next generation sequencing. The junction probe set may comprise three or more probes with different proportions of overlap on the two sides of the junction. The junction probes in the junction probe set may be adapted to have melting temperatures within 5°C (or 3°C, 7°C, 10°C, 15°C, or 20°C) of each other hybridizing to their respective target sequences across the junction. The MIP may comprise a first homology region (HR1) comprising a feature selected from the group consisting of: one or more ribonucleotides, a mismatch base, an internal tag, a cleavage substrate, a length longer than a second homology region (HR2) at the opposite end of the MIP, an attached first member of an affinity pair, and a flap with a sequence different from a sequence of the HR2.

Alternately, junctions can be provided in other probe types. For example, a disclosed but presently not claimed method of identifying a target allele in a particular sample can include: providing a first oligonucleotide ligation assay (OLA) probe comprising a first PCR primer binding site and a second OLA probe comprising a second PCR primer binding site, the pair of OLA probes adapted to specifically bind to a target nucleic acid of interest abutting each other without a gap; probing the sample with the first and second OLA probes under hybridizing conditions so that the probes abut if the target nucleic acid is present in the sample; contacting the probes with a ligase, thus ligating the probes together if they have been hybridized to the target nucleic acid; providing a first PCR primer comprising a sequence complementary to the first PCR primer binding site and comprising a sequence encoding a sample ID 5' from the PCR primer sequence; providing a second PCR primer comprising a sequence complementary to the second PCR primer binding site and comprising a 5' sequence encoding a target allele ID; amplifying ligated OLA probes using the first and second PCR primers, thereby producing amplicons with sample ID encoded on one end and target allele ID encoded at the other end; circularizing the amplicon, thus bringing the sample ID sequence into contact at a junction with the target allele ID sequence; and, detecting the junction, thereby confirming the presence of the target in the particular sample. This method may further comprise probing a different second sample with third and fourth OLA probes, wherein at least one of the probes binds to a different target sequence than the first or second OLA probe. Detecting the junction may comprise a technique selected from the group consisting of: probing with a junction probe set, capture on an array with a capture probe specific to a sequence complementary to nucleotides on both sides of the junction, and next generation sequencing. The junction probe set may comprise three or more probes with different proportions of overlap on the two sides of the junction. In some embodiments, the probes of the junction probe set are adapted to have melting temperatures within 5°C (or 3°C, 5°C, 7°C, 10°C, 15°C, or 20°C) of each other hybridizing to their respective target sequences across the junction.

Optionally, wherein release of a removable group is associated with a MIP hybridization, the hybridization can be detected in association with the free removable group. For example, a presently not claimed method of tagging a released MIP fragment can include providing a molecular inversion probe (MIP) with a 5' or 3' end specifically removable when the MIP is hybridized to the complementary target nucleic acid, hybridizing the MIP to the corresponding target nucleic acid, specifically cleaving the 5' or 3' end from the hybridized MIP, thus releasing the removable end from the MIP. The released removable end can be detected by any appropriate technique, such as by bDNA, NGS, PCR, MALDI TOF, etc.

The removable ends can be released as described herein, e.g., the end can be removable by a cleavage using one or more of a flap endonuclease, contacting with an RNase, contacting with a cleavase, contacting with a DNA intercalator, contact with chemical cleavage system, contacting with an endonuclease V, contacting with a mismatch endonuclease, and contacting with a glycolase. For example the removable end (region) can be removed from a MIP, as described herein, using the cleavage activity of a endonuclease V, a 3' cutting ribonuclease, a 5' cutting ribonuclease, a celery mismatch endonuclease (CEL I), a glycosylase TDG, a glycosylase MutY, and AP endonuclease/ lyase, a T4 endonuclease VII, a T7 endonuclease I, a deoxyinosine 3'-endonuclease, a mung bean nuclease, a resolvase, a flap endonucleases, a cleavase, a 3-methyladenine-DNA glycosylase, a thymine mismatch-DNA glycosylase, a uracil DNA glycosylase, a hypoxanthine DNA N-glycosylase, an endonuclease IV, an APE 1 AP endonuclease, an exonuclease III, an endonuclease IV (nfo), a 8-oxoguanine-DNA glycosylase, a 3-methyladenine-DNA glycosylase, DNA intercalator, a substrate for the hydroxylamine/permanganate/piperidine cleavage reaction, a substrate for the osmium tetroxide/piperidine cleavage reaction; and a thymine mismatch-DNA glycosylase.

A method of detecting released removable ends further comprises providing one or more semi-duplex probes, each probe comprising a double stranded tag sequence and a single stranded capture sequence, and capturing the released removable end by specific hybridization to a semi-duplex probe capture sequence. Capture on an array can be by hybridization of the tag sequence or a tag/removable end junction to a capture probe on a solid support. In certain instances, the semi-duplex probes are a set of tagged stochastic probes with randomized capture sequences (useful, e.g., in counting a number of target sequence copies in a sample). Optionally, the detection can be facilitated by ligating the releasable end to the semi-duplex probe and amplifying the ligated probe to provide amplicons. The amplicons can be detected by, e.g., qPCR, NGS, and capture on an array. In some embodiments, capture on an array comprises hybridization of the tag sequence or a tag/removable end junction to a capture probe on a solid support.

The use of stochastic probes can allow enumeration of the amount of a particular sequence that is present in a test sample. For example, A not claimed method of quantitating a number present in a sample for one or more target nucleic acids can include the steps of hybridizing one or more MIPs to one or more of the target nucleic acids (wherein the MIPs comprise a removable region specifically cleavable when the MIP is hybridized to the target nucleic acid comprising an allele of interest), specifically cleaving the one or more hybridized MIPs releasing one or more removable regions, attaching different random stochastic labels to each of the one or more released removable regions, and determining a number of target nucleic acids in the sample by counting the number of different labels are attached to removable regions, or by counting the number of different removable region/label junctions are present. Counting the number of labels or junctions may be by specific capture and detection at an array location. Optionally, the counting a number of removable region/label junctions can be by hybridization with sets of two or more junction probes or by NGS of the labeled released regions. The methods may further comprise amplifying the labeled released regions. The removable region may be released using an enzyme or chemistry selected from the group consisting of: a endonuclease V, a 3' cutting ribonuclease, a 5' cutting ribonuclease, a celery mismatch endonuclease (CEL I), a glycosylase TDG, a glycosylase MutY, and AP endonuclease/lyase, a T4 endonuclease VII, a T7 endonuclease I, a deoxyinosine 3'-endonuclease, a mung bean nuclease, a resolvase, a flap endonucleases, a cleavase, a 3-methyladenine-DNA glycosylase, a thymine mismatch-DNA glycosylase, a uracil DNA glycosylase, a hypoxanthine DNA N-glycosylase, an endonuclease IV, an APE 1 AP endonuclease, an exonuclease III, an endonuclease IV (nfo), a 8-oxoguanine-DNA glycosylase, a 3-methyladenine-DNA glycosylase, DNA intercalator, a substrate for the hydroxylamine/permanganate/piperidine cleavage reaction, a substrate for the osmium tetroxide/piperidine cleavage reaction; and a thymine mismatch-DNA glycosylase.

Attaching the random stochastic labels can be by providing a set of different semi-duplex label probes, each comprising a double stranded segment coding tag information and a single stranded segment putatively complementary to a released region; and, hybridizing the set of label probes to the released regions. The hybridized complex can be ligated and amplified, e.g., as required by intended detection techniques.

The technique can be used to count copies of a particular target nucleic of interest in a sample, or to quantitate a variety of different target sequences, e.g., in a multiplexed fashion. For example, two or more of the MIPs are hybridized to two or more nucleic acids of interest, e.g., with release of different removable regions. There can be, e.g., ten or more different labels available to randomly attach to any and each of the released regions.

A powerful method of multiplexed detection of MIP probe products can be by massively parallel sequencing, e.g., next generation sequencing (NSG). For example, a method of determining a pattern of alleles in a sample can include: probing the sample with two or more MIPs, wherein the presence of a target sequence having an allele of interest results in circularization of one or more target-complementary MIPs; cleaving the circularized MIPs to form linearized MIPs; introducing next generation sequencing adaptors to ends of the linearized MIPs; and, determining the sequences of the linearized MIPs, thus identifying the identity and/or quantity of alleles in the sample. By probing two or more samples with tag-identified probe sets, the results for more than one sample can be read unambiguously with NGS. Introduction of next generation primers can include providing a 50 to 90 base hybrid primer oligomer comprising a 3' PCR primer region and a 5' NGS primer region; and, amplifying the linearized MIPs by PCR using the hybrid primer, thereby producing amplified MIPs competent for NGS sequencing. In some embodiments, the MIP comprises segments selected from the group consisting of: an HR1, an HR2, a first cleavage site, a forward (e.g. PCR) primer binding site, and a reverse (e.g. PCR) primer binding site, a tag ID, a tag within an HR sequence, and an attached first affinity pair member. One or more of the MIPs may comprise a first homology region (HR1) comprising a feature selected from the group consisting of: one or more ribonucleotides, a mismatch base, a cleavage substrate, a length longer than a second homology region (HR2) at the opposite end of the MIP, an attached first member of an affinity pair, and a flap with a sequence different from a sequence of the HR2. In some embodiments, the NGS technique is selected from the group consisting of: sequencing by solid phase bridge amplification (Illumina), single-molecule real-time sequencing (Pacific Bio), pyrosequencing, emulsion PCR (emPCR), ion semiconductor sequencing, and sequencing by ligation.

Certain MIPs can provide more information in a more compact space, e.g., while enhancing specificity. A MIP composition may include an informational tag sequence within one or both HR sequence. For example, a molecular inversion probe can include a first homology region (HR1) sequence at a 5' end and a second homology region (HR2) sequence at a 3' end, with the HR1 comprising a sequence complementary to a target nucleic acid and the HR2 comprising a sequence complementary to the target nucleic acid, and wherein the target nucleic acid sequence complementary to HR1 is located 5' from the a target sequence complementary to HR2. A feature of this particular MIP is that the HR1 sequence and/or the HR2 sequence comprises a 3' sequence and a 5' sequence with a tag sequence therebetween. That is, and informational tag sequence is inserted between bases of the HR. Typically, the tag is inserted so that the 3' and 5' sequence segments are about of equal length or of similar (e.g., within 5°C, 3°C, or 2°C) melting temperature. With regard to MIPs with tags incorporated into one or more HR, a target sequence complementary to HR1 can be located 3' adjacent to a target sequence complementary to HR2, or a target sequence complementary to HR1 can be located 5' within 15 nucleotides from a target sequence complementary to HR2. The tag sequence can encode any desired information, e.g., a sample identification sequence, target information sequence, a SNP information sequence, a capture probe sequence complement, a primer probe sequence complement, sample acquisition time encoding sequence, and/or the like. In certain embodiments, the HR1 sequence and the HR2 sequence can each have a 3' sequence and a 5' sequence with the tag sequence positioned between them. It can be useful in some assay schemes to have a cleavage site between the tag sequence and an HR1 sequence or an HR2 target complement sequence.

The MIPs having informational tags described herein, can be used in e.g., in method of providing information in a molecular inversion probe assay. For example, such methods can include providing a first molecular inversion probe (MIP) comprising a first homology region (HR1) sequence at a 5' end and a second homology region (HR2) sequence at a 3' end, wherein the HR1 comprises a sequence complementary to a first target nucleic acid and the HR2 comprises a sequence complementary to the first target nucleic acid. The target sequence complementary to HR1 can be located 5' from the target sequence complementary to HR2. In these methods the HR1 sequence and/or the HR2 sequence can have a 3' sequence and a 5' sequence with a tag sequence therebetween (e.g., a tag sequence separating two segments of the HR). The MIP is hybridized to a first sample comprising one or more putative target nucleic acids. With HR1 and HR2 of the MIP hybridized to the target, the MIP3' end can be ligated to the 5' end (optionally after an extension step across a gap); thus, circularizing the MIP. After further processing, such as exonuclease treatment to remove non-circularized MIPs, the circularized MIPs can be detected by any number of assays described herein, e.g., hybridization arrays, amplification (e.g., PCR), bDNA, nucleic acid sequencing, electrophoresis, northern/Southern blotting, NGS , and/or the like. From these assays, information can be acquired from the presence or sequence of the tag embedded in the HR. For example, the tag sequence information can include a sample identification sequence, target information sequence, a SNP information sequence, a capture probe sequence complement, a primer probe sequence complement, and sample acquisition time encoding sequence, and/or the like.

In the methods of using tagged HRs, described above and herein, there are optional steps and structures providing benefits alone or in combination. For example, the HR1 3' sequence and 5' sequence segment can have at least 5 bases (e.g., 5 to 20 bases), and/or the HR2 3' sequence and 5' sequence comprises at least 5 bases (e.g., 5 to 20 bases). The method can include a step of extending across a gap between the HR1 and the HR2 using a nucleic acid polymerase in the presence of nucleoside triphosphates (gap fill). The detection or reading of the tag can comprise capturing the tag on an array, amplifying the tag, hybridizing a PCR primer to the tag, next generation sequencing (NGS) of the tag, and/or the like. The method can optionally include hybridizing a second MIP to a second sample, wherein the second MIP has a second tag sequence different from the first tag sequence. After hybridization (optionally - extension, ligation, and/or exonuclease treatment), the first and second MIP reaction products can be pooled and detected in the same assay, e.g., including reading of first tag and second tag information.

The MIP assays described herein are well adapted to matrix or massively parallel detection. For example, information in MIP reaction products can allow multiple MIP products from multiple different samples to be detected together. One sample can be interrogated by multiple (e.g., thousands) of MIPs and the MIP products detected in one assay. Alternately, several samples can each be separately interrogated with a group of relevant MIPs of interest, and the reactions of several samples pooled for efficient detection in the same assay. In one aspect of this idea, different samples are reacted with different MIP panels before pooling and detection together. For example, a first subpanel of one or more molecular inversion probes (MIPs) can be provided, which first subpanel MIPs encode a first sample identification based on a first set of nucleic acid targets interrogated by the first subpanel of MIPs. A second subpanel of one or more (e.g., in a different combination) MIPs is provided, which second panel MIPs encode a second sample identification different from the first sample identification based on a second set of nucleic acid targets interrogated by the second subpanel of MIPs. MIP reactions (including, e.g., hybridization, extension, ligation, and/or exonuclease treatment), are carried out separately between the first sample and the first subpanel of MIPs; and a second MIP reaction is carried out between the second sample and the second subpanel of MIPs. The first and second reaction products are pooled together and their reaction products are detected in the same assay. Because of the information content encoded in the MIP products, the output signals of the assay (sequencing, array, NGS, amplification (e.g., PCR), blotting, etc.) can be assigned to particular samples. Reaction products can be detected by any number of techniques, e.g., capturing the tag (or HR/tag interface sequences) on an array, amplifying the tag, hybridizing a PCR primer to the tag, and next generation sequencing (NGS) of the tag. Such methods can provide increased efficiency and lowered costs by sharing assay expenses and only acquiring desired information per sample.

### DEFINITIONS

Before describing the present invention in detail, it is to be understood that this invention is not limited to particular devices or biological systems, which can, of course, vary. It is also to be understood that the terminology used herein is for the purpose of describing particular embodiments only, and is not intended to be limiting. As used in this specification and the appended claims, the singular forms "a", "an" and "the" include plural referents unless the content clearly dictates otherwise. Thus, for example, reference to "a surface" includes a combination of two or more surfaces; reference to "bacteria" includes mixtures of bacteria, and the like.

Unless defined otherwise, all technical and scientific terms used herein have the same meaning as commonly understood by one of ordinary skill in the art to which the invention pertains. Although any methods and materials similar or equivalent to those described herein can be used in the practice for testing of the present invention, the preferred materials and methods are described herein. In describing and claiming the present invention, the following terminology will be used in accordance with the definitions set out below.

Two nucleic acids "abut" each other when hybridized on a complementary target nucleic acid when there is no gap between them, but the abutting ends are not linked through a joined phosphate-sugar back bone. Typically, such abutting nucleic acids constitute a substrate for a ligase enzyme.

An "affinity pair" is a couple of molecular or molecular group scale moieties with an affinity that causes binding in contact, as is known in the art. For example, representative affinity pairs include biotin/avidin, antibody/antigen, metal/chelator, ligand/receptor, nucleic acid and binding protein, complementary nucleic acids, and the like. In some embodiments, the affinity pair does not include a nucleic acid member. Binding of affinity pair members is typically high affinity (e.g., K_{d} ranging from 10⁻³ M to 10⁻¹⁶ M, 10⁻⁵ M to 10⁻¹⁵ M or 10⁻⁷ M to 10⁻¹² M), or the binding can create a covalent bond. An affinity pair member is one member of the affinity pair.

The term "array" as used herein refers to an intentionally created collection of molecules which can be prepared either synthetically or biosynthetically, as is known in the art. The molecules in the array can be identical or different from each other. The array can assume a variety of formats, for example, libraries of soluble molecules; libraries of compounds attached (tethered with a linker group or directly bound) to resin beads, silica chips, or other solid supports.

To "circularize", as used herein, refers to ligating a 3' end of a nucleic acid to a 5' end of the nucleic acid, thereby creating a continuous uncut loop or circle. To "linearize" is to cut a circular nucleic acid so that it no longer has an uncut continuous sugar/phosphate backbone.

The term "complementary" as used herein refers to the hybridization or base pairing between nucleotides or nucleic acids, such as, for instance, between the two strands of a double stranded DNA molecule or between an oligonucleotide primer and a primer binding site on a single stranded nucleic acid to be sequenced or amplified. Complementary nucleotides are, generally, A and T (or A and U), or C and G. Two single stranded RNA or DNA molecules are said to be complementary when the nucleotides of one strand, optimally aligned and compared and with appropriate nucleotide insertions or deletions, pair with at least about 80% of the nucleotides of the other strand, usually at least about 90% to 95%, and more preferably from about 98 to 100%. Alternatively, complementarity exists when an RNA or DNA strand will hybridize under selective hybridization conditions to its complement. Typically, selective hybridization will occur when there is at least about 65% complementary over a stretch of at least 14 to 25 nucleotides, preferably at least about 75%, more preferably at least about 90% complementary. See, M. Kanehisa Nucleic Acids Res. 12:203 (1984). Typically, with homology regions of a MIP there is 100% complementarity with target nucleic acid of interest, e.g., unless there is a mismatch at the position of the interrogated nucleotide of interest.

The term "endonuclease" refers to an enzyme that cleaves a nucleic acid (DNA or RNA) at internal sites in a nucleotide base sequence. Cleavage may be random along the nucleic acid or at a specific recognition sequence, or at sites of modified sequence of bases on the nucleic acid. Specifically, endonuclease biochemical activity is the hydrolysis of the phosphodiester backbone at sites in a DNA sequence. Examples of endonucleases include endonuclease V (Endo V) also called deoxyinosine 3' endonuclease, which recognizes DNA containing deoxyinosines (paired or not). Endonuclease V cleaves the second and third phosphodiester bonds 3' to the mismatch of deoxyinosine with a 95% efficiency for the second bond and a 5% efficiency for the third bond, leaving a nick with 3' hydroxyl and 5' phosphate. Endo V, to a lesser, degree, also recognizes DNA containing abasic sites and also DNA containing urea residues, base mismatches, insertion/deletion mismatches, hairpin or unpaired loops, flaps and pseudo-Y structures. See also, Yao et al., J. Biol. Chem., 271(48): 30672 (1996), Yao et al., J. Biol. Chem., 270(48): 28609 (1995), Yao et al., J. Biol. Chem., 269(50): 31390 (1994), and He et al., Mutat. Res., 459(2):109 (2000). Endo V from *E. coli* is active at temperatures between about 30 and 50°C and preferably is incubated at a temperature between about 30°C to 37°C. Endo V is active in NE Buffer 4 (20 mM Tris-acetate, 50 mM potassium acetate, 10 mM magnesium acetate and 1 mM DTT, pH 7.9 at 25°C), but is also active in other buffer conditions, for example, 20 mM HEPES-NaOH (pH 7.4), 100 mM KCl, 2 mM MnCl₂ and 0.1 mg/ml BSA. Endo V makes a strand specific nick about 2-3 nucleotides downstream of the 3' side of inosine base, without removing the inosine base. Endonucleases, including Endo V, may be obtained from manufacturers such as New England Biolabs (NEB) or Fermentas Life Sciences.

A "gap-fill reaction" is a reaction, described herein, in which a gap is filled by the action of a polymerase between 5' and 3' ends of a molecular inversion probe hybridized to a complementary target nucleic acid. In many embodiments, the filled gap consists of a single nucleotide. However, in some MIP gap-fill reactions the gap can be more than one nucleotide, e.g., 2, 3, 4, 5, 6, 7, 8, 9, 10, 15, 20, 25, 30, 40, 50 75, 100, 150, 200, 250 or more nucleotides, e.g., between first and second MIP homology regions specifically hybridized to a target nucleic acid.

"Homology regions", as used herein are those parts of a molecular inversion probe that are complementary to the target nucleic acid of interest. MIPs typically have two homology regions (HRs), one at or near the 5' end of the probe and one at or near the 3' end. In many embodiments, the HRs are adapted to hybridize to a target nucleic acid of interest so that they abut each other or are separated by a gap of a single target nucleotide or a plurality of target nucleotides. A gap of a plurality of target nucleotides can include, e.g., from 1 to about 2000 nucleotides, preferably from 1 to 500 nucleotides, and more preferably 1 to 250 nucleotides. The size of the gap will depend on a variety of factors, including the sequence of the intended target, the size of the overall MIP, the quantity and size of non-HR portions of the MIP, the desired purpose of the assay and associated characteristics, and other factors. For instance, a MIP designed to interrogate a SNP may have a gap of a single nucleotide while a MIP designed to interrogate a multi-base insertion may have a gap of multiple nucleotides. Certain HRs of the present disclosure also include special features, such as, e.g., longer length that overlaps with the other HR, cleavage substrate nucleotides, flap ends not complementary to the target, and the like, described fully herein.

The term "hybridization" as used herein refers to the process in which two single-stranded polynucleotides bind non-covalently to form a stable double-stranded polynucleotide; triple-stranded hybridization is also theoretically possible. The resulting (usually) double-stranded polynucleotide is a "hybrid." Hybridizations are usually performed under stringent conditions, for example, at a salt concentration of no more than about 1 M and a temperature of at least 25°C. For example, conditions of 5X SSPE (750 mM NaCl, 50 mM NaPhosphate, 5 mM EDTA, pH 7.4) and a temperature of 25°C-30°C are suitable for allele-specific probe hybridizations or conditions of 100 mM MES, 1 M [Na⁺], 20 mM EDTA, 0.01% Tween-20 and a temperature of 30°C-50°C, preferably at about 45°C-50°C. Hybridizations may be performed in the presence of agents such as herring sperm DNA at about 0.1 mg/ml, acetylated BSA at about 0.5 mg/ml. As other factors may affect the stringency of hybridization, including base composition and length of the complementary strands, presence of organic solvents and extent of base mismatching, the combination of parameters is more important than the absolute measure of any one alone. Additional guidance for hybridization conditions suitable for various assays can be found, e.g., in Michael R. Green & Joseph Sambrook, Molecular Cloning: A Laboratory Manual, (4th ed. 2012).

A "hybridizing condition" is a condition expected to result in specific hybridization between complementary sequences, e.g., test nucleic acid is said to specifically hybridize to a probe nucleic acid when it hybridizes at least 50% as well (e.g., quantitatively under the same hybridization conditions) to the probe as to the perfectly matched complementary target, i.e., with a signal to noise ratio at least half as high as hybridization of the probe to the target under conditions in which the perfectly matched probe binds to the perfectly matched complementary target with a signal to noise ratio that is at least about 5x-10x as high as that observed for hybridization to any of the unmatched target nucleic acids.

An "interrogator" is a moiety that tests for the presence of a condition of interest. For example, an interrogator nucleotide in a MIP homology region is a base that will complement a target nucleotide of interest; if the nucleotide of interest is present, hybridization occurs at that position, and typically this is the key event determining whether or not the MIP becomes a ligase substrate. In other embodiments discussed herein, the interrogator may be a nucleotide that creates a mismatch with the corresponding target nucleotide of interest; if the nucleotide of interest is present, an enzyme that cuts at mismatched bases may initiate a signal by cutting at that location.

A "junction" is a point of contact between a pair of adjacent nucleic acid regions, such as, e.g., a sample ID tag and a homology region, a sample ID tag and an allele ID tag, and the like. A junction probe set is a set of two or more oligomer probes adapted to specifically hybridize to sequences ranging across the junction to contact nucleotides on both sides of the junction. The junction probes are typically arranged to include at least one probe covering more sequence on one side of the junction and at least one other probe covering more sequence on the other side of the junction. Review of the relative hybridization strength or the various probes can confirm the presence of the appropriate sequence on each side of the junction.

A "molecular inversion probe" (MIP) is a nucleic acid probe that hybridizes to the complementary sequences of a target nucleic acid of interest with the MIP 5' end hybridizing 5' on the target from the position at which the MIP 3' end hybridizes to the target. Where the target is circular, the orientation is with regard to the closest positions of the 3' and 5' ends. When hybridized to target, MIPs form a loop back from one end to the other. The MIP sequences configured to hybridize to target sequences are referred to as homology regions.

The term "nucleic acids" as used herein may include any polymer or oligomer of pyrimidine and purine bases, preferably cytosine, thymine, and uracil, and adenine and guanine, respectively. *See* Albert L. Lehninger, Principles of Biochemistry, at 793-800 (Worth Pub. 1982). Indeed, the present invention contemplates any deoxyribonucleotide, ribonucleotide or peptide nucleic acid component, and any chemical variants thereof, such as methylated, hydroxymethylated or glucosylated forms of these bases, and the like. The polymers or oligomers may be heterogeneous or homogeneous in composition, and may be isolated from naturally-occurring sources or may be artificially or synthetically produced. In addition, the nucleic acids may be DNA or RNA, or a mixture thereof, and may exist permanently or transitionally in single-stranded or double-stranded form, including homoduplex, heteroduplex, and hybrid states.

The term "base pair mismatch" indicates a base pair combination that generally does not form in nucleic acids according to Watson and Crick base pairing rules, as is known in the art. For example, when dealing with the bases commonly found in DNA, namely adenine, guanine, cytosine and thymidine, base pair mismatches are those base combinations other than the A-T and G-C pairs normally found in DNA. As described herein, a mismatch may be indicated, for example as C/C meaning that a cytosine residue is found opposite another cytosine, as opposed to the proper pairing partner, guanine.

The terms "oligonucleotide" and "polynucleotide" as used herein refers to a nucleic acid ranging from at least 2, preferable at least 8, and more preferably at least 20 nucleotides in length or a compound that specifically hybridizes to a polynucleotide. Polynucleotides of the present invention include sequences of deoxyribonucleic acid (DNA) or ribonucleic acid (RNA) which may be isolated from natural sources, recombinantly produced or artificially synthesized and mimetics thereof. A further example of a polynucleotide of the present invention may be peptide nucleic acid (PNA). The invention also encompasses situations in which there is a nontraditional base pairing such as Hoogsteen base pairing which has been identified in certain tRNA molecules and postulated to exist in a triple helix. "Polynucleotide" and "oligonucleotide" are used interchangeably in this application.

Polynucleotide elements are "in order" if they appear in the identified order serially along the polynucleotide (e.g., 3'-5', or 5'-3'). The elements are considered in order regardless of other unspecified elements therebetween.

"Polymerase chain reaction," or "PCR," means a reaction for the in vitro amplification of specific DNA sequences by the simultaneous primer extension of complementary strands of DNA, as is notoriously well known in the art. In other words, PCR is a reaction for making multiple copies or replicates of a target nucleic acid flanked by primer binding sites, such reaction comprising one or more repetitions of the following steps: (i) denaturing the target nucleic acid, (ii) annealing primers to the primer binding sites, and (iii) extending the primers by a nucleic acid polymerase in the presence of nucleoside triphosphates. Usually, the reaction is cycled through different temperatures optimized for each step in a thermal cycler instrument. Particular temperatures, durations at each step, and rates of change between steps depend on many factors well-known to those of ordinary skill in the art, e.g. exemplified by the references: McPherson et al, editors, PCR: A Practical Approach and PCR2: A Practical Approach (IRL Press, Oxford, 1991 and 1995, respectively). For example, in a conventional PCR using Taq DNA polymerase, a double stranded target nucleic acid may be denatured at a temperature >90°C, primers annealed at a temperature in the range 50-75°C, and primers extended at a temperature in the range 72-78°C. The term "PCR" encompasses derivative forms of the reaction, including but not limited to, RT-PCR, real-time PCR, nested PCR, quantitative PCR, multiplexed PCR, and the like. Reaction volumes range from a few hundred nanoliters, e.g. 200 nL, to a few hundred uL, e.g. 200 uL. "Reverse transcription PCR," or "RT-PCR," means a PCR that is preceded by a reverse transcription reaction that converts a target RNA to a complementary single stranded DNA, which is then amplified, e.g. Tecott et al, U.S. Pat. No. 5,168,038. "Real-time PCR" means a PCR for which the amount of reaction product, i.e. amplicon, is monitored as the reaction proceeds. There are many forms of real-time PCR that differ mainly in the detection chemistries used for monitoring the reaction product, e.g. Gelfand et al, U.S. Pat. No. 5,210,015 ("taqman"); Wittwer et al, U.S. Pat. Nos. 6,174,670 and 6,569,627 (intercalating dyes); Tyagi et al, U.S. Pat. No. 5,925,517 (molecular beacons). Detection chemistries for real-time PCR are reviewed in Mackay et al, Nucleic Acids Research, 30: 1292-1305 (2002). "Nested PCR" means a two-stage PCR wherein the amplicon of a first PCR becomes the sample for a second PCR using a new set of primers, at least one of which binds to an interior location of the first amplicon. As used herein, "initial primers" in reference to a nested amplification reaction mean the primers used to generate a first amplicon, and "secondary primers" mean the one or more primers used to generate a second, or nested, amplicon. "Multiplexed PCR" means a PCR wherein multiple target sequences (or a single target sequence and one or more reference sequences) are simultaneously carried out in the same reaction mixture, e.g. Bernard et al, Anal. Biochem., 273: 221-228 (1999)(two-color real-time PCR). Usually, distinct sets of primers are employed for each sequence being amplified. "Quantitative PCR" means a PCR designed to measure the abundance of one or more specific target sequences in a sample or specimen. Quantitative PCR includes both absolute quantitation and relative quantitation of such target sequences. Quantitative measurements are made using one or more reference sequences that may be assayed separately or together with a target sequence. The reference sequence may be endogenous or exogenous to a sample or specimen, and in the latter case, may comprise one or more competitor templates. Typical endogenous reference sequences include segments of transcripts of the following genes: beta.-actin, GAPDH, .beta..sub.2-microglobulin, ribosomal RNA, and the like. Techniques for quantitative PCR are well-known to those of ordinary skill in the art, as exemplified in the following references: Freeman et al, Biotechniques, 26: 112-126 (1999); Becker-Andre et al, Nucleic Acids Research, 17: 9437-9447 (1989); Zimmerman et al, Biotechniques, 21: 268-279 (1996); Diviacco et al, Gene, 122: 3013-3020 (1992); Becker-Andre et al, Nucleic Acids Research, 17: 9437-9446 (1989); and the like.

"Polymorphism" refers to the occurrence of two or more genetically determined alternative sequences or alleles in a population. A polymorphic marker or site is the locus at which divergence occurs. Preferred markers have at least two alleles, each occurring at frequency of greater than 1%, and more preferably greater than 5%, 10% or 20% of a selected population. A polymorphism may comprise one or more base changes, an insertion, a repeat, or a deletion. A polymorphic locus may be as small as one base pair. Polymorphic markers include restriction fragment length polymorphisms, variable number of tandem repeats (VNTR's), hypervariable regions, minisatellites, dinucleotide repeats, trinucleotide repeats, tetranucleotide repeats, simple sequence repeats, and insertion elements such as Alu. The first identified allelic form is arbitrarily designated as the reference form and other allelic forms are designated as alternative or variant alleles. The allelic form occurring most frequently in a selected population is sometimes referred to as the wildtype form. Diploid organisms may be homozygous or heterozygous for allelic forms. A diallelic polymorphism has two forms. A triallelic polymorphism has three forms. Single nucleotide polymorphisms (SNPs) are included in polymorphisms.

The term "primer" as used herein refers to a single-stranded oligonucleotide capable of acting as a point of initiation for template-directed DNA synthesis under suitable conditions for example, buffer and temperature, in the presence of four different nucleoside triphosphates and an agent for polymerization, such as, for example, a DNA polymerase, RNA polymerase or reverse transcriptase. The length of the primer, in any given case, depends on, for example, the intended use of the primer, and generally ranges from 15 to 30 nucleotides. Short primer molecules generally require cooler temperatures to form sufficiently stable specific hybrid complexes with the template. A primer need not reflect the exact sequence of the template but must be sufficiently complementary to hybridize with such template. The primer site is the area of the template to which a primer hybridizes. The primer pair is a set of primers including a 5' upstream primer that hybridizes with the 5' end of the sequence to be amplified and a 3' downstream primer that hybridizes with the complement of the 3' end of the sequence to be amplified.

"Sample" means a quantity of material from a biological, environmental, medical, animal, bacterial, plant or patient source in which detection or measurement of target nucleic acids is sought. Often a sample is a lysate of an organism tissue of cells. Typically, samples in the present context include materials comprising nucleic acids. On the one hand it is meant to include a specimen or culture (e.g., microbiological cultures). On the other hand, it is meant to include both biological and environmental samples. A sample may include a specimen of synthetic origin. Biological samples may be animal, including human, fluid, solid (e.g., stool) or tissue, as well as liquid and solid food and feed products and ingredients such as dairy items, vegetables, meat and meat by-products, and waste. Biological samples may include materials taken from a patient including, but not limited to cultures, blood, saliva, cerebral spinal fluid, pleural fluid, milk, lymph, sputum, semen, needle aspirates, and the like. Biological samples may be obtained from all of the various families of domestic animals, as well as feral or wild animals, including, but not limited to, such animals as ungulates, bear, fish, rodents, etc. Biological samples may also be obtained from plants, such as maize, rice, wheat, lettuce and pepper. Environmental samples include environmental material such as surface matter, soil, water and industrial samples, as well as samples obtained from food and dairy processing instruments, apparatus, equipment, utensils, disposable and non-disposable items. These examples are not to be construed as limiting the sample types applicable to the present invention. The term "admixture" refers to the phenomenon of gene flow between populations resulting from migration. Admixture can create linkage disequilibrium (LD).

The term "solid support", "support", and "substrate", as used herein, are used interchangeably and refer to a material or group of materials having a rigid or semi-rigid surface or surfaces. In many embodiments, at least one surface of the solid support will be substantially flat, although in some embodiments it may be desirable to physically separate synthesis regions for different compounds with, for example, wells, raised regions, pins, etched trenches, or the like. According to other embodiments, the solid support(s) will take the form of beads, resins, gels, microspheres, or other geometric configurations. See U.S. Patent No. 5,744,305 for exemplary substrates.

"Specific" or "specificity" in reference to the binding of one molecule to another molecule, such as a labeled target sequence for a probe, affinity pairs, means the recognition, contact, and formation of a stable complex between the two molecules, together with substantially less recognition, contact, or complex formation of that molecule with other molecules. In one aspect, "specific" in reference to the binding of a first molecule to a second molecule means that to the extent the first molecule recognizes and forms a complex with another molecule in a reaction or sample, it forms the largest number of the complexes with the second molecule. Preferably, this largest number is at least fifty percent. Generally, molecules involved in a specific binding event have areas on their surfaces or in cavities giving rise to specific recognition between the molecules binding to each other. Examples of specific binding include antibody-antigen interactions, enzyme-substrate interactions, formation of duplexes or triplexes among polynucleotides and/or oligonucleotides, receptor-ligand interactions, and the like. As used herein, "contact" in reference to specificity or specific binding means two molecules are close enough that weak non-covalent chemical interactions, such as Van der Waal forces, hydrogen bonding, base-stacking interactions, ionic and hydrophobic interactions, and the like, dominate the interaction of the molecules.

"Tm" is used in reference to "melting temperature." Melting temperature is the temperature at which a population of double-stranded nucleic acid molecules becomes half dissociated into single strands. Several equations for calculating the Tm of nucleic acids are well known in the art. As indicated by standard references, a simple estimate of the Tm value may be calculated by the equation. Tm=81.5+0.41 (% G+C), when a nucleic acid is in aqueous solution at 1 M NaCl (see e.g., Anderson and Young, Quantitative Filter Hybridization, in Nucleic Acid Hybridization (1985). Other references (e.g., Allawi, H. T. & SantaLucia, J., Jr., Biochemistry 36, 10581-94 (1997)) include alternative methods of computation which take structural and environmental, as well as sequence characteristics into account for the calculation of Tm.

The term "target nucleic acid of interest", as used herein, refers to the sample nucleic acid putatively including a target sequence of interest. The target sequence of interest, with regard to a MIP includes those sequences complementary to the MIP homology regions. The sequence may include one or more interrogated nucleotides that may or may not match a corresponding nucleotide on a MIP homology region, or may or may not provide a substrate for a polymerase provided with the complementary NTP.

### BRIEF DESCRIPTION OF THE DRAWINGS

Figure 1 is a schematic diagram showing a typical initial MIP probe structure. The homologous regions, homology region 1 (HR1) and homology region 2 (H2), are adapted to hybridize to a target nucleic acid, e.g., in a region bracketing a SNP or somatic mutation to be interrogated. The example includes forward and reverse PCR primer binding sites and an identification tag.
Figure 2 is a schematic diagram showing a structure of the MIP probe after annealing, gap-fill, and fragmentation by Uracil N-Glycosylase (UNG - for cleavage of the UUU sequence). Cleavage results in PCR primer binding sites at opposite ends of the linearized probe, for ready amplification.
Figure 3 is a schematic diagram of probes hybridized to target before the gap-fill reaction.
Figure 4 is a schematic diagram of probes hybridized to target after the gap-fill reaction. Each of the four gap-fill reactions were provided with the proper complementary NTP and the polymerase was able to fill the single base gap.
Figure 5 is a schematic diagram depicting a problematic strand displacement that can occur during the gap-fill reaction. The polymerase may push aside the specifically hybridized 5' end MIP and add one or more 3'bases that can prevent hybridization and ligation of the 5' MIP end to the 3' MIP end. Because the strand displaced MIP is not subject to ligation, there would be no signal from this specific hybridization event to the target after the removal of non-circularized MIPs.
Figure 6 is a schematic diagram showing how a MIP with a longer homology region and a ribonucleotide interrogator can be used in a scheme to employ MIPs not requiring a gap-fill polymerase step, instead using a 5' cutting RNase.
Figure 7 is a schematic diagram showing how a MIP with a longer homology region and ribonucleotide interrogator can be used in a scheme to employ MIPs not requiring a gap-fill polymerase step, instead using a 3' cutting RNase.
Figure 8 presents a table of MIP homology region sequences appropriate for interrogating a number of SNP alternates to the wild type BRAF gene.
Figure 9 is a schematic diagram showing how a MIP with a longer homology region interrogator can be used in a scheme to employ MIPs not requiring a gap-fill polymerase step, instead using a 5' cutting enzyme at a base mismatch substrate.
Figure 10 is a schematic diagram showing how a MIP with a longer homology region interrogator can be used in a scheme to employ MIPs not requiring a gap-fill polymerase step, instead using a 3' cutting enzyme at a base mismatch substrate.
Figure 11 a schematic diagram showing how a MIP with an interrogating homology region with non-hybridizing flap which can be used in a scheme to employ MIPs not requiring a gap-fill polymerase step, instead using a flap endonuclease.
Figure 12 is a schematic diagram showing how an oligomer insert probe can be used to bridge an MIP probe gap, without the need for a gap-fill reaction.
Figure 13 is a schematic diagram depicting a gap-fill reaction wherein bases incorporated past the gap are ribonucleotides, readily removed with an RNase.
Figures 14 and 15 show schematic diagrams of MIPs having affinity pair members and a cleavage target interrogating base in the longer homology region. Specific cleavage, when hybridized to target, removes the affinity member and opens access to the target by the shorter homology region. Hybridization of the shorter homology region to target abuts the two homology regions providing a ligase substrate. The Figures present ribonucleotide interrogators subject to 5' and 3' cutting RNase, respectively.

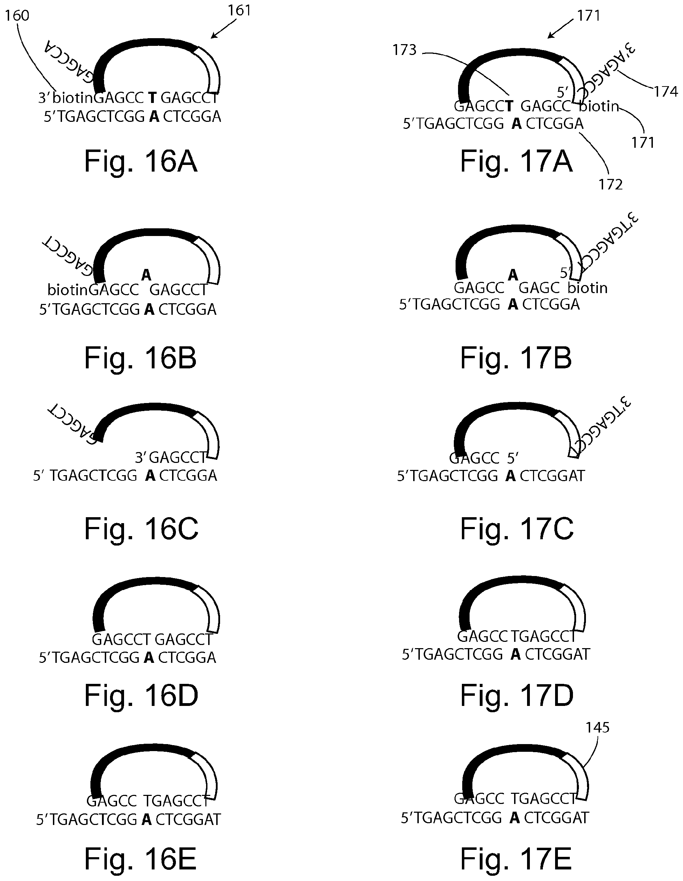

Figures 16 and 17 show schematic diagrams of MIPs having affinity pair members and a cleavage target interrogating base in the longer homology region, wherein the interrogating base is configured to be a mismatch endonuclease substrate. Figure 16 presents a scheme to identify a SNP using a 5' cutting endonuclease and Figure 17 a 3' cutting endonuclease. Specific cleavage, when hybridized to target, removes the affinity member and opens access to target by the shorter homology region. Hybridization of the shorter homology region to target abuts the two homology regions providing a ligase substrate.
Figure 18 a schematic diagram showing how a MIP with an interrogating homology region with non-hybridizing flap can be used in a scheme to employ MIPs not requiring a gap-fill polymerase step using a flap endonuclease. The flap has an affinity member useful in removal of the probe if it has not hybridized to target and become circularized through ligation.
Figures 19 to 21 present tables of enzymes and substrates useful for configuration of specific cleavage sites in MIP probes.
Figures 22 A to C are schematic diagrams showing an initial MIP before hybridization to a target, the MIP that has been circularized while hybridized to the target, and the MIP after cleavage generating a linear product having PCR primer binding sites on each end.
Figure 23 is a schematic diagram showing 6 different MIPs, each with its own tag identifier, hybridized to genomic DNA in three separate samples.
Figure 24 presents a table of data indicating how 6 tagged probes can unambiguously provide a SNP genotype for three different samples.
Figures 25 A and B show how a junction probe set can be used to confirm a junction between a homology region and a tag sequence.
Figures 26 A to C show how a pair of 5' interrogating oligomer ligation assay (OLA) probes and a 3' probe can be used to identify a SNP. In Figure 26A, only the 5' probe complimentary to the SNP base can align with the 3' probe to form a ligase substrate. Figure 26B shows the ligated product. Figure 26C shows how sample and SNP identifying information can be encoded onto the ligated product during PCR amplification using specially encoded PCR primers that introduce an Animal Barcode (ABC) and a SNP Barcode (SBC).
Figure 27 is a schematic diagram showing a process sequence wherein a MIP is hybridized to a target nucleic acid to form a MIP product configured for a preliminary PCR amplification. The PCR product receives adaptors appropriate for any desired next generation sequencing (NGS) protocol.
Figure 28 is a schematic diagram showing optional PCR primers that can be used for one or more rounds of MIP product amplification and to incorporate NGS adaptors.
Figure 29 is a flow diagram presenting options for processing of MIP assays with a microarray and/or NGS detection step, with potential use of the same MIP assay product with both detection approaches.
Figure 30 is a schematic diagram showing the incorporation of an additional cleavage site in the MIP for assays adapted for both NGS and microarray readouts in order to remove excess segments such as NGS adapters that may interfere with the microarray readout.
Figure 31 is a schematic diagram showing how stochastic probes can be used to quantitate alleles in a sample.
Figure 32 is a schematic diagram showing sequential steps converting a linearized MIP product to a circular nucleic acid comprising a unique barcode junction.
Figure 33 shows an agarose gel comparing side by side PCR products from 3000-plex and 10x300-plex MIP assays.
Figure 34 presents charts comparing results of exemplary AXIOM^{®} SNP array results for samples reacted to a 3000 MIP probeset (pure) versus samples reacted to the same MIPs but in 10 separate probe sub panels (pooled).

### DETAILED DESCRIPTION

Disclosed herein are methods and compositions for improving and utilizing molecular inversion probe (MIP) technologies. A number of methods and compositions are discussed in the Summary of the invention and further details are provided herein and in the Examples section. As would be readily appreciated by the skilled person, the disclosures can be read in combination. Solutions are provided to problems in ligation of MIPs resulting from errors in the gap-fill reaction. Cleavage sites and tags of MIPs are described that aid in ligation efficiency and confirmation of hybridization to a target of interest. Modified MIPs are described, which facilitate multiplexed detection of, e.g., more than one allele in more than one sample, in a single detection step.

In a typical MIP method, the target nucleic acid sequence of interest is previously known. Often MIPs are provided in pairs to detect the presence of alternate alleles, such as single nucleotide polymorphisms (SNPs) at a particular position in a target nucleic acid sequence of interest. MIPs are designed to have sequences (discussed as "homology regions" herein) complementary to the target nucleic acid sequence of interest, but wherein the complementary probe sequences are split to two subsets at opposite ends of the probe. On hybridization to their targets the MIPs typically bind with, e.g., the 5' end of a first homology region bound to target, accessory regions of the MIP not bound to target but looping around to a region further 3' on the target, and the second homology region hybridized 3' from the first homology region, e.g., with the 3' end of the MIP near or abutting the 5' end of the first homology region. With the MIP looped around and its ends in close proximity on the target, the ends can be joined to form a single strand circle, e.g., by polymerase activity to fill any gap along the target between the ends and ligase activity to covalently join the ends. The single stranded circular MIP reaction product has unique character and can be readily detected against the background of other nucleic acids that may be in the sample and reaction.

One common problem in the MIP reaction is that a polymerase used to fill any gap between MIP ends on the target can occasionally push away the hybridized 5' end of the probe in a strand displacement that allows the polymerase to continue reading through, resulting in an undesirable overextension "flap" that interferes with ligation and circularization. Described herein are methods and compositions increasing, e.g., the sensitivity and quantitative accuracy of MIP assays by avoiding the inefficiencies of failed circularizations.

Another inefficiency in certain MIP reactions can be in the separation of circularized MIPs from linear MIPs (e.g., MIPs that did not hybridize to their intended target). Such unreacted MIPs can interfere with subsequent assay steps, raise background signal, and/or lead to non-specific signal in certain MIP procedures. In procedures wherein non-circularized nucleic acids are digested for removal, some circularized MIPs may be lost due to side reactions or undesirable nuclease contaminants in certain reagents. MIP constructs and processes are described herein to provide improved clearance of interfering nucleic acids from the circularized MIPs.

MIP reaction products are excellent intermediates for input into any number of further amplification and/or detection schemes. The typical linearization step in MIP reactions can include cleavage by hydrolysis at a strategically positioned uracil. For example, uracil-N-glycosidase (UNG), can attack the N-glycosylic bond between the deoxyribose sugar and the uracil creating an abasic site sensitive to cleavage by heat and/or enzymes. However, where such a cleavage may create issues for downstream process steps (e.g., certain PCR protocols), alternate cleavage schemes are presented herein. In addition to PCR, certain MIP embodiments utilize alternative amplification approaches, such as rolling circle amplification with respect to the circularized MIPs.

We have found MIPs can be adapted to provide input material for a variety of amplification and detection schemes, while retaining their many advantages in probing, e.g., complex and dirty samples. Information can be encoded into the nucleic acid sequences of MIPs to allow multiplexed processing and unique detection of specific signals associated with particular samples in complex mixtures.

It is notable that the methods and compositions mentioned above and described in detail herein are complementary and can be used in unique combinations to provide compounded benefits. For example, the methods and compositions for enhancing ligation efficiency can be used in combination with methods of enhancing isolation of circularized probes, alternate linearizations, tagging and identifying techniques, and/or multiplexing schemes.

### Methods and Compositions for Enhancing MIP Circularization.

Because of the complexity of many samples, and the imperfections of many reagents, the important step of ligating MIPs hybridized to targets can suffer inefficiencies that reduce signal and/or sensitivity, provide inadequate specificity for certain target nucleic acids of interest (e.g., rare alleles in a mixed sample of normal and tumor cells), or increase background noise in an assay. We have found that gap-fill and ligation reactions can be improved by reducing the amount of strand displacement reactions, removing the undesired products of strand displacement, and/or providing MIPs adapted to be specifically circularized without the need for a gap-fill reaction step.

One effective strategy to overcome gap-fill inefficiencies and errors can be to prevent polymerases from ever gap-filling beyond the 5'-3' MIP gap on the intended target. For example, the activity of certain polymerases can be enhanced by inclusion of NTP analogs in the gap-fill reaction mixture, where the analogs function to prevent the polymerase from adding nucleotide bases past those required to fill the gap. For example, a gap-fill reaction can include one or more natural NTPs that complement the one or more target bases in the gap between the MIP 3' and 5' ends. Useful NTP polymerase stop analogs can have the Formula I:

NTP - Tether - Inhibitor

Wherein NTP is a nucleoside triphosphate or an analog thereof capable of incorporating onto the 3' end of a polynucleotide strand hybridized to a template presenting the complement of the NTP; the inhibitor comprises a group that blocks further extension from the 3' end of the NTP; and the tether is a linker group or simple covalent bond. In many embodiments, the tether is adapted to allow ready removal of the inhibitor group, e.g., by chemical, physical and/or enzymatic separation of the linker from the NTP. In certain embodiments, the inhibitor is charged or capable of becoming charged, e. g., under reaction conditions, rendering the NTP an unsuitable substrate for the polymerase and inhibiting a subsequent incorporation of a nucleotide (or analog thereof).

In other embodiments, the stop NTP is simply an NTP analog with a modification of the pyridine or pyrimidine base rendering it unrecognizable by the polymerase. For example, suitable NTP analogs to prevent strand displacement can be analogs with modifications at C7 or adenine or guanine, or the C5 of uracil, thymine, or cytosine. The modifications can be, e.g., addition of an -OH group, -O-P(O)(OH)₂, -O-C(O)-Rx, -NHRy, and an -O-blocking agent, where Rx and Ry are alkyl groups. Preferred modifications include -COOH, -PO₄, -SO₄, -SO₃, and -SO₂. In many cases, the 3'OH of the analog is unmodified, and the 3' analog extended end of the MIP remains a proper substrate for subsequent ligation (e.g., ligation with the 5' end of the MIP).

Non-limiting examples of representative purine and pyrimidine bases for stopping polymerase over extensions include analogs of adenine, cytosine, guanine, thymine, uracil, or hypoxanthine. Non-limiting examples of derivatives of purine and pyrimidine bases include naturally-occurring and synthetic derivatives of a base, including pyrazolo [3,4-d] pyrimidines, 5-methylcytosine (5-me-C), 5-hydroxymethyl cytosine, xanthine, hypoxanthine, 2-aminoadenine, 6-methyl and other alkyl derivatives of adenine and guanine, 2-propyl and other alkyl derivatives of adenine and guanine, 2-thiouracil, 2-thiothymine and 2-thiocytosine, 5-propynyl uracil and cytosine, 6-aZo uracil, cytosine and thymine, 5-uracil (pseudouracil), 4-thiouracil, 8-halo (e.g., 8-bromo), 8-amino, 8-thiol, 8-thioalkyl, 8-hydroxyl and other 8-substituted adenines and guanines, 5-halo particularly 5-bromo, 5-trifuoromethyl, and other 5-substituted uracils and cytosines, 7-methyl guanine and 7-methyladenine, 8-aza guanine and 8-azaadenine, deazaguanine, 7-deazaguanine, 3-deazaguanine, deazaadenine, 7-deazaadenine, 3-deazaadenine, pyrazolo [3,4-d] pyrimidine, imidazo [1,5-a]1,3,5 triazinones, 9-deazapurines, imidazo [4,5-d] pyrazines, thiazolo [4,5 -d] pyrimidines, pyrazin-2-ones, 1,2,4-triazine, pyridazine; and 1,3,5 triazine.

In practice, the gap-fill reaction can include an analog of the intended target nucleic acid complement. Once the polymerase has incorporated that analog, polymerization stops and the MIP probe is the proper length with 5' and 3' ends abutting. In some cases, ligation may require treatment of the new 3' end to render it a proper substrate for the ligation reaction. In other instances, the ends may be ligated without interference from the one or more untreated base analogs.

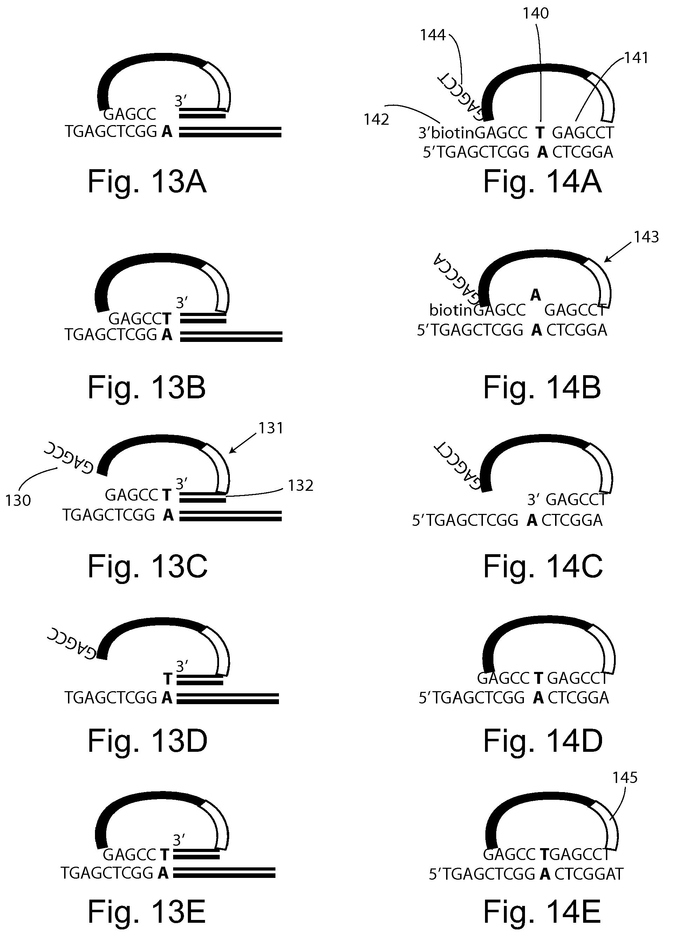

Another effective strategy is to design methods and components to accurately fill the gap between a MIP's 3' and 5' ends with natural NTPs, but arrange that any extension beyond the gap be readily removable. For example, a gap-fill reaction can include 4 bases, but with only one base type being a dNTP corresponding to the complement of the target in the gap between the MIP's ends. In such a modified approach, the NTPs being used for the other base types are, e.g., ribonucleotide triphosphates (rNTPs). The proper NTP will be permanently incorporated across the gap. A poorly selective polymerase (e.g., see Patel, J. Biol Chem. 275(51): 40266) may continue polymerizing with the rNTPs in a strand displacement, creating a flap not subject to the ligase reaction. In order to establish conditions for the ligation, an RNAse enzyme can be added to the reaction to remove the ribonucleotide bases, without removing the desired deoxyribonucleotide base from the gap. See, e.g., Figure 13. This approach can also be utilized to increase the gap fill efficiency of assays utilizing highly selective polymerases, as even these enzymes may still add one or more additional bases during the gap fill reaction and inhibit subsequent ligation and circularization of a MIP that has hybridized to a target.

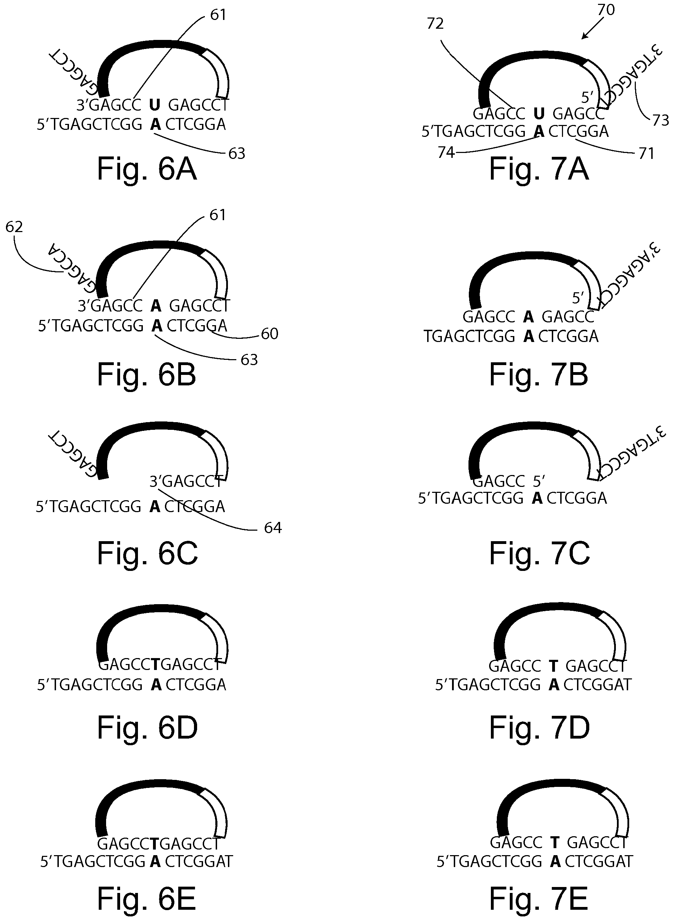

The strand displacement problem can also be addressed by adjusting the MIP to include a cleavable element at the point of target sequence interrogation to provide a ligatable product without the requirement of a gap-fill reaction. For example, the MIP can be designed with a standard first homology region, but with a larger second homology region that extends into the complementary target of the first homology region, thus blocking the first region from hybridizing to the target (see, e.g., Figure 6). Additionally, the second homology region can be designed to provide a higher melting temperature compared to the first homology region, thus allowing for hybridization of the MIP to its corresponding target (if present) at an elevated temperature that will favor hybridization of the longer second homology region over the shorter first homology region. The MIP second homology region can have a base subject to cleavage at or near the position corresponding to a target base of interest (e.g., a SNP). If the target base of interest exists on the target, a substrate for a cleavage enzyme (or cleavage chemistry) is formed on hybridization of the second homology region to the target. This specific cleavage can permit the first homology region to banish the resulting second homology region fragment, e.g., to hybridize with its end abutting the newly cut end of the second homology region. The sequence of events produces abutting MIP ends hybridized to the target and providing a substrate for a ligase reaction.

As shown in Figures 6 and 7, the process of providing a gap-fill substrate can include strategic positioning of a ribonucleotide base in the second homology region, thus providing a substrate for, e.g., a ribonuclease H enzyme, to cut the region only if the target hybridizes to the second homology region at the ribonucleotide base. If the second homology region is cut, the blocking segment is lost and the first homology region can take its place on the target, with its end abutting the cut end of the second homology region, thus forming a ligase substrate. On ligation, a circularized MIP is formed and subsequently detectable as confirmation of the presence of the target sequence.

Ribonuclease enzymes are available to cut on either the 3' or 5' side to the hybridized ribonucleotide base. In Figure 6, the enzyme cuts on the 5' side of the ribonucleotide base, leaving a 3' cut end in the second homology region. Alternately, as shown in Figure 7, the enzyme cuts on the 3' side of the ribonucleotide base, leaving a 5' cut end in the second homology region (shown on the left side of the schematic diagram of the Figure). Thus, either the 5' or 3' end of the MIP can have the longer homology region that prevents the other end of the MIP from hybridizing with the target.

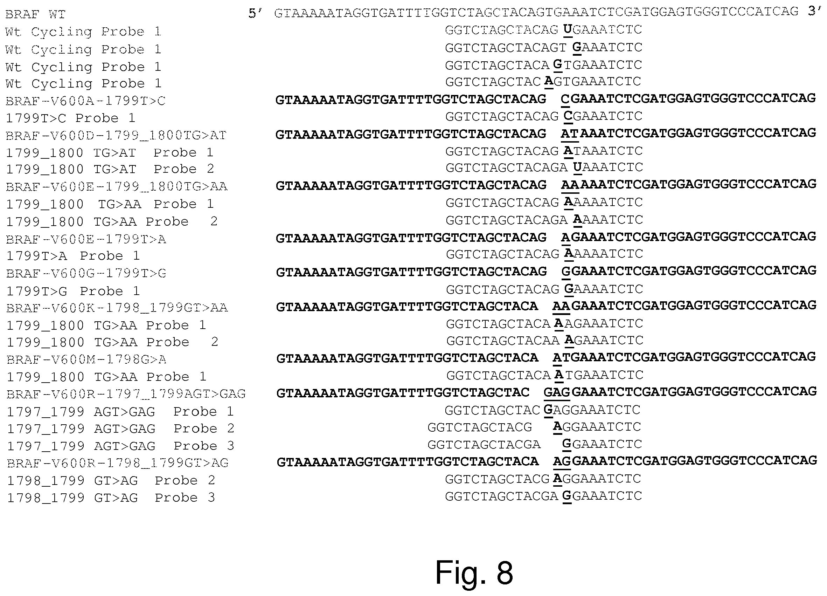

Figure 8 provides exemplary sequences for the longer ("second" in description above) homology regions complementary to a variety of target variants of BRAF (otherwise known as v-Raf murine sarcoma viral oncogene homolog B1). The longer sequence of the second homology region includes, e.g., at least a portion of the same sequence that would be present in the shorter first homology region. Because the longer region has a higher Tm it can effectively exclude the shorter region until the cleavage step under appropriate hybridization conditions (e.g., an elevated hybridization temperature). Note, MIPs can be designed to have the shorter region be the sequence on either side of the interrogating base (e.g., ribonucleotide base), while the longer region can include the full sequence on both sides of the interrogating base. Where the target sequence of interest includes multiple mutations from the wild type, this can be unambiguously identified using multiple MIPs. Note the probe and target are same strand, to simplify presentation. However, in practice the probes would hybridize to the complementary target strand, or the complement of the probe could hybridize with the target strands presented.

In a similar fashion, the scheme above can work with a cleavage target other than a ribonucleotide hybrid. For example, the general process of specific binding with a long sequence, specifically cutting at a hybridized (or even mismatch) allele and abutting replacement with a shorter MIP homologous region arm can be practiced using any number of enzymes or chemistries that can cut the long region specifically at a site determined by the allele of interest.

Enzymes are available that cut at or near mismatches in hybridized nucleic acids. For example, celery mismatch endonuclease (CEL I), endonuclease V, glycosylase TDG, glycosylase MutY, AP endonuclease/lyase, T4 endonuclease VII, T7 endonuclease I, deoxyinosine 3'-endonuclease, mung bean nuclease, resolvases, and/or the like.

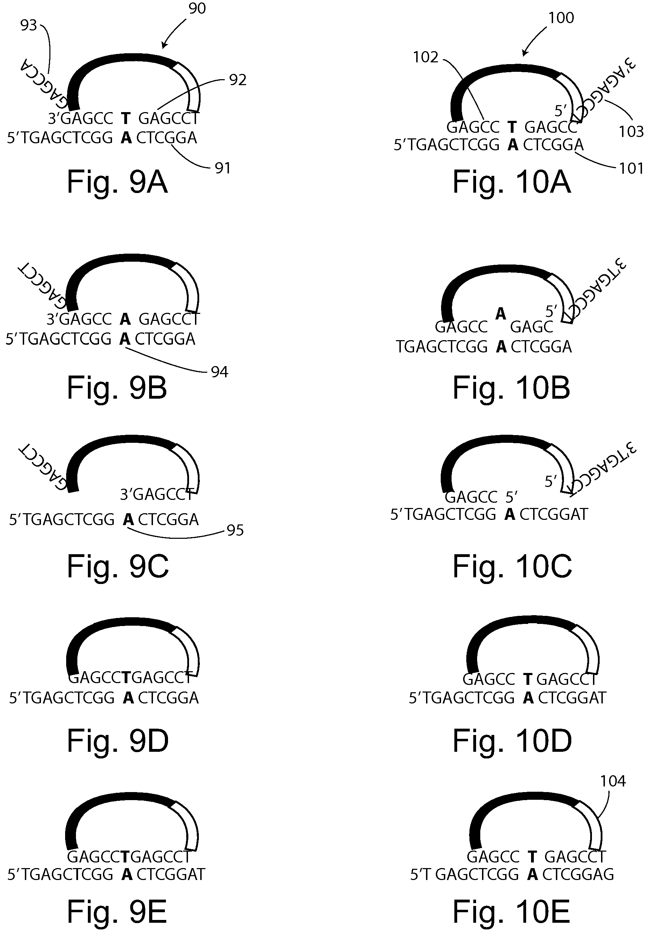

In the context of mismatch endonucleases, the cut occurs when there is a mismatch and the long region does not perfectly hybridize with target. So, the test results are interpreted a little different from the RNase method. In the RNase method above, a circularized MIP signal indicates the presence of a match between the interrogating ribonucleotide base and the target DNA base. In the mismatch detection method a circularized MIP is generated where the interrogating MIP base within the longer homology region does not match the target (see, e.g., Figures 9 and 10). It is a simple matter to interpret the results, e.g., for a SNP detection where the wild type is "G" and the SNP is "C". The sample can be probed with a pair of MIPs that have a second homology region with interrogating bases of "C" and "G", respectively. If the MIP containing the "C" interrogating base is circularized, this suggests that a mismatch was present, and apparently "C" was present at the interrogation position in the target. This is corroborated by a lack of a circularized MIP signal from the "G" containing probe that must have hybridized with the target "C", without generating a mismatch enzyme substrate (thus preventing circularization).

Mismatches can also be detected by chemical means. For example, the present procedures can be slightly modified to take advantage to chemical cleavage reactions such as use of DNA intercalator/photoactivation; hydroxylamine/ permanganate/piperidine, osmium tetroxide/piperidine; and/or the like. Typically, a chemical reaction attacks a particular mismatched base, and a second reaction specifically cleaves the adjacent sugar. Again, as with enzymatic mismatch detection described above, generation of a circularized MIP signal suggests a particular mismatch, and it can be corroborated by the lack of a signal from a corresponding MIP for the target region but without a mismatch at the interrogation position.

In many of the methods described herein, a preexisting MIP structure (such as the end of a long homology region) is removed during the process of hybridizing the MIP to target. These methods can be carried out using similar MIPs but with an affinity group attached to the fragment ultimately removed before circularization of the probe. Remaining MIPs that have not been hybridized with the target nucleic acid and subsequently circularized through ligation retain the affinity group and can be captured and removed by the appropriate affinity partner.

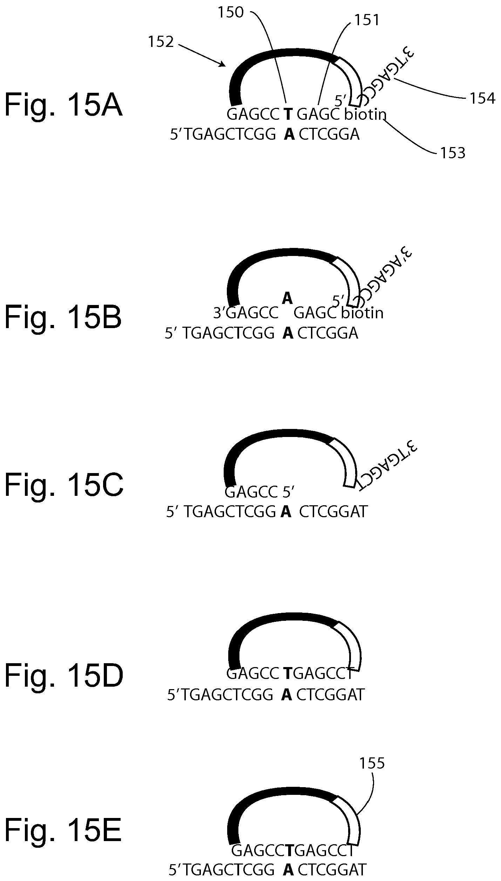

In a typical embodiment, the MIP is designed to include a first member of an affinity pair at an end of the MIP that would be removed, if the MIP has hybridized to a target sequence of interest. For example, a MIP can have a longer homology region that ranges across all of the target sequence of interest, and have an affinity group at or near the end. The other MIP homology region covers a shorter segment of the target sequence, and thus is blocked from binding to target by overlap with the stronger binding longer homology region. As shown in Figure 14, the longer homology region can include an interrogation base (there, a ribonucleotide subject to 5' cutting RNase activity) subject to specific cutting depending on whether or not it is complementary to the target. Should the target include an appropriate interrogated base, this provides a substrate for specific cutting and the end of the longer homology region will be cut away: 1) releasing the affinity group from the MIP, and 2) allowing the shorter homology region to hybridize to target, e.g., abutting ends with the remains of the longer homology region. The cut away end of the longer region can be adapted to have a lower Tm than the shorter homology region to facilitate binding of the shorter homology region to its segment of the target. Thus, the specific cutting aligns and abuts MIP ends for circularization and removes the affinity group from the remaining portion of the MIP is subsequently circularized. Figure 15 shows a similar process, but using an RNase that cuts 3' of the interrogating ribonucleotide of the longer homology region. The unique combination of MIP structures and specific cleavage work in concert to efficiently provide both an analyte-specific ligation and a trouble free way to remove unreacted MIPs from the reaction mixture and simplify subsequent analysis.

Longer and shorter are relative terms, and they are relative to each other. That is, if a first homology region (complementary to the target of interest) is longer (e.g., comprising more nucleotides) than a second homology region (e.g., on the other end of the MIP) the "longer" homology region is the first homology region, and the second homology region is the "shorter" homology region. If the longer homology region is subsequently cut in a process step, it can continue to be designated the longer homology region for clarity and consistency. The "longer" homology region is intended to bind more stringently (e.g., with a higher Tm) to the target. The longer homology region can have, e.g., 5% more bases complementary to the target than the shorter homology region, 10%, 20%, 50%, 100%, or 200% more bases complementary to the target.

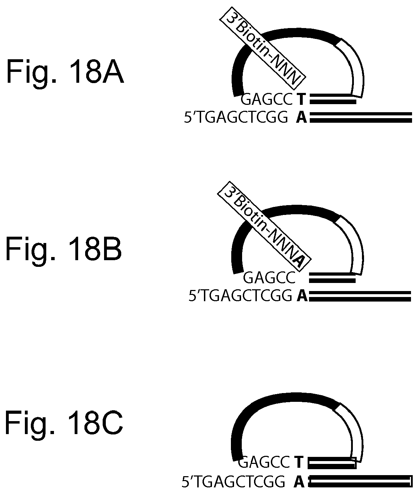

Affinity removal of unreacted MIPs can be practiced using MIPs configured to be specifically cut in other ways. For example, the longer homology region, e.g., terminated with an affinity pair member, can be designed to have a mismatch interrogation, and cut 5' or 3', e.g., cut with endonculease V, or with other enzymes or chemistries discussed herein. (see, e.g., Figures 16 and 17). Optionally, the end of the longer region does not compete with the shorter region for target sequence hybridization, but includes a flap not complementary to target, but presenting a substrate for specific cleavage, e.g., by a flap endonuclease or cleavase. The flap can be attached to an affinity pair member and be lost on specific cleavage. See, e.g., Figure 18.

Figure 8 provides exemplary sequences for the longer homology regions complementary to a variety of target variants of a B-Raf. The end of the longer homology region can include, e.g., the same target complement sequence that would be present in the shorter first homology region. Because the long region has a higher Tm it can effectively exclude the shorter region until the cleavage step. Note, MIPs can be designed to have the shorter region be the sequence on either side of the interrogating base (e.g., ribonucleotide base), while the longer region can include the full sequence on both sides of the interrogating base. Where the target sequence of interest includes multiple mutations from the wild type, this can be unambiguously identified using multiple probes.

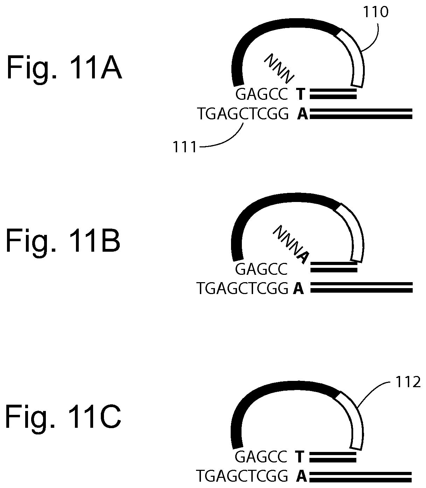

Another way to use an enzyme to confirm single base specific probe complementarity is by using a MIP with a prepositioned un-hybridizing flap substrate. There are flap endonucleases and cleavases that will only cut a flap if it originates at a hybridized base directly abutting another hybridized strand. For example, see cleavase endonuclease enzymes engineered from the nuclease domain of Taq DNA polymerase or those enzymes used in the INVADER TM assay. As shown in Figure 11, a MIP can be configured to have first and second homology regions perfectly complementary to a target sequence right up to abutting bases, while one homology region includes a further sequence generating a non-hybridizing flap. The cleavase enzyme will cut off the flap if only the first and second homology regions are abutting on the target, but not if there is a mismatch at the base of the flap. If the cleavase does cut the flap, the MIP can be ligated and circularized, signaling the presence of a complementary base at the intersection of the homology regions. If not, the flap will remain and no circularized product can be generated. Some flap endonucleases (FENs) only remove flaps starting at the first non-complementary base, e.g., competing with an underlying invading sequence (e.g., the homology region without a flap, typically the 5' end of the MIP). With these FENs, the flap to be removed would be designed to complement the target base at the same position as the end base of the other end of the MIP. If the first flap base complements the same base as the final MIP base, the FEN would cut and the two ends of the MIP will abut on the target as a ligase substrate.

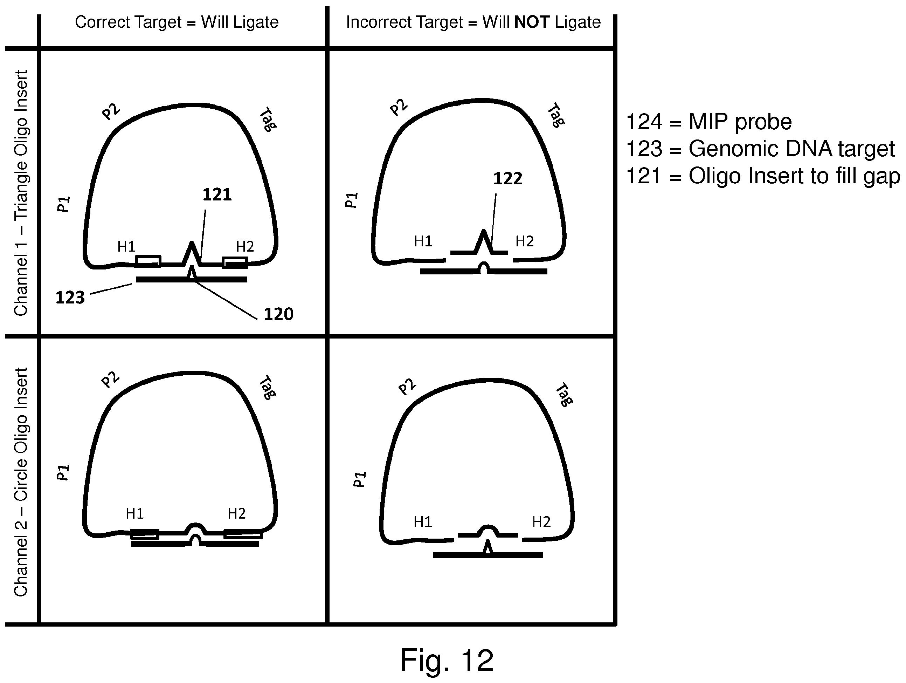

Another way to improve appropriate ligation and circularization efficiency in MIP analyses is to fill the gap between hybridized MIP homology regions with an oligomer insert probe under stringent hybridization conditions. For example, as shown in Figure 12, an oligomer probe can detect the presence of a particular sequence by binding under stringent conditions at a target sequence filling the gap between MIP probe homology regions. The 5' end of the oligo probe can abut the 3' end of the MIP probe and the 3' end of the oligo probe can abut the 5' end of the MIP probe when hybridized to the target. The oligomer probe can be adapted to hybridize to a perfect match but not hybridize and bind if there is even a single base mismatch. This can be accomplished, as known in the art, e.g., by adjusting the probe length, position of interrogator, GC percentage, hybridization conditions, and/or the like, so that hybridization (or extent of hybridization) can be distinguished between perfect complementarity and a single base mismatch. With both the oligomer probe and MIP hybridized to the target, there is no need for a gap-fill polymerase reaction, and no potential for strand displacement and flap generation. With the oligo ends and MIP ends adjacent on the target, ligase can circularize the MIP, e.g., for detection according to techniques described herein.

The oligomer insert probe can range in length from 4 bases to 30 base or more, 5 bases to 25 bases, 6 bases to 20 bases, 7 bases to 15 bases, 8 bases to 10 bases. It is preferred the interrogator nucleotide(s) be near the center of the probe, but it may be located anywhere along the length of the probe. As discussed above, it can be important to detect the presence of even a single base mismatch between the target and the oligomer insert probe, so positioning of the interrogator nucleotide(s) in a position where any mismatches would most destabilize the duplex between the target and oligomer insert probe is favored in many embodiments. The oligomer insert probe is can be 100% complementary to the target sequence, or mismatched at one or more bases, e.g., where the position has an alternate allele (e.g. SNP) at the interrogator position. The oligomer insert probe can be used to interrogate a single base position, such as a SNP, or a particular region for another polymorphism of interest (e.g., simple or complex indels). Optionally, percent complementarity can be adjusted to meet melting temperature constraints of all reaction members and to optimize specificity of the signal.

### Methods and Compositions for Removal of Non-Circularized Probe from a MIP Reaction.

As discussed above, a typical method for removal of non-circularized nucleic acids (target nucleic acids and MIPs that did not become circularized) from a completed MIP reaction is to digest any non-circular nucleic acids with a cocktail of exonucleases. However, as these nucleases are not always free of undesirable contaminants, some circularized product can be destroyed and some corrupting nucleic acids can remain, reducing the sensitivity of the assay and potentially leading to false positives. We describe here methods and compositions to remove unreacted MIPs while leaving circularized MIPs for further processing and detection.

MIPs for use within assays that employ non-enzymatic digestion methods of non-circularized MIP removal typically have an affinity pair member at or near one end of the probe and a cleavage site at or near the MIP interrogator base(s). For example, the MIP can include in order: affinity member, longer homologous region (e.g., target complement 1, interrogator nucleotide, target complement 2), accessory sequences (e.g., tags, cleavage sites, primer binding sites, etc.), and the shorter homology region. MIP probe segments in order can each be in direct contact in sequence or can have other unnamed sequences between as long as the sequence of listed elements is sequential along the length of the probe.

In practice, the unreacted MIP can have one member of an affinity pair and the other affinity member can be adapted to separate unreacted MIPs from the reaction mixture. Affinity pairs can be any pair with the affinity and specificity adequate to substantially reduce interference with a given assay, e.g., so that desired resolution, accuracy, sensitivity, and/or quantitation parameters can be met. In one aspect, the affinity pair is biotin/avidin. Typically, the biotin affinity pair member is covalently bound to the MIP and the avidin is attached to a substrate (e.g., solid support or precipitatable molecule) readily removable from the reaction. A non-limiting example of such a pair would be a MIP that has a biotinylated nucleotide that would be used with streptavidin coated magnetic microparticles. Other examples of affinity pairs that can be adapted to remove unreacted MIPs can include, e.g., metal groups and chelators, antigen and antibodies, nucleic acids and nucleic acid binding proteins, complementary nucleic acids, reactive chemical groups (e.g., linker groups and their targets), hydrophobic groups and lipid substrates, and/or the like.

### Methods and Compositions for Opening Circularized MIPs.

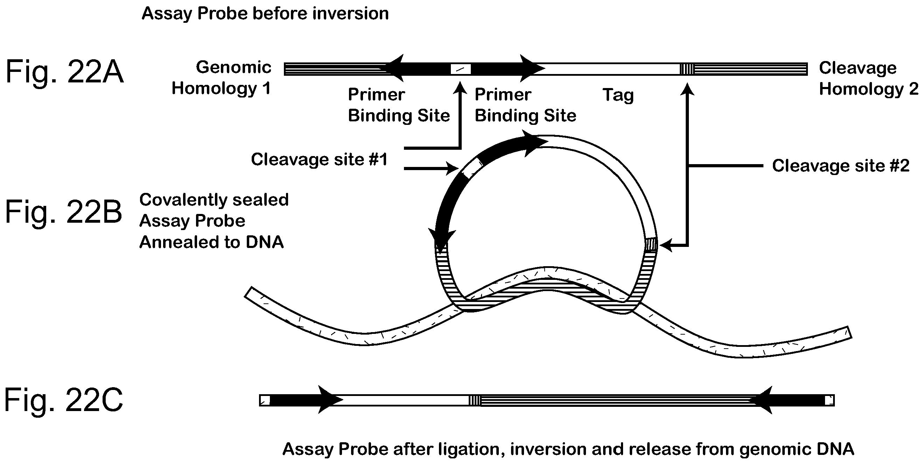

In many MIP assay procedures, circularized MIPs are later linearized to complement certain desirable detection schemes. For example, MIPs often incorporate binding sites for forward and reverse PCR primers, with a cleavage site in between (e.g., see Figure 22). Commonly, the cleavage site between the PCR binding sites is a set of one or more uridine deoxyribonucleotides (dUMP) in the MIP backbone, acting as substrate for Uracil-N-Glycosylase (UNG). However, depending on the nature of other cleavage sites in the MIP reaction, or cleavage steps used in downstream detection, it may be advantageous to employ alternate cleavage sites in the MIP and/or cleavage mechanisms.

As discussed in Example 6, below and outlined in Table Figures 19 to 21, we have identified many alternate cleavage schemes that can complement processing requirements for MIPs.

For specific cleavage, MIPs can incorporate particular nucleotides subject to base removal. Thereafter, the abasic position will be subject to backbone enzymatic, chemical, or physical cleavage. For example, incorporated methyladenine, thymine mismatch, or hypoxanthine can be rendered abasic, e.g., by a 3-methyladenine-DNA glycosylase, thymine mismatch-DNA glycosylase, alternate uracil DNA glycosylases, or hypoxanthine DNA N-glycosylase. See table at Figure 19. Specific enzymes useful in cleaving at abasic sites include, e.g., endonuclease IV, APE 1 AP endonuclease, exonuclease III, endonuclease IV (nfo), and/or the like.

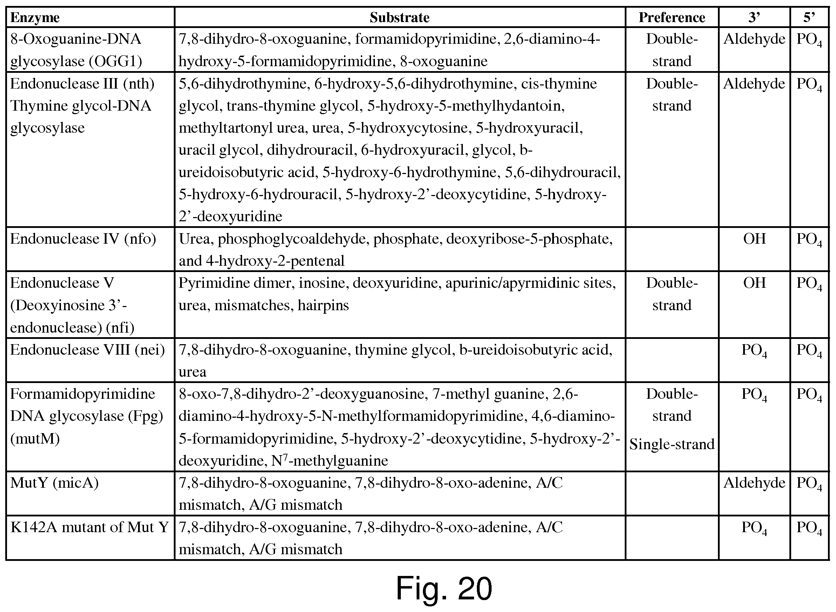

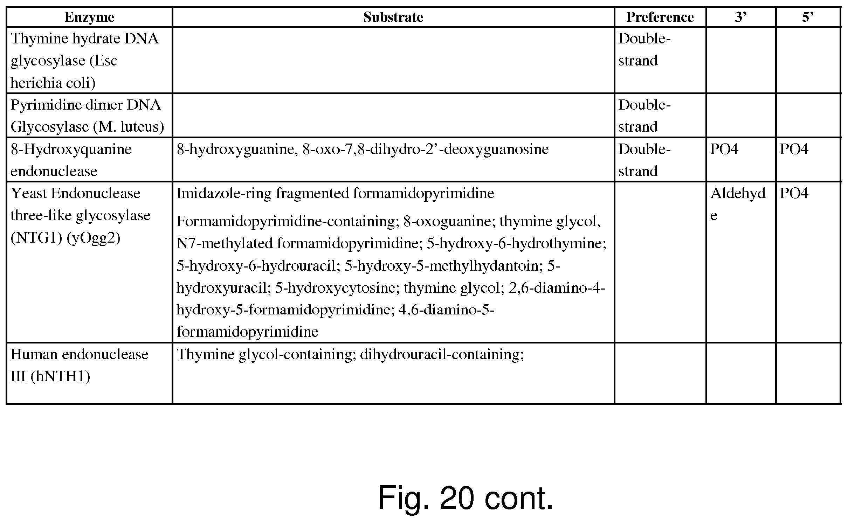

Alternately, there are several bifunctional DNA glycosylases that not only remove bases, but can also break the backbone. For example, various nucleotide analogs can be attacked and cleaved by 8-oxoguanine-DNA glycosylase, various endonucleases, and glycosylases. See table at Figure 20.

These alternate cleavage methods and compositions can complement the other concepts, such as gap fill improvements, gap-fill avoidance techniques, affinity removal steps, and multiplex assays described herein. For example, a MIP can include a shorter homology region, first primer binding site, first cleavage site, second primer binding site, information tag, second cleavage site, longer homology region, and/or affinity group. The first and or second cleavage site can comprise one or more of the cleavage sites above, to serve as a substrate to the enzymes referenced above.

### Multiplexed Detection of MIP Reaction Products.

Information can be encoded into MIPs so that any detectable signal they provide can be assigned to, e.g., a particular sample and/or a particular allele. In this way, MIP products from several samples can be unambiguously detected in a single detection event. For example, one or more tag elements can be incorporated or attached to MIP probes. The one or more tag elements can include information, such as within a "barcoded" nucleic acid sequence, which is readable by, e.g., a sequencing procedure or specific hybridization. Alternately, the one or more tag elements can be an affinity moiety that can specifically bind the MIP to an identifiable array location.

In practice, the probe can include an identifiable tag sequence that is initially located between homology regions, e.g., as shown in Figure 22A within the initial linear form of the MIP before hybridization to a target. The tag identification (ID) sequence can be assigned to a particular sample. Different tags IDs on other MIPs can be assigned to other samples. The different MIPs can each be added to interrogate their assigned samples. If the sample includes a nucleic acid complementary to the MIP homology regions, the probe will be inverted, as shown in Figure 22B. The probe can be gap-filled, ligated, and separated from non-circularized probe, as appropriate for the probe design (e.g., the gap-fill step may be omitted in alternative embodiments that replace the gap-fill with an allele specific homology region cleavage step). At this point, the various different sample reactions can be combined for common detection. The tag information is retained through processing and any detected signal from each circularized MIP can be assigned during analysis back to the sample to which they were originally added.

An important aspect of multiplexed detections is the presence of the tag in the MIP probe. In many cases the tag is a unique nucleic acid sequence that is readily identifiable by any number of detection methods. For instance, the tag can be interrogated by a nucleic acid array having polynucleotide probes that are complementary to the various tag sequences. For example, the tag can include a sequence complementary to a capture probe of a bDNA procedure. bDNA capture probes can be laid out in an array on a solid support with each location corresponding to a particular tag and sample. Optionally, the tag could correspond with a bead having appropriate capture and signaling elements, e.g., for detection in a FACS flow device or by imaging with a charge coupled device. Optionally, the identity of a MIP assay product sample source and/or associated target sequence can be determined by a DNA sequencing procedure.

In a non-multiplexed system, suppose the wild type at a position is a cytosine (C) and a known SNP position of interest at that position is adenine (A). To determine genotype, a sample could be split into two aliquots. The first aliquot could be probed with a first MIP having a thymine (T) at the position, and the second aliquot could be probed with a second MIP having a guanine (G) at the corresponding position. If the sample had the SNP, the aliquot probed with the first MIP having a T would produce a circularized MIP product. If there were also a signal from the second aliquot, investigators would know the genotype includes both wild type and SNP allele. However, if both probes were used in the same sample, the genotype could not be determined unless the first and second aliquots were analyzed separated (e.g., splitting the sample into two channels (A/T and C/G) for assay processing and analysis) or if the genotype was separately determined later in the assay.

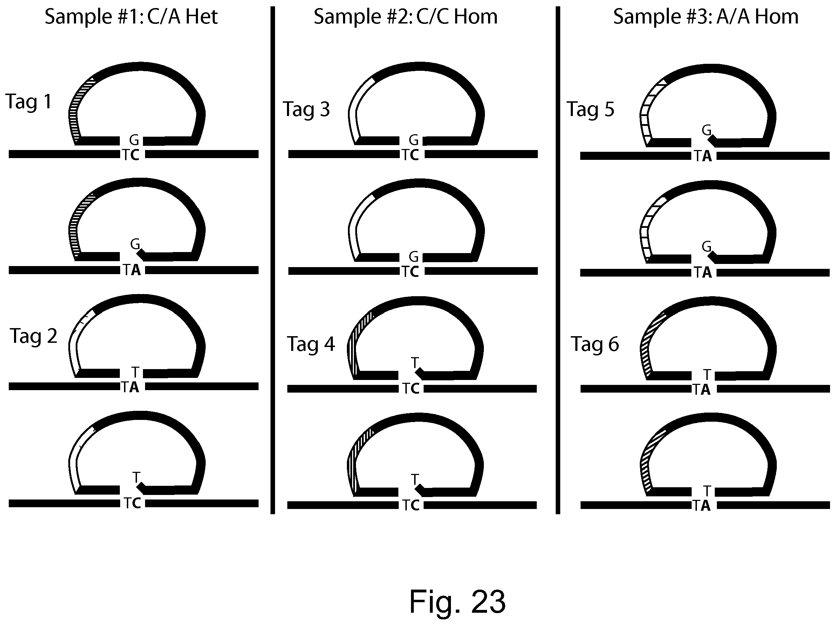

In order to allow detection of more than one allele from more than one sample in the same detection step, MIP probes with identifying tags can be used. In a basic version of multiplexed MIP genotyping, the wild type/SNP genotype at a certain position can be determined in a single detection step for any number of samples, e.g., using two MIPs with different tags in each sample. For example, as shown in Figure 23, for each of three samples a G MIP with a first tag and T MIP with a second different identifiable tag are introduced into each sample, for a total of 6 MIPs and associated tags. Once the hybridization, gap-fill, and ligation steps are complete for each sample, any uncircularized MIPs are removed from each sample reaction. At this point, the MIP probe products from all three samples can be combined in a batch detection step.

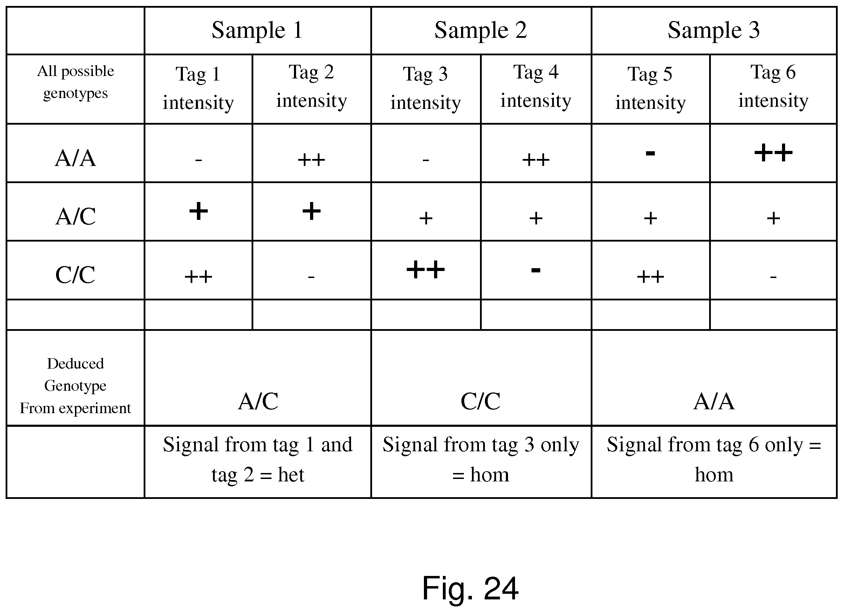

The presence and intensity of tag signals (e.g., from a hybridization based interrogation of the tag sequences) from the combined MIP probe products can be interpreted to identify the genotype originally present in each sample. For example, as shown in Figure 24, a moderate (or roughly equivalent) signal from both MIP probes from sample 1 confirms the sample has, e.g., a heterozygous genotype with both wild type and SNP alleles. No signal from one MIP tag, but a strong signal from the second MIP tag from a sample confirms a homozygous genotype, as in Figure 24 samples 2 and 3.

In one embodiment, tagging of MIPs can allow later pooling and determination of any of the four bases (A, T, G, C) interrogating the same target position, but reading from the same array. For example, a sample can be aliquoted to four separate MIP hybridizations. The MIPs each include a tag sequence that identifies the interrogated base as A, T, G, or C. Gap-fill/ligation reactions are then carried out in the presence of the appropriate dNTP (ATP, TTP, GTP, CTP) and uncircularized MIPs removed by exonuclease digestion. Alternately, in other contexts, the NTPs can be rNTPs, e.g., for incorporation by an RNA polymerase or poorly selective DNA polymerase. In some old art schemes, the reactions must remain separate because the detecting capture array is specific to the target but not the allele (e.g., SNP). However, with each MIP for each base having a distinct tag, a capture array can be provided wherein each of the four possible MIP reaction products can be captured at a different location. In this way, after circularization and removal of unreacted MIPs, capture of the MIP reaction product at separate A, T, G, or C locations for the target can confirm which one (homozygote) or two (heterozygote) alleles were in the sample; this using only one solid support capture array.

It is notable that the above information can optionally be gathered using only a single tagged MIP probe for each sample, without corroboration from a second probe. For example, assuming the only alleles at the position are the wild type and one known SNP, the genotype can be determined using either the wild type or SNP MIP probe. The presence of a first allele can be confirmed by a signal from the associated tag. Homozygosity or heterozygosity can be inferred by the intensity of the tag signal. For example, using only SNP tagged MIPs, a moderate signal would indicate a heterozygote, no signal homozygous wild type, and strong signal homozygous SNP alleles.

In the above examples, a single tag signal is associated with the combination of both sample and allele information. However, to increase the number of different signals available, MIPs can be tagged with more than one tag, e.g., a sample identifier and an allele identifier. At the detection step, a signal for a particular allele may show up more than one sample, but this can be deconvoluted by the association of a sample tag signal with each allele signal. For example, where the detection takes place on a physical array, a sample ID tag can be captured at a location assigned to the sample, and any number of allele tags detected at that location will be assigned to the sample. Optionally, a bead with a unique bar code (e.g., a nanorod stripe pattern or other optically encoded barcode or a fluorescent dye barcode) for an allele may capture all MIP products with the allele, while sample ID tag signals on the bead can be interpreted to confirm each of several samples that have the allele. Optionally, MIP reaction products can be sequenced (e.g., in parallel, at once using NGS), thereby confirming combinations of samples and alleles.

In many cases, the combination of a homology region sequence and sample ID tag is adequate to associate a sample with an allele. That is, the homology region sequence is often unique to an allele of interest being interrogated. With the homology region itself acting as an allele tag, sample/allele combinations can be identified, as described above.

Optionally, a combination of a sample ID and allele ID can be detected as the unique junction of sample and allele encoding nucleic acid sequence. For example, the MIP detection of an allele in a sample can be confirmed by the presence of an allele tag/homology region junction in the MIP reaction product. A junction between the tag and homology region can be detected by nucleic acid sequencing or by stringent hybridization with an oligomer probe complementary to the junction.

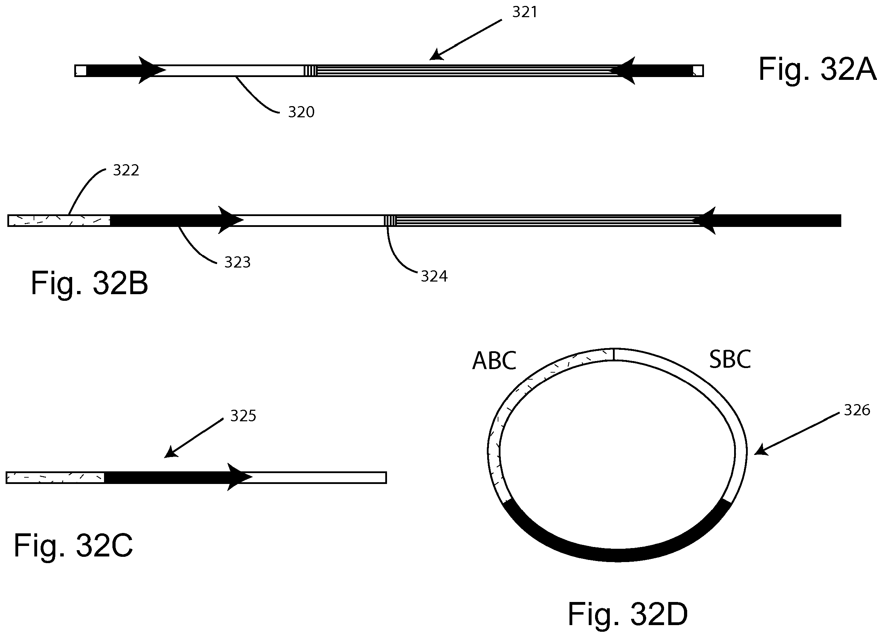

The sample/allele ID tag junction can be present initially in the MIP (e.g., with one homology region adjacent to the tag before circularization) or the sample ID tag can be incorporated during amplification of the MIP reaction product. For example, a sample can be probed with a number of MIPs having allele ID tags. The separated circularized MIP reaction products can be cleaved between a pair of PCR probe binding sites and amplified using PCR probes having a 3' extension encoding, e.g., the source organism (e.g., human patient, animal, plant) for the sample. Now, there is an amplified set of probes having tags for any allele that was present in the sample, and each is end labeled by PCR with the identity of the sample. The combination of sample ID (e.g., an animal bar code (ABC)) and allele ID (e.g., a SNP bar code (SBC)) can be read, e.g., on an array or by sequencing (see, e.g., Figure 32 and Example 8). Alternately, the amplified probes can be further processed to generate a unique detectable sample ID/allele ID junction, e.g., as discussed in Example 9. In the situation where the tag is adjacent to a homology region, there would commonly be no other portions of the MIP in-between the two regions, but in other embodiments the two regions be separated by one or more bases but still be sufficiently adjacent for further processing and detection of the junction.

In certain cases, the detection sensitivity and accuracy can be enhanced by separating the informative tag from the no longer required segments and regions of the MIP product. For example, removal of superfluous probe segments can reduce false positive hybridizations in complex mixtures and enhance reaction kinetics. Once a MIP reaction is complete, and the product has been linearized and supplemented with the addition of a sample source code to the probe end, portions of the MIP that are no longer necessary can be eliminated from the reaction mixture that actually undergoes the detection/processing phase of the assay, while retaining the sample/allele information in the one or more tags. For example, for MIPs that only initially include one tag, such as depicted in the linearized MIP of Figure 22C, a sample ID tag can be amplified to add an allele ID tag to the primer binding site, then the probe can be cut at the second cleavage site to free a fragment comprising the sample and allele information, e.g., for detection by sequencing, hybridization (e.g., junction probe set), array capture, and/or the like. Further, the released informational fragment can be recircularized to bring the sample ID and allele ID tags in contact at a uniquely identifiable junction.

Another way to generate a probe product subject to detection schemes described herein is through oligonucleotide ligation assay (OLA) technology. The OLA reaction product can include allele and sample tags detectable by, e.g., bDNA techniques, array capture techniques, sequencing techniques, etc. Further, the OLA product can be circularized to bring in contact sample and allele tags, forming a detectable junction.

### Detecting and Quantitating MIP Products by Next Generation Sequencing.

Next generation sequencing (NGS) can determine nucleic acid sequences, e.g., of probes or sample fragments in a massively parallel fashion. Such sequencing has the potential to determine the sample and allele combinations of thousands of MIPs in complex mixtures.

First generation nucleic acid sequencing techniques, such as Sanger based extension termination techniques, required cumbersome separate serial reactions and detections. The new NGS technologies employ various strategies that rely on combinations of template preparation, sequencing, imaging, and bioinformatic genome alignment methods. Exemplary NGS technologies include, e.g., sequencing by synthesis (Illumina, Inc., San Diego, California), single-molecule real-time sequencing with zero-mode waveguides (Pacific Biosciences of California, Inc., Menlo Park, California), pyrosequencing, ion semiconductor sequencing (Thermo Fisher Scientific Corporation, Carlsbad, California), and sequencing by ligation (Thermo Fisher Scientific Corporation, Carlsbad, California). Typically for genomic sequencing by NGS technologies, full length nucleic acid (e.g., gDNA) is broken into "template" fragments. The templates are then commonly captured or immobilized at spatially separated detector positions allowing hundreds to billions of sequencing reactions to be performed simultaneously. In some cases, the templates must be amplified before sequencing to allow sufficient signal. Because MIP products are of a typical NGS template size, readily amplified, and subject to various NGS library preparation steps such as ready addition of any required sequencing adaptors, they are ideal input for NGS analyses.

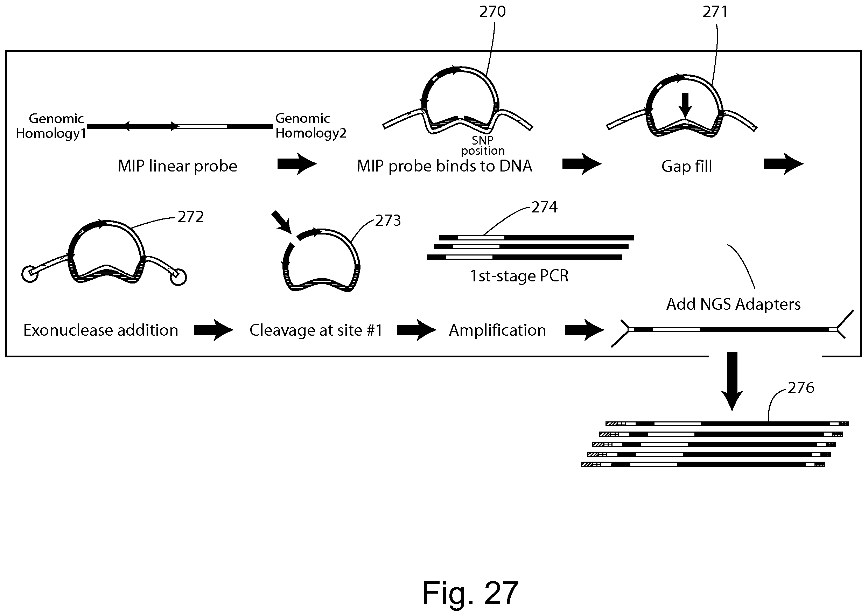

MIP reaction product (e.g., in the OncoScan^{®} FFPE Assay, Affymetrix, Inc., Santa Clara, California) can be subjected to a first stage PCR amplification before transfer to a physical capture array for detection. As shown at Figure 27, the MIP reaction products can receive any necessary NGS adaptors, e.g., during or after PCR amplification, and then transferred to the NGS process for detection. For example, necessary adaptors for any capture or priming required in the NGS system of choice can be applied to the MIP products by selection of PCR primers that also introduce a required NGS sequence, linker group, or reporter group. Alternately, any required adapter group can be applied to the MIP product by, e.g., hybridization, crosslinking, or enzymatic ligation.

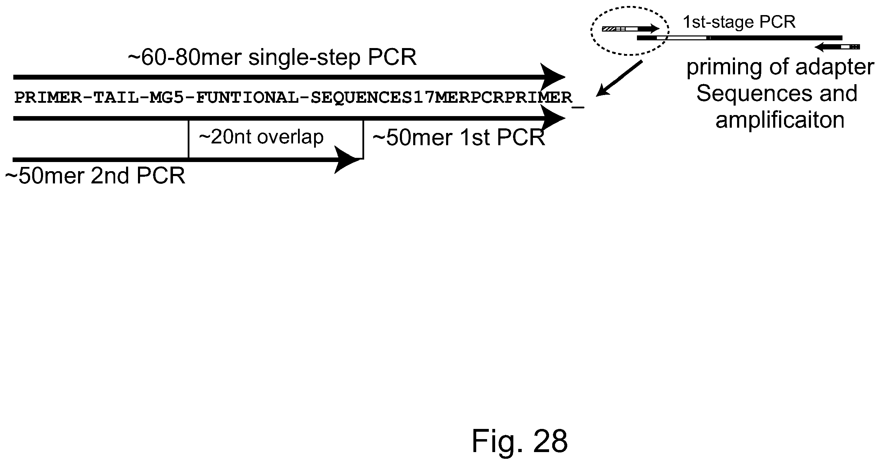

Figure 28 shows ways PCR primers can be employed to add required adaptors to MIP product ends during PCR amplification. Instead of, e.g., typical 17-mer forward and reverse PCR primers, the PCR primer can include various accessory sequences, e.g., to facilitate a second PCR amplification and/or to incorporate a NGS-functional sequence onto one or both ends of the MIP product. The PCR/NGS primer can have a standard PCR primer sequence, followed by an NGS adaptor sequence. Ultimately, the PCR amplicons will include the NGS adaptor and the MIP product is now a NGS template ready for sequencing.

In many cases the NGS adaptors function to allow capture of template to a sequencing location. For example, in the Illumina solid phase clonal bridge amplification approach, the solid support is closely populated with certain forward and reverse universal primers. An appropriate NGS adaptor for this system can be provided in the PCR amplification step wherein the primers can include sequences complementary to the capture/amplification primers on the solid support. In fact, the Illumina process includes an amplification system in which templates bridge between forward and reverse primers on the solid support until an amplified "cluster" of captured amplicons is formed. Therefore, it is possible to adapt MIPs to the Illumina system by simply requiring that the "PCR primer binding sites", e.g., of Figure 22A actually be complements of the Illumina solid support universal capture probes.

Several other systems capture by hybridization to a nucleic acid capture probe. Therefore, as with the Illumina system, appropriate NGS adaptors can be simply incorporated by, e.g., selection of a PCR primer probe for MIP amplification, wherein the primer comprises a complement of the capture probe.

Certain other systems do not directly capture the template. However, adaption would be essentially as described above. For example, the ion semiconductor sequencing of Thermo Fisher Scientific and single-molecule real-time sequencing with zero-mode waveguides of Pacific Biosciences systems do not capture the template directly on a solid support. However, the templates are indirectly captured, e.g., by a DNA polymerase or on a bead after clonal emulsion PCR, which itself is covalently bound to a substrate. Adaption for these systems requires only that the template be made an effective substrate for the polymerase, e.g., by provision of a polymerase primer.

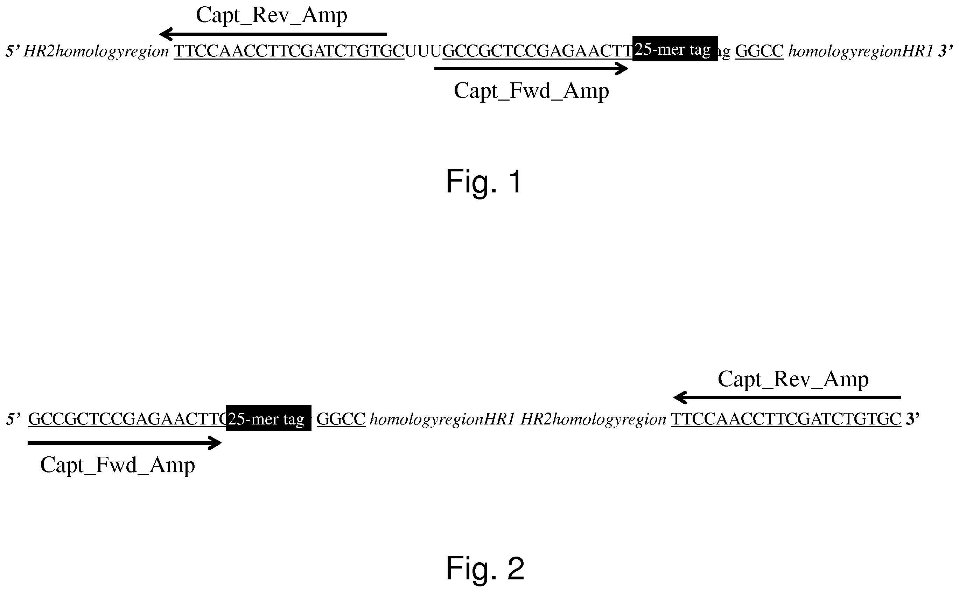

Because NGS detections can resolve a nearly unlimited number of tag identities based on the number of different tags that can be generated with, e.g., 20mers, 25mers, 30mers, etc., these techniques can evaluate massive numbers of MIP products with read outs of highly specific sample and allele determinations. For example, combined MIP to NGS assays could readily analyze hundreds of samples each for hundreds of alleles. A set of one hundred or more MIPs could be prepared with homology regions adapted (e.g., according to any of the schemes described above, and in Figures 1, 2, and 6 to 18, and 22) to specifically become circularized in the presence of each of the 100 or more alleles of interest. A first tag could be incorporated into each member of the set. The tag can have a unique combination of the four nucleotide bases that can be assigned to the first sample. Keep in mind that it only takes a 4-mer sequence to encode any of 256 unique ID tags. For additional samples, a separate different sample ID tag sequence can be incorporated into each member of the set of MIP allele probes. Each sample can be interrogated with a full set of the MIP probes, but the set for each set would have a different sample ID tag. After the MIP reaction is complete for each sample, the MIP products can be pooled and detected in one NGS sequencing run. As would be appreciated by one of skill in the art, by expansion of the number of bases in the tags at issue, many more alleles of interest can be simultaneously detected in this fashion to meet the desired level of allele/sample multiplexing (e.g., assays interrogating hundreds of thousands of alleles in a smaller number of samples, or a few thousand alleles in a larger number of samples).

The data readout from the NGS analysis would, e.g., consist of the sequence of every MIP product for every allele present in every sample. For 100 samples and 100 alleles, there would be 10,000 sequences present, at most. This is an easy task for modern NGS and computer systems to evaluate. For each sample (tag ID sequence) a list of present alleles (e.g., wild type and/or SNP) could be compiled and reported. This analysis can easily be accomplished on a small work space with MIP reactions taking place in a 96-well plate, 96-well PCR amplification if necessary, pooling of the MIP products, and NGS analysis of the pooled material.

MIP allele probe sets could be arranged to identify sets of alleles associated with health condition risk factors, oncogene combinations, drug efficacy correlates, personal phenotypes, and the like. 100 samples and 100 alleles is only an example. Using the MIP to NGS technology one can review a single sample for 100 or less, 1000, 10,000, 100,000, a million or more genotype sequences. Using the MIP to NGS technology one can review a single allele in 100 or less, 1000, 10,000, 100,000, a million or more samples. Clearly, it is envisioned that the MIP to NGS combination can effectively be used to screen any combination of samples and alleles in ranging among the above.

### Combinations of MIPs Improvements.

It is envisioned that the compositions and techniques disclosed herein can be used in combinations with surprisingly complementary and beneficial results. For example, the various tags, cleavage sites, gap-fill error avoidance schemes, multiplexing schemes, and NGS detection techniques can be used in fruitful combinations.

MIP probes described herein can include any of a variety of elements. For example, the MIP probes can include in order: 1) HR1,HR2; 2) HR1, cleavage site, HR2; 3) HR1, Tag, HR2; 4) HR1, PCR primer binding site, cleavage site, HR2; 5) HR1, PCR primer binding site, cleavage site, PCR primer binding site, HR2; 6) HR1, PCR primer binding site, cleavage site, PCR primer binding site, tag, HR2; 7) HR1, PCR primer binding site, cleavage site, PCR primer binding site, tag, cleavage site, HR2; 8) HR1, PCR primer binding site, cleavage site, PCR primer binding site, tag, cleavage site, HR2, affinity member; 9) HR1, PCR primer binding site, cleavage site, PCR primer binding site, tag, cleavage site, HR2, flap; 10) shorter HR1, PCR primer binding site, cleavage site, PCR primer binding site, tag, cleavage site, longer HR2; 11) shorter HR1, PCR primer binding site, cleavage site, PCR primer binding site, longer HR2; 12) shorter HR1, PCR primer binding site, cleavage site, PCR primer binding site, longer HR2, affinity member; 13) HR1, PCR primer binding site, cleavage site, HR2, affinity member; and/or 14) HR1, PCR primer binding site, cleavage site, PCR primer binding site, HR2, affinity member. In addition, in any of the above, a tag sequence can be incorporated within one or more of the HR sequences. In any of the above, the HR1 can include any of a ribonucleotide, a mismatch base, or a chemically sensitive base, e.g., at a position interrogating a target base of interest. In any of the above, the HR2 can include any of a ribonucleotide, a mismatch base, or a chemically sensitive base, e.g., at a position interrogating a target base of interest. In any of the above, the MIP can be adapted to leave a gap of at least 5 bases when hybridized to target, and the composition including an oligomer insert at least 80% complementary to the gap. In any of the above, the MIP can be configured to provide an elongated gap fill reaction (more than 1 nucleotide gap) with hybridization and polymerization in the specific target nucleic acid. In any of the above, the cleavage site can be a UNG substrate, a glycosylase substrate, a restriction enzyme substrate, or an endonuclease substrate. Any of the above, MIPs can also include an NGS adaptor allowing sequencing by an NGS method and platform, e.g., such as the MiSeq, MiSeqDx, NextSeq, and HiSeq platforms by Illumina, Ion PGM and Ion Proton platforms by Thermo Fisher Scientific, or the PacBio RS II platform by Pacific Biosciences. In any of the above, the tag can be a sample ID tag, allele ID tag, animal car code, and/or SNP bar code.

The MIP methods disclosed herein can be beneficially be used in various combinations that do not conflict. For example, the methods can include providing any of the MIP probes described herein, and in the paragraph immediately above. The methods can include: 1) contacting and hybridizing a target nucleic acid with a MIP having HR1 and HR2 separated by a gap of 1 to 3 bases on the target; 2) contacting and hybridizing a target nucleic acid with a MIP having HR1 and HR2 not separated by a gap on the target; 3) contacting and hybridizing a target nucleic acid with a MIP having HR1 and HR2, wherein HR2 is at least 10% longer than HR1; 4) any of 1 to 3 above, and further hybridizing the MIP to target and enzymatically removing any bases beyond those required to fill the gap; 5) any of the 1 to 4 above, and further providing a modified NTP (e.g., rNTP, dNTP, or analog) that bears a polymerase inhibiting function; 6) any of 1 to 5 above, further providing an affinity pair member to the MIP and capturing the MIP if it has not been circularized in the MIP reaction; 7) any of 1 to 6 above, further wherein the MIP has a HR flap and no gap when hybridized to target; 8) any of 1 to 7 above, further contacting the MIP with a cleavase; 9) any of 1 to 8 above, further cleaving the MIP with an enzyme that is not UNG; 10) any of 1 to 9 above, further adapting the MIP with a tag and capturing the MIP by the Tag with a capture probe on an array; 11) any of 1 to 10 above, further detecting the MIP with a series of junction set probes; 12) any of 1 to 11 above, further creating a junction between a sample ID tag and allele ID tag by circularization of the probe; 13) any of 1 to 12 above, further incorporating or generating NGS adaptors on ends of an MIP reaction product; and, 14) any of 1 to 13 above, further comprising sequencing two or more MIP reaction products by NGS techniques. The methods include any of the above wherein HR1 and/orHR2 has a tag sequence inserted within the HR sequence. The methods include any of the above, wherein the MIP is configured to provide an elongated gap fill reaction (more than 1 nucleotide gap) with hybridization and polymerization on the specific target nucleic acid.

### EXAMPLES

The following examples are offered to illustrate, but not to limit the claimed invention. The examples identify various complementary technical features that can be employed alone or in combination with other features described herein.

### EXAMPLE 1 - IMPROVING MIP AMPLICON PRODUCTION

Methods are described for increasing MIP technology allelic specificity and somatic mutation detection sensitivity. The techniques are well tailored to detect SNPs and somatic mutations. The allelic-specific probes described herein can also provide methods for addressing samples with multiple SNPs and/or somatic mutations.

The basic prior MIP platform uses DNA polymerase specificity to distinguish between two alleles during the gap fill reaction. However, a problem can exist when polymerase overruns the gap, resulting in an undesired extension (flap) that can prevent circularization of the probe at the subsequent ligation step. Described herein are approaches for gaining better discrimination between single base changes, while reducing the presence of flaps on MIP amplicons.

To allow ready removal of undesired overrun flaps (see, e.g., Figure 13), effective enzyme target substrates can be incorporated into a polymerase reaction for cleavage to eliminate any flaps that may be formed. For example, probes can be designed to contain a single ribonucleotide at a position of interest (base of a flap) in a targeted allele. The position can be specifically and effectively cleaved by ribonuclease H, e.g., to remove any overextended flaps, rendering the amplicon a proper substrate for ligase circularization. Probes and methods can be configured to optionally employ cleavases or flap endonucleases to remove flaps. Finally, enzymes can be employed in certain contexts wherein DNA duplexes containing single-base mismatches are the substrate for a flap removal enzyme.

The approaches above maintain key advantages of the MIP technology: 1) the cooperative hybridization between the genomic homology region 1 (HR1) and genomic homology region 2 (HR2), resulting in both increased relative Tms and more rapid hybridization kinetics since the two homology regions reside on a single probe; 2) ability to perform highly multiplex amplification, such as PCR with universal primers or rolling circle amplification; 3) inclusion of one or more tag sequences in the probe, enabling subsequent spatial separation of each target signal; 4) ability to use relatively short sequences within the target template; and, 5) amplification of clean synthetic polynucleotides rather than the initial, potentially highly modified (e.g., degraded, methylated), genomic target.

The MIP technology provides key advantages over alternative platforms. The cooperative hybridization between the HR1 and HR2 results in both increased relative melting temperatures (Tms) and more rapid hybridization kinetics since the two homology regions reside on a single probe. The use of universal primers can allow a highly multiplexed polymerase chain reaction. The presence of unique tags in the backbone for each target, can allow subsequent spatial separation of each resultant target signal. See an exemplary MIP probe structure at Figure 1. Unlike PCR, the MIP technology only requires short genomic template sequences, e.g., in the order of 15 to 30 total bases, sufficient for hybridizing to HR1 and HR2.

The current OncoScan^{®} MIP technology (Affymetrix, Inc., Santa Clara, California) can be divided into several function steps: 1) hybridization of the MIP probe to the target; 2) the gap-fill and ligation reaction circularizing the molecular inversion probe in the presence of the targeted genomic sequence; 3) digestion of uncircularized MIPs and target nucleic acids; 4) linearization of the probe to generate the PCR template; and 5) multiplex amplification by PCR.

The gap-fill reaction is used to provide specificity in identification of alleles or somatic mutations of interest. In previous work, at least two alternate methods were used to achieve allele discrimination during the gap-fill reaction.

In the most commonly used method, a single base gap is inserted between the HR1 and HR2 probe sequences at the position of interest in the target nucleotide. See Figures 1 and 2. The sample is split and placed into two separate gap-fill reactions. The gap-fill reactions each contain a different dNTP bases, e.g., complementary to one of two allele sequences of interest, such as one gap-fill reaction being provided with A and T and the other gap-fill reaction provided with G and C. The allele can be identified according to which reaction NTP is able to fill the gap. The concept also applies to embodiments wherein the polymerase substrate is an alternate NTP, such as, e.g., an rNTP or analog of a natural NTP.

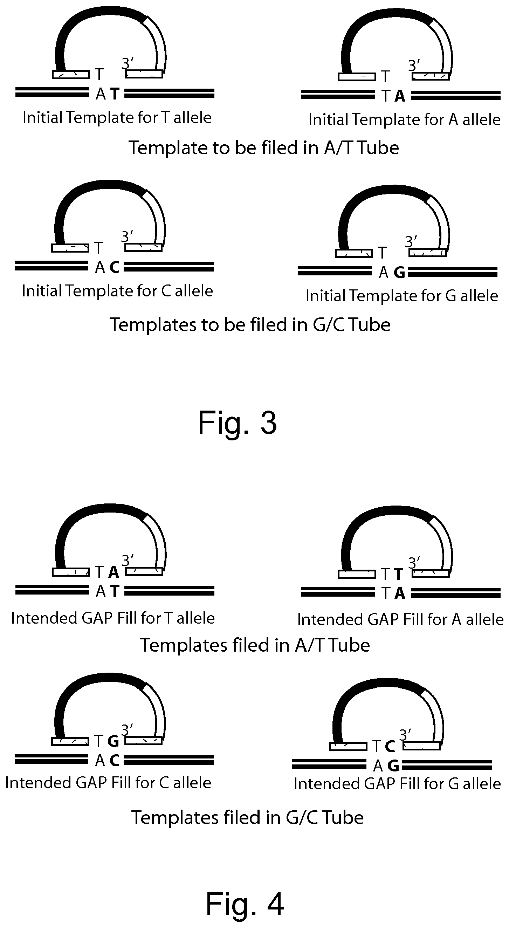

The second approach we have used for allele discrimination during the gap-fill reaction employs allele-specific probes in which the mismatch is positioned under the 3' end of the probe (at the 3' end of the HR1 region). See Figure 3. If the 3' base is complementary to the target, the polymerase is able to fill in the gap, permitting subsequent ligation and formation of the circularized MIP probe. If the 3' base is not complementary to the target, the polymerase does not efficiently fill in the gap, and, hence, formation of the circularized MIP probe does not occur efficiently.

However, the above reactions are not perfect and problems can exist wherein the gap-fill reaction does not provide adequate efficiency, diversity, sensitivity and/or specificity needed for measuring rare alleles (e.g. the use of allele-specific copy number analysis of tumors (ASCAT) to analyze samples containing mixtures of tumor and normal samples; or with the presence of a small fraction of cells containing somatic mutations within a larger population of cells that do not contain those somatic mutations).

### EXAMPLE 2 - IMPROVED ALLELIC MIP TECHNOLOGY DISCRIMINATION

As discussed below, the gap-fill reaction may not provide the efficiency, diversity, sensitivity and/or specificity needed for measuring rare alleles (e.g. the use of ASCAT to analyze samples containing mixtures of tumor and normal samples; or the presence of a small fraction of cells containing somatic mutations).

Again with regard to the MIP probe shown in Figure 1, the homologous regions, HR1 and HR2, hybridize to a target template surrounding the SNP or somatic mutation to be interrogated. Because of "cooperative hybridization", the Tm for each of the two genomic homology regions are effectively higher than if the two sequences were on separate oligonucleotides.

Figure 2 shows the structure of the MIP probe after annealing, gap-fill, and cleavage (linearization of circular probe) by UNG (cleavage of the UUU sequence).

The gap-fill reaction can be implemented, e.g., in two tubes: one containing dATP and dTTP; the other contains dCTP and dGTP. During the gap-fill reaction, the gaps containing an A or T in the template strand are filled in and ligated in the A/T tube while the gaps containing a G or C in the template strand are filled in and ligated in the G/C tube (Figure 3). Allele discrimination occurs during the gap-fill reaction. Efficient detection and allele discrimination requires: 1) proper incorporation of the correct base complementary to the template in the gap; 2) very low incorporation of a base when the template does not encode for the complement of one of the two NTPs in the gap-fill reaction; 3) very low incorporation of more than one base (when the gap is one base), which would result in stand displacement and formation of a flap that cannot be subsequently ligated; and 4) efficient ligation of the gap-filled template. Proper incorporation of the correct base with very low incorporation of the wrong base can be important to optimum allelic discrimination.

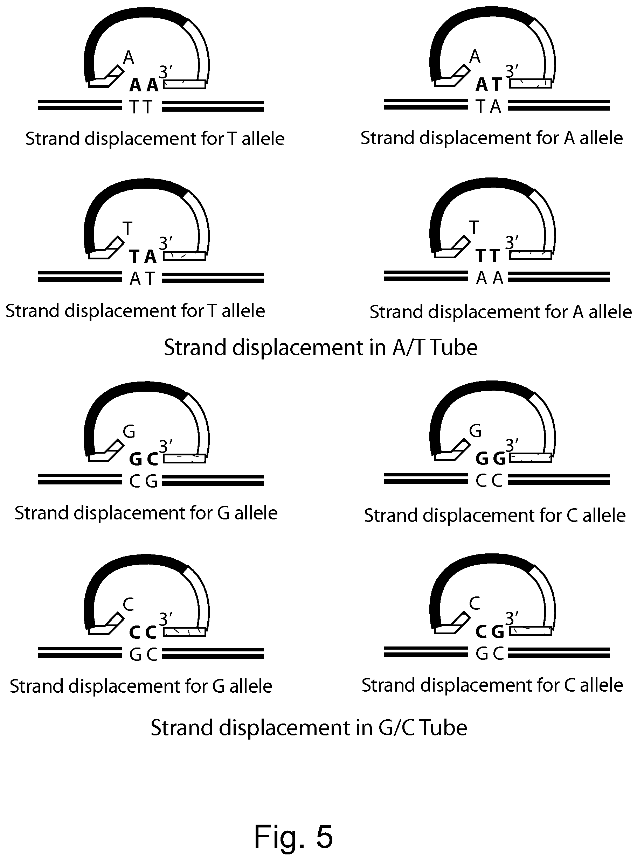

Figure 4 presents the "productive" results of the gap-fill reaction, leading to the production of a detectable circularized MIP probe. Incorporation of more than one base can result in a flap which cannot be ligated (see, Figure 5). As a result, the amount of signal generated for the proper allele may be reduced. In a four tube gap-fill reaction, displacement reactions can only occur if the next base is the same as that present in the gap. However, in a two tube gap-fill reaction, strand displacement can occur if the next base is an A or T, for the A/T gap-fill reaction, or a G or C, for the G/C gap-fill reaction. One way in which current MIP assays limit the amount of stand displacement is by keeping the dNTP concentration low. Again, as with other gap-fill methods, the polymerase NTP substrate can alternately be a dNTP, rNTP, or analog, e.g., depending on the specificity of the polymerase.

The Kₘ^{app} of Taq DNA polymerase for dNTPs has been reported to range between 0.16 µM and 16 µM . A typical PCR provider recommends dNTP concentrations between 40 µM and 200 µM when using Stoffel fragment for PCR. Use of dNTPs at concentrations significantly lower than the Kₘ will result in a lower DNA polymerase activity. Increased base misincorporation has been reported for Taq polymerase when one or more of the dNTPs are well below the Kₘ or when the concentration of one dNTP is very low relative to the other dNTPs. Base misincorporation was not observed when the concentration of all four dNTPs is similar or when all four dNTPs are present at > 10 µM each.

Several studies have shown that the Stoffel fragment of *Thermus aquaticus* DNA polymerase has significant strand displacement activity. Increasing the dNTP concentration will typically increase the strand displacement activity, resulting in the generation of a flap during the gap-fill reaction that cannot be subsequently ligated.

Generation of flaps during gap-fill in MIP assays causes a loss of signal because the probe fails to circularize, which then commonly leads to those MIPs not being detected (e.g., after an exonuclease digestion of the non-circularized single stranded nucleic acids). One way to mitigate the loss is to digest away any flaps formed. In this example, conditions are established to include ribonucleic acids in any flaps formed, so they can be removed by an RNase, without damage to the DNA probe. The concept is generalizable to the use of other specific cleavage schemes at probe interrogation positions, e.g., as discussed exhaustively herein.

The class of Ribonuclease H enzymes vary greatly with respect to their substrate specificities. An RNase of special utility here is *Pyrococcus abyssi* RNase HII, which has specific endonuclease activity against RNA-DNA/DNA duplex substrates. The enzyme cleaves one nucleotide upstream from an RNA-DNA junction. The RNA must be completely hybridized to the DNA template. Substrates containing a 5' RNA flap or containing mismatches near the RNA-DNA junction are poor substrates for the enzyme. Ribonuclease HII from *Bacillus subtilis* and *Thermococcus kodakaraensis* cleave DNA-RNA-DNA/DNA substrates containing a single ribonucleotide. In contrast, *Eschericia coli* RNase HI and *B. subtilis* RNase HII do not cleave substrates containing single ribonucleotides. A number of ribonucleases can cleave single-base mismatches in RNA/DNA or RNA/RNA targets but will not cleave perfectly matched RNA/DNA and RNA/RNA targets. Other enzymes cleave both, but show a significantly reduced activities when the ribonucleotide is positioned at a mismatch.

When an RNase selected for use cleaves on the 5' side of ribonucleotides, MIP probes can be configured to allow HR1 to hybridize only where a probe ribonucleotide complements the corresponding deoxyribonucleotide on the target fragment. For example, as shown in Figure 6, a ribonuclease H enzyme is selected which cleaves on the 5' side of a ribonucleotide only when the probe ribonucleotide complements the target base. The probe would be designed with the ribonucleotide in the HR1 region. Alternately, as shown in Figure 7, probes can be designed for enzymes that cleave on the 3' side of the ribonucleotide, and the probe would be designed with the ribonucleotide residing in the HR2 region. The respective MIP probe homology regions would contain sufficient deoxyribonucleotide or ribonucleotide bases needed for efficient cleavage by the ribonuclease H enzyme. The ribonucleotide base used to distinguish the single-base match or mismatch would reside adjacent to, or within, the deoxyribonucleotide portion of the probe homology region. The homology region containing the ribonucleotide is constructed to be longer, thus having a higher melting temperature than the shorter homology region on the other side of the probe. As a result, the initial hybridization of the MIP probe is carried out at an elevated temperature to ensure that the longer homology region hybridizes to the target, initially excluding the shorter homology region. Following the initial hybridization, the temperature is lowered and the RNaseH digestion is carried out. As a result of cooperative hybridization, the homology regions rapidly hybridize to the target. Ligation can be carried out either simultaneously with, or subsequent to, the RNase H digestion, resulting in generation of the circularized MIP probe.

The MIP can also include one or more identifying tag sequences associated with the target/sample, a UNG or other cleavage site, and universal primers enabling a high degree of multiplexing.

Figure 6 illustrates an example of embodiments using a form of the MIP probe cooperating with a ribonuclease enzyme that cleave on the 5' side of ribonucleotides. There are two allele-specific forms of the MIP probe. Each MIP probe contains a ribonucleotide at the position to be identified, one with a U (shown at 6A) and one with an A (shown at 6B). MIP probes hybridize to the target **60.** As a result of the higher melting temperature, the extended HR2 region **61** preferentially hybridizes to the target, preventing hybridization of the shorter HR1 region **62.** If the probe contains the ribonucleotide complementary at the position of the SNP (as in Figure 6A) **63,** the ribonuclease cleaves the probe, generating a free 3' end **64** (see Figure 6C). The short fragment beyond the excised ribonucleotide of the HR2 deoxyribonucleotide leaves the target due to its low melting temperature. With the inherent cooperative binding, the previously free HR1 region **62** on the MIP probe rapidly hybridizes to the template (as shown in Figure 6D) and is subsequently ligated (as shown in Figure 6E) to the HR1 region, generating the circularized MIP probe. Higher specificity of the overall reaction can be introduced by performing the initial incubation at elevated temperatures appropriate for the hybridization temperatures of the extended HR1 sequences. The temperature is then reduced during the more rapid ribonuclease H and ligation steps.

Figure 7 illustrates an arrangement wherein the elected ribonuclease cleaves on the 3' side of ribonucleotides. To determine between two different bases at the position of interest, two allele-specific forms of the MIP probe are provided in different reactions. Each MIP probe contains a different ribonucleotide at the position to be identified ( see 7A and 7B). The MIP probes **70** are hybridized to the target **71.** As a result of the higher melting temperature, the extended HR1 region **72** preferentially hybridizes to the target, preventing hybridization of the shorter HR2 region **73.** If the probe contains the ribonucleotide complementary at the position of the SNP **74** (Figure 7A), the ribonuclease cleaves the probe, generating a free 5' end (Figure 7C). The short fragment of the deoxyribonucleotide leaves the target due to its low melting temperature. As a result of cooperative binding, the HR2 region **73** on the MIP probe rapidly hybridizes to the template (as shown in Figure 7D) and is subsequently ligated (as shown in Figure 7E) to the HR1 region **72,** generating the circularized MIP probe. Higher specificity of the overall reaction can be introduced by performing the initial incubation at elevated temperatures appropriate for the hybridization temperatures of the longer HR1 sequences. The temperature can then be reduced during the more rapid ribonuclease and ligation steps.

An example of a series of MIPs designed to distinguish a set of BRAF mutation are indicated in Figure 8. The bold bases in the BRAF sequences represent the mutated (e.g., SNP) bases. The dark bold bases in the probe sequences represent the position of the ribonucleotide analogue.

Similar probe constructs are designed to functionally cooperate with enzymes directed to mismatches within duplex DNA. For example, the MIP probe can have a structure similar to that described for RNase H-based probes above with the exception that the allele-specific base would consist of deoxyribonucleotides. An enzyme useful with such constructs can be endonculease V, which has been reported to nick DNA at the second phosphodiester bond 3' to a mismatched base. The *Thermotoga maritime* Endonuclease V has been used in combination with a high-fidelity DNA ligase to develop a method for mutation scanning. Combinations of enzymes can be used as well. For example, the glycosylase TDG removes mismatched thymidines and the glycosylase MutY removes mismatched adenines. In the case of the TDG and MutY glycosylases, a second enzyme, such as an AP endonuclease/lyase, which cleaves abasic sugar substrates completes the strand cleavage. Combining these enzymes with subsequent PCR amplification, mutation frequencies as low as 1% have been detected, using as low as 5 ng of genomic DNA. Other enzymes reported to cleave at mismatches within DNA include: T4 endonuclease VII, T7 endonuclease I, deoxyinosine 3'-endonuclease from Escherichia coli, and modified to endonuclease V enzymes (see Gao, NA Res 35(1): e2). With some of the enzymes, the mismatch is designed at the penultimate base, depending on the mechanism of the enzymatic cleavage and the required substrate for the particular enzyme.

MIP probes based on the alternative enzymes listed above would function similar to that of the RNase H probes, as shown in Figure 9. The targeted base would reside either within the HR1 region or the HR2 region of the MIP probe, depending on which side of the mismatched base the enzyme cleaves. As with the RNase H-based probes, initial hybridization of the probe to the target is typically carried out at elevated temperatures. The temperature can subsequently be lowered and cleavage of the mismatched bases carried out followed by hybridization of the second HR region and ligation.

In Figure 9, MIP probe **90** is designed to be a double-stranded mismatch nuclease target form for nucleases cleave on the 5' side of mismatches in double-stranded DNA. There are two allele-specific forms of the MIP probe to detect normal and expected mutant forms of the target. Third or fourth forms can be provided in separate reactions, e.g., to detect alternate SNPs at the same location. Each MIP probe contains a deoxyribonucleotide at the position to be identified (see Figures 9A and 9B). The MIP probes are hybridized to the target **91.** As a result of the higher melting temperature, the longer extended HR2 region **92** preferentially hybridizes to the target, preventing hybridization of the shorter HR1 region **93** of the probe. If the probe contains a deoxyribonucleotide **94** not complementary at the position of the SNP (see Figure 9B), the nuclease specific for mismatches in double-stranded DNA cleaves the probe, generating a free 3' end (see Figure 9C). The short fragment of the deoxyribonucleotide cut from HR2 leaves the target due to its low melting temperature. Due to the kinetic advantage of cooperative binding, the HR1 region **93** on the MIP probe rapidly hybridizes to the template (see Figure 9D) and is subsequently ligated (see Figure 9E) to the HR2 region, generating the circularized MIP probe. Again, higher specificity of the overall reaction can be introduced by performing the initial incubation at elevated temperatures appropriate for the hybridization temperatures of the extended HR2 sequences. The temperature can then be reduced during the more rapid nuclease digestion and ligation steps.

Optionally, as shown in Figure 10, MIP probe **100** is designed to be a double-stranded mismatch nuclease target form for nucleases cleave on the 3' side of mismatches in double-stranded DNA. Typically, there are at least two allele-specific forms of the MIP probe (e.g., normal consensus and mutated). Each MIP probe contains a deoxyribonucleotide base at the position to be identified (see Figures 10A and 10B). The MIP probes hybridize to the target **101.** As a result of the higher melting temperature, the extended H1 region **102** preferentially hybridizes to the target, preventing hybridization of the shorter HR2 region **103.** If the probe contains the deoxyribonucleotide not complementary at the position of the SNP (see Figure 10B), the nuclease specific for mismatches in double-stranded DNA cleaves the probe, generating a free 3' end (see Figure 10C). The short fragment of the deoxyribonucleotide cut from the HR1 region leaves the target due to its relatively low melting temperature. As a result of cooperative binding, the previously free HR2 region on the MIP probe rapidly hybridizes to the template (see Figure 10D) and is subsequently ligated (see Figure 10E) to the HR1 region, generating the circularized MIP probe **104.** Higher specificity of the overall reaction can be introduced by performing the initial incubation at elevated temperatures appropriate for the hybridization temperatures of the extended HR1 sequences. The temperature can then be reduced during the more rapid nuclease digestion and ligation steps.

In another optional configuration, the MIP probe can be designed with a preexisting flap sequence providing a substrate for a flap endonuclease enzyme. A variety of flap endonucleases or cleavases can also be used to generate circularized MIPs after cleavage of a flap and ligation of the MIP's 5' and 3' ends (see Figure 11C). In many instances, the circularized MIP probes described herein can be specifically cleaved to linearize the MIP before further amplification, e.g., with PCR.

Flap endonucleases, cleavase I, and cleavase VIII have been used for allele-specific detection. The enzyme cleaves unpaired regions of DNA which form when the 5' end of a DNA sequence overlaps the hybridization site of the 3' end of an upstream oligonucleotide by at least one base pair. For example the Invader Assay (Hologic, Inc., Bedford, Massachusetts) is based on the ability of these structural-dependent nucleases to discriminate between single-base differences. The Invader assay (see, e.g., Olivier, Mutation Res. 573: 103), by itself has limited signal generation. One approach to increase sensitivity has been to initially amplify the target by PCR followed by allele identification using the Invader assay. The Invader approach has also been demonstrated with RNA as a template (see, e.g., Olson, Met. in Mol. Bio. 258: 53).

An example of MIP probes using the flap endonucleases or cleavase enzymes are shown in Figure 11. The MIP probes are hybridized to the target in a fashion as described above. Following addition of the enzyme, the flap corresponding to the targeted base is cleaved. Following ligation, the circularized MIP probe is linearized and amplified.

As shown in Figure 11, a cleavase can be used to generate, with allele specificity, MIPs ready for circularization, e.g., in the presence of a complementary SNP. Two (or 3 or 4) allele-specific forms of the MIP probe are included within the assay. Each MIP probe **110** contains the allele-specific base followed by a series of nucleotides (NNN in the example Figure 11) which do not hybridize to the target template **111.** The proper substrate for the cleavase enzymes contain an upstream sequence hybridized directly adjacent to a flap (as in Figure 11A). Substrates containing a gap between the 3' end of an upstream sequence (Figure 11B) and the flap are poor substrates for cleavases. Cleavase removes the Figure 11A substrate flap, generating a product (Figure 11 C) which can then be ligated, generating a product that can be converted into a circularized MIP probe **112.**

In further options to enhance the efficiency of successful MIP probe conversion, MIPs with unequal blocking homology regions can be cut after initial hybridization to a target in a fashion similar to those described above, except, wherein the cleavage is by non-enzymatic chemical means. A chemical cleavage configuration can be designed into the MIP by including a base, or base pairing, sensitive to chemical attack. For example, non-enzymatic methods can also be used to cleave duplex DNA containing mismatches. In an embodiment, the DNA intercalator [Rh(bpy)₂(chrysi)]³⁺ specifically binds in destabilized regions near DNA base mismatches. Upon photoactivation, the complex cleaves the DNA backbone. Specific DNA cleavage has been observed at over 80% of mismatch sites in all the possible single base pair sequences. The process for using chemical cleavage would be similar to that described under above for constructs consisting of mismatches within duplex DNA, except the cleavage is by chemical (e.g., non-enzymatic) and/or photolytic means.

### EXAMPLE 3 - ALTERNATIVE MIP ARCHITECTURE

Typical MIP assays utilize a gap-fill and ligation step. Presented here is an alternative architecture of the MIP structure to facilitate mutation interrogation in the context of complex mutations such as those seen in BRAF, and can simplify the protocol by avoiding enzymatic extension (e.g., gap-fill) and/or cleavage steps to leave a single enzymatic step of ligation to form the circularized MIP. The goal, as would apply to other MIP structures, would include genotyping and would utilize many of the current procedures used in MIP assays such as the DMET^{™} Plus Solution for drug metabolism enzymes and drug transporters variation analysis (Affymetrix, Inc., Santa Clara, California) and the OncoScan^{®} FFPE Assay for solid tumor copy number analysis (Affymetrix, Inc., Santa Clara, California). Thus, these alternative MIP architectures can be used for, e.g., genotyping and determining copy number alterations in a given DNA sample.

As shown in Figure 12, the method is based on detection of whether or not an oligonucleotide hybridizes to the regions 3' and 5' of a SNP or other polymorphism position under stringent conditions. The presence of the hybridized interrogating oligonucleotide is detected by bracketing it with a MIP probe and subsequent ligation, without the necessity of a gap-fill step. The presence of circularized probe would indicate the oligonucleotide specifically bound to the sequence with a particular SNP.

Results are determined very similar to other oligonucleotide hybridization assays, by measuring, e.g., fluorescent signal, from reactions with fully complementary oligomer and single mismatch oligomer. In many cases, hybridization differences between the match and mismatch probes is enhanced when the interrogation base or bases are at or near the center of the oligonucleotide probe. A statistically distinguishing difference will be present between intensity of fluorescent signals, even where some level of hybridization is present in the mismatch reaction.

For example, a SNP detection method is practiced by designing oligomer probes different only at a position **120** to be interrogated and establishing hybridization stringency conditions that provide the greatest difference in hybridization of matched **121** and mismatched **122** oligomer probes. In separate reactions, oligomer probes are hybridized with the target nucleic acid **123** of interest. Perfectly complementary oligomer probes will bind well to the target, and probes with a mismatch will bind substantially less well, if at all depending on the design of the probe sequence and the hybridization conditions employed. A MIP probe **124** in the reaction is configured to hybridize well to adjacent regions of the target nucleic acid bracketing the oligo probe complement sequence. If the oligomer probe is hybridized to the target, it will abut the MIP probe HR1 region at one end and the HR2 region at the other end. Thus, a substrate is presented for a ligase to join the oligo probe to the MIP probe in a circularized product. The MIP probe, circularized without a gap-fill step, can then be detected by any number of techniques discussed herein.

Subsequent processing can be almost identical to current OncoScan^{®} FFPE Assay protocols. In many contexts, particularly OncoScan, it is an advantage to have two separate tag sequences on MIP probes interrogating a particular position of a polymorph, one for each of the 2 channels (e.g., for the case of two different MIPs being used to interrogate a biallelic SNP).

### EXAMPLE 4 - A MORE EFFICIENT METHOD FOR CARRYING OUT MOLECULAR INVERSION PROBE ASSAYS

The efficiency and specificity of the MIP technology can be improved by increasing the concentration of the intended NTP for gap-fill, while providing other NTPs in removable form. The approach increases the efficiency of generating the ligated, circularized MIP probe by increasing the concentration of the deoxyribonucleotide triphosphates used in the gap-fill reaction while reducing the formation of flaps, which will prevent the subsequent ligation needed to form the circularized molecular inversion probe product. The approach maintains the MIP advantage of the cooperative hybridization between the HR1 and HR2 regions, resulting in both increased relative Tₘ s and more rapid hybridization kinetics since the two homology regions reside on a single probe.

The efficiency of the gap fill reaction can be increased by increasing the NTP concentration. However, increasing the NTP concentration would result in increased strand displacement, resulting in a flap that cannot be ligated. One approach to preventing the formation of a gap that cannot be ligated is to add bases which can subsequently be removed prior to ligation. DNA-dependent polymerases have been described that can incorporate ribonucleotides in addition to deoxyribonucleotides (see, e.g., Patel, J. of Bio. Chem. 275(51): 40266).

The discussion below describes methods using a combination of ribonucleotide triphosphates and ribonuclease H. However, any combinations of modified nucleotide triphosphate analogues that can serve as a substrate for DNA polymerase and that can be subsequently removed enzymatically or chemically can also be used.

In general, as in the typical MIP assay, the MIP probe contains the same structures as outlined in Figure 1. The anneal reaction is carried out in the same manner, but the gap-fill reaction is carried out in the presence of one deoxynucleotide (dNTP) of interest and three ribonucleotides (rNTPs). The concentration of the dNTP and the rNTPs can be set closer the Km of the enzyme in order to increase efficiency. Following the gap-fill reaction, the added ribonucleotides are removed by addition of a ribonuclease, such as RNase H. Once the portion containing the ribonucleotides is removed, the other genomic homology region hybridizes. The product can then be ligated to form the molecular inversion probe.

The exemplary approach is as follows (Figure 13):
1. Hybridize a MIP probe to its target nucleic acid, as shown in Figure 13A, e.g., as is currently being done with the OncoScan FFPE Assay.
2. Carry out the gap-fill reaction with the desired dNTP (e.g. dTTP - shown in bold Figure 13B) in the presence of the three alternative rNTPs (e.g. rATP, rCTP and rGTP). The concentration of the four nucleotide triphosphates can then be adjusted to improve the incorporation of the desired dNTP. For example, the concentrations of both the deoxynucleotide and ribonucleotide triphosphates can be set at the relative Km's for the polymerase being used. As will be discussed below, strand displacement may not be an issue. Following insertion of the targeted deoxyribonucleotide (dT in the example), ribonucleotides can then be potentially, and likely will be, incorporated (GAGCC in the example) resulting in displacement of the HR1 region **130** of the MIP probe **131** (see Figure 13C). Note that a combination of dNTP and rNTP can also be included for the targeted base. If there are repeats of the targeted base (e.g. AAA), incorporation of both forms of the nucleotide triphosphates will result in a mixed population of the desired gap-filled product.
3. A ribonuclease, capable of cleaving ribonucleotide-containing hybrid, such as ribonuclease H, is added to remove any undesired follow on extensions that may have occurred. See Figure 13D.
4. The HR1 region then anneals (if it was displaced), followed by ligation to form a molecular inversion probe. See Figure 13E. Even if the polymerase displaces the HR1 portion of the probe, the MIP probe remains hybridized to the template through the HR2 **132** portion of the probe. Once the extended region is removed by RNase H, the HR1 region will rapidly hybridize to the genomic target as a result of cooperative hybridization.
5. The resultant product is then ligated to form a circularized molecular inversion probe.

The above steps can be combined in order to generate a more efficient, user-friendly process. That is, the steps do not have to be practiced in series, but may take place at the same time in the reaction mixture.

This disclosure describes the method using a combination of ribonucleotide triphosphates and ribonuclease H. However, any combinations of modified nucleotide triphosphate analogues that can serve as a substrate for DNA polymerase and can be subsequently removed enzymatically or chemically.

### EXAMPLE 5 - METHOD TO REMOVE NON-CIRCULARIZED MIP PROBES

This example describes an alternate efficient method for removing excess MIP probes that have not been circularized following the gap-fill reaction. The OncoScan platform typically utilizes a combination of three exonucleases (exonuclease I, exonuclease III, and exonuclease VII) to remove uncircularized MIP probes following the gap-fill reaction. This removal of uncircularized MIP probe is important to MIP assay performance. In addition to unreacted MIP probe, the gap-fill reaction can result in polymerization of MIP probes, especially at high MIP probe concentrations. During a subsequent PCR reaction, both unreacted and polymerized MIP probe can compete for primer hybridization, decreasing the efficiency of amplification of the true product. In addition, polymerized MIPs result in a template which can be amplified during the subsequent PCR reaction. Therefore, removal of any form of linear MIP probe is very important, where PCR is involved in the amplification step.

In typical MIP workflow, any form of linear MIP probe is removed following the gap-fill reaction by digestion with a combination of exonucleases. The exonuclease mixture contains exonuclease I, exonuclease III and exonuclease VII. Exonuclease I digests single-stranded DNA in a 3'-5' direction, requires a free 3'-hydroxyl terminus, but does not digest double-stranded DNA. Exonuclease III is a 3'-exonuclease which catalyzes the removal of mononucleotides from the 3'-OH end of double stranded DNA. It also dephosphorylates DNA strands which possess a 3'-phosphate group and has RNase H activity. Exonuclease VII digests DNA from free 3' or 5' ends. Exonuclease VII has been reported to have little activity on circularized DNA. However, during the initial development of padlock and MIP probes, it was suggested that the commercially available exonuclease VII enzyme contained contaminating endonucleases.

During the initial development of padlock and MIP probes, exonuclease VII was the only enzyme used to digest uncircularized probes. These studies suggested that some endonuclease activity was present in the exonuclease VII preparation and found that increasing the exonuclease VII concentration resulted in digestion of the circularized probe. The current nuclease cocktail presents problems in balancing the need to systematically remove linear nucleic acids and the danger of undesired side reactions or contaminant activity.

Here, we discuss how nonenzymatic methods can be used for efficient removal of linear MIP probe product without the risk of removal of the desired circularized MIP probe. The general concept is to replace enzymatic methods for removal of the linear form of MIP probe with a more consistent, controllable, and automatable approach.

MIP probes can be designed to contain biotin for removal of the probes from the reaction at the appropriate time. However, the biotin is bound to the uncircularized probes in a way such that they are removed during the circularization process. For example, the biotin can be incorporated into one end of the probe. Upon hybridization and circularization of the probe, the end containing the biotin is cleaved, generating the circular product which no longer contains biotin. Upon exposure of the reaction mixture to a surface containing streptavidin or avidin (e.g., streptavidin coated magnetic microparticles), all unreacted or polymerized species are efficiently removed.

The approach is easily controlled and does not suffer from the potential for contaminating endonucleases. In addition, the approach lends itself to automation by immobilizing streptavidin or biotin to magnetic particles, nonmagnetic particles, filters, columns or walls of a device. Although the biotin-streptavidin (or avidin) system is used in the examples listed below, any ligand-receptor combination could be used. Other representative examples include, but are not limited to: 1) digoxigenin/anti-digoxigenin; 2) fluorescein/anti-fluorescein; 3) estrogen/anti-estrogen; 4) hexa-his/nickel-NTA; and/or the like. Essentially any capture system can be used which is compatible with the incorporation of the ligand within the nucleic acid probe. Although the figures display the ligand at the end of the probe, the ligand could be place anywhere within the region of the probe which would be removed upon enzymatic or chemical cleavage. There are several approaches to achieving the approach.

RNase H Configuration. Ribonuclease H recognizes RNA-DNA hybrids. The class of ribonuclease H enzymes varies greatly with respect to their substrate specificities. *Pyrococcus abyssi* RNase HII is a specific endonuclease acting on RNA-DNA/DNA duplexes. The enzyme cleaves one nucleotide upstream from the RNA-DNA junction. The RNA must be completely hybridized to the cDNA template. Substrates containing a 5' RNA flap or containing mismatches near the RNA-DNA junction are poor substrates for the enzyme. Ribonuclease HII from *Bacillus subtilis* and *Thermococcus kodakaraensis* cleave DNA-RNA-DNA/DNA substrates containing a single ribonucleotide. In contrast, *Eschericia coli* RNase HI and *B. subtilis* RNase HII do not cleave substrates containing single ribonucleotides. A number of ribonucleases can cleave single-base mismatches in RNA/DNA or RNA/RNA targets but will not cleave perfectly matched RNA/DNA and RNA/RNA targets. See, e.g., Ichinose, Anal. Chem. 77:7047, and Myers, Science 230:1242. Other enzymes cleave both, but show a significantly reduced activities when the ribonucleotide is positioned at a mismatch.

According to the type of RNase H used, MIP probes can be configured to function in providing efficient gap-fill substrates and ligase substrates to detect, e.g., SNP variants. When using Ribonuclease H enzymes which cleave on the 5' side of the ribonucleotide, the ribonucleotide **140** would reside in the HR2 region **141** (Figure 14). For these constructs, the biotin **142** would be placed within the 3' H2 region that would be removed upon cleavage by ribonuclease H. When the enzyme cleaves on the 3' side of the ribonucleotide, the ribonucleotide base **150** would reside in the HR1 region **151** (Figure 15). The respective homology regions would contain sufficient deoxyribonucleotide or ribonucleotide bases needed for efficient cleavage by the ribonuclease H enzyme. The ribonucleotide base used to distinguish the single-base mismatch would reside adjacent to, or within, the deoxyribonucleotide portion of the homology region. The homology region containing the ribonucleotide is longer, and, hence, has a higher melting temperature. As a result, the initial hybridization of the MIP probe can be carried out at an elevated temperature to ensure that the longer homology region hybridizes to the target. Following the initial hybridization, the temperature can be lowered and the RNase H digestion can be carried out. As a result of cooperative hybridization, the shorter homology region will rapidly hybridize to the target. Ligation can be carried out either simultaneously with, or subsequent to, the RNase H digestion, resulting in generation of the circularized MIP probe. Following cleavage of the probe by ribonuclease H, the biotin would be removed. Any linear ligation of MIP probes would still contain an unreacted end, containing the biotin. Therefore, nonspecific linear ligation of the probe would continue to be removed upon contact with streptavidin.

The remainder of the MIP probe incorporates other components for the MIP assay in question, such as a tag sequence associated with the target, a UNG or other cleavage site as needed, and universal primers enabling a high degree of multiplexing (if PCR amplification is used).

Figure 14 shows a form of MIP probe **143** subject to for Ribonuclease H enzymes that cleave on the 5' side of ribonucleotides. The biotin **142** would be engineered on the 3' portion of the probe which would be removed upon cleavage by ribonuclease H. In the technique shown, there are two allele-specific forms of the MIP probe (additional reactions can be arranged for detection of alternate mutants, e.g., C or G SNPs). Each MIP probe contains a ribonucleotide at the position to be identified (see Figures 14A and 14B). MIP probes hybridize to the target. As a result of the higher melting temperature, the extended target Homology Region HR2 **141** preferentially hybridizes to the target, preventing hybridization of the shorter HR1 **144** region. If the probe contains the ribonucleotide complementary at the position of the SNP (Figure 14A), ribonuclease H cleaves the probe, generating a free 3' end (Figure 14C). The short fragment of the deoxyribonucleotide leaves the target due to its low melting temperature. As a result of cooperative binding, the HR12 region on the MIP probe rapidly hybridizes to the template (Figure 14D) and is subsequently ligated (Figure 14E) to the HR2 region, generating the circularized MIP probe **145.** Higher specificity of the overall reaction can be introduced by performing the initial incubation at elevated temperatures appropriate for the hybridization temperatures of the extended HR2 sequences. The temperature can then be reduced during the more rapid ribonuclease H and ligation steps.

Figure 15 shows a form of MIP probe **152** subject to for Ribonuclease H enzymes that cleave on the 3' side of ribonucleotides. The biotin **153** would be engineered on the 5' portion of the probe which would be removed upon cleavage by ribonuclease H. There are two allele-specific forms of the MIP probe. Each MIP probe contains a ribonucleotide at the position to be identified (Figures 15A and 15B). MIP probes hybridize to the target. As a result of the higher melting temperature, the extended HR1 region preferentially hybridizes to the target, preventing hybridization of the shorter HR2 region **154.** If the probe contains the ribonucleotide complementary at the position of the SNP (Figure 15A), ribonuclease H cleaves the probe, generating a free 5' end (Figure 15C). The short fragment of the deoxyribonucleotide leaves the target due to its low melting temperature. As a result of cooperative binding, the HR2 region on the MIP probe rapidly hybridizes to the template (Figure 15D) and is subsequently ligated (Figure 15E) to the HR1 region, generating the circularized MIP probe **155.** Higher specificity of the overall reaction can be introduced by performing the initial incubation at elevated temperatures appropriate for the hybridization temperatures of the extended HR1 sequences. The temperature can then be reduced during the more rapid ribonuclease H and ligation steps.

An example of a series of MIPs designed to distinguish a set of BRAF mutations are indicated in Figure 8. Shown are a series of MIPs targeted against a set of adjacent BRAF mutations. The dark bases in the BRAF sequences represent the mutated bases. The dark bold bases in the probe sequences represent the position of the ribonucleotide analogue.

DNA Mismatches Based Version. Similar constructs and techniques can be carried out in the context of mutation identification at locations of DNA duplex mismatches. Nucleases that digest mismatches within double stranded DNA can be used. The MIP probe would have a structure similar to that described for RNase H-based probes with the exception that the allele-specific bases would consist of deoxyribonucleotides.

Endonuclease V has been reported to nick DNA at the second phosphodiester bond 3' to a mismatched base. The *Thermotoga maritime* Endonuclease V has been used in combination with a high-fidelity DNA ligase to develop a method for mutation scanning (see, e.g., Gao, NA Res. 35(1): e2). A combination of enzymes can be used as well. For example, the glycosylase TDG removes mismatched thymidines and the glycosylase MutY removes mismatched adenines. In the case of the TDG and MutY glycosylases, a second enzyme, such as an AP endonuclease/lyase, which cleaves the abasic sugar, is required to generate strand cleavage. Combining these enzymes with subsequent PCR amplification, mutation frequencies as low as 1% have been detected using as low as 5 ng of genomic DNA. Other enzymes reported to cleave at mismatches within DNA include: T4 endonuclease VII , T7 endonuclease I , deoxyinosine 3'-endonuclease from Escherichia coli, and modifications of endonuclease V. With some of the enzymes, the mismatch would be designed at the penultimate base, depending on the mechanism of the enzymatic cleavage and the substrate required for the particular enzyme at issue.

MIP probes based on the alternative enzymes listed above would function similar to that of the RNase H probes, e.g., as shown in Figure 16. The targeted base would reside either within the HR1 region or HR2 region of the MIP probe, depending on which side of the mismatched base the enzyme cleaves. The biotin would be incorporated within the sequence which would be removed upon cleavage. As with the RNase H-based probes, initial hybridization of the probe to the target can be carried out at elevated temperatures. The temperature can subsequently be lowered and cleavage of the mismatched bases carried out followed by hybridization of the second genomic homology region and ligation.

Figure 16 shows a mismatch double-stranded nuclease-form of the MIP probe for nucleases that cleave on the 5' side of mismatches in double-stranded DNA. The biotin **160** would be incorporated on the 3' portion of the probe **161** which would be removed upon cleavage by the nuclease. In Figure 16, two allele-specific forms of the MIP probe are presented in a technique to distinguish, e.g., a wild type sequence from a SNP variant. Each MIP probe contains a deoxyribonucleotide at the position to be identified (Figures 16A and 16B). MIP probes hybridize to the target. As a result of the higher melting temperature, the extended HR2 region preferentially hybridizes to the target, preventing hybridization of the shorter HR1 region. If the probe contains the deoxyribonucleotide not complementary at the position of the SNP (Figure 16B), the nuclease specific for mismatches in double-stranded DNA cleaves the probe, generating a free 3' end (Figure 16C). The short fragment of the deoxyribonucleotide leaves the target due to its low melting temperature. As a result of cooperative binding, the HR1 region on the MIP probe rapidly hybridizes to the template (Figure 16D) and is subsequently ligated (Figure 16E) to the HR2 region, generating the circularized MIP probe. Higher specificity of the overall reaction can be introduced by performing the initial incubation at elevated temperatures appropriate for the hybridization temperatures of the extended HR2 sequences. The temperature can then be reduced during the more rapid nuclease digestion and ligation steps.

Figure 17 shows a mismatch double-stranded nuclease-form of the MIP probe **170** for nucleases that cleave on the 3' side of mismatches in double-stranded DNA. The biotin **171** (or other affinity pair member, e.g., as discussed herein) would be incorporated on the 5' portion of the probe which would be removed upon cleavage by the nuclease. There are two allele-specific forms of the MIP probe. Each MIP probe contains a deoxyribonucleotide base at the position to be identified (Figures 17A and 17B). MIP probes hybridize to the target. As a result of the higher melting temperature, the extended HR1 region **173** preferentially hybridizes to the target, preventing hybridization of the shorter HR2 region **174.** If the probe contains the deoxyribonucleotide not complementary at the position of the SNP (Figure 17B), the nuclease specific for mismatches in double-stranded DNA cleaves the probe, generating a free 5' end (Figure 17C). The short fragment of the deoxyribonucleotide can then leave the target nucleic acid **172** due to its low melting temperature. As a result of cooperative binding, the HR2 region on the MIP probe rapidly hybridizes to the template (Figure 17D) and is subsequently ligated (Figure 17E) to the HR2 region, generating the circularized MIP probe. Higher specificity of the overall reaction can be introduced by performing the initial incubation at elevated temperatures appropriate for the hybridization temperatures of the extended target Homology Region HR1 sequences. The temperature can then be reduced during the more rapid nuclease digestion and ligation steps.

Flap Endonuclease Configuration. A variety of flap endonucleases or cleavases can also be used to generate padlock probes which can be subsequently ligated and cleaved to form the MIP probe (Figure 18). Flap endonucleases, cleavase I, and cleavase VIII have been used for allele-specific detection. The enzyme cleaves unpaired regions of DNA which form when the 5' end of a DNA sequence overlaps the hybridization site of the 3' end of an upstream oligonucleotide by at least one base pair.

An example of MIP probes using the flap endonucleases or cleavase enzymes are shown in Figure 18. The biotin is incorporated within the flap that would be removed following reaction of the probe with cleavase. The MIP probes would be hybridized to the target similar to the current OncoScan FFPE Assay. Following addition of the enzyme, the flap corresponding to the targeted base would be cleaved and any MIPs that retain their biotin-containing flap would be removed. Following ligation, the circularized MIP probe would be linearized and amplified.

As shown in Figure 18, the biotin would be incorporated on the 3' portion of the probe which would be removed upon cleavage by the cleavase. There are two allele-specific forms of the MIP probe. Each MIP probe contains the allele-specific base followed by a series of nucleotide (NNN in the example) which do not hybridize to the template. The substrates for cleavase enzymes are substrates that contain an upstream sequence hybridized directly adjacent to a flap (as in Figure 18A). Substrates containing a gap between the 3' end of an upstream sequence (see Figure 18B) and the flap are poor substrates for cleavases. Cleavase removes the substrate flap with SNP complementarity (Figure 18B), generating a product (Figure 18C) which can then be ligated, generating a product that can be converted into a circularized molecular inversion probe.

Chemical Cleavage Configuration. Nonenzymatic methods can also be used to cleave duplex DNA containing mismatches. [Rh(bpy)₂(chrysi)]³⁺ is a DNA intercalator that specifically binds in destabilized regions near DNA base mismatches. Upon photoactivation, the complex cleaves the DNA backbone. Specific DNA cleavage has been observed at over 80% of mismatch sites in all the possible single base pair sequence. The process for using chemical cleavage would be similar to that described under the "Mismatches Based Version" section, above. The biotin would be placed in the portion of the probe which would be removed during chemical cleavage.

### EXAMPLE 6 - ALTERNATIVE METHODS FOR LINEARIZING THE MIP PROBE

There is an advantage in incorporating a specific nuclease target in the MIP probes, so they can be readily and specifically linearized, e.g., to provide material appropriate for any number of detection techniques. For example, MIP probes often have uridine deoxyribonucleotides (dUMP) in the backbone, acting as substrate for uracil-N-glycosylase (UNG). After linearization, the probe nucleic acid can be analyzed by methods requiring linear polynucleotides. Further, universal primers can bracket the site of cleavage, e.g., for amplification by PCR approaches.

Performing PCR in the presence of dUTP and incorporation of UNG into the amplification master mix is the most frequently used method to control PCR contamination and is important when developing PCR-based diagnostic tests as these diagnostic tests are frequently conducted by a clinical testing laboratory that is processing numerous patient samples simultaneously and providing the results of the tests back to a physician. However, using dUMP in the MIP backbone complicates the use of UNG for PCR contamination control. This example describes a series of alternative substrates that can be incorporated into the MIP backbone and enzymes that can be utilized to achieve linearization of the circularized MIP probe.

Again, a typical design for a MIP probe is outlined in Figure 1. Three dU residues are incorporated into the backbone. The probe is hybridized to the template and circularized using the gap-fill reaction. Efficient PCR amplification requires linearization of the probe by removal of the dU residue by UNG and cleavage of the abasic site by heat or enzymatic cleavage. Alternative modified bases and enzymes can be used to accomplish the same goal. Although many of the enzymes have specificities for double-stranded DNA, a number of the enzymes have been shown to have activity on single-stranded nucleic acids as well. For example, hypoxanthine-DNA glycosylase can act on single-stranded DNA containing dI, although at 10 to 15 times lower efficiency than that seen with double-stranded DNA. Endonuclease V has been shown to nick both single-stranded and double-stranded DNA containing deoxyuridine. 3-methyladenine-DNA glycosylase from L-cells has activity on single-stranded DNA, 20 - 40% of that seen with double-stranded DNA. Alternatively, a generic complement to the region containing the modified base can be added to create a double-stranded template prior to, or simultaneous with, addition of the enzyme.

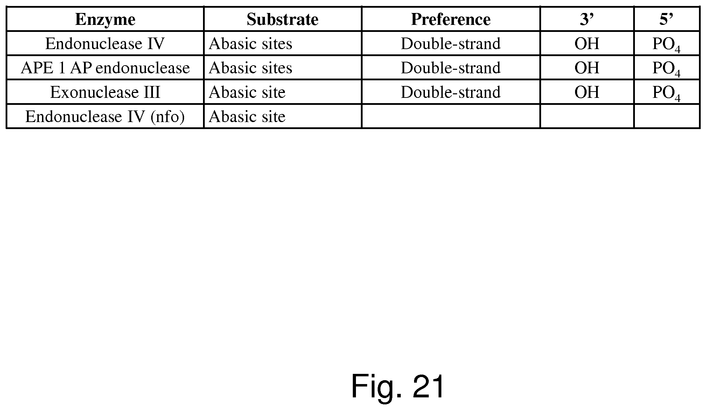

For those enzymes which lack lyase activity (Figure 19; Table 1), an additional enzyme capable of cleaving the abasic site will be required (Figure 21; Table 3). Alternatively, heat can be used to cleave the abasic site. Those enzymes which contain both DNA glycosylase and lyase activities (Figure 20; Table 2) remove the modified base and cleave the abasic site. Because the cleaved strand serves as a template for the subsequent PCR reaction, the chemistry of the cleaved end is not important (phosphate, OH, aldehyde, etc.).

### EXAMPLE 7 - MULTIPLEXED SNP GENOTYPING

This example is directed to a method of multiplexed analysis of nucleic acid sequences from several samples at once in the same mixed sample. In one technique, each sample would be used to accumulate a different specific set of SNP or other polymorphism MIP probes unique to the sample. Then the MIP reaction products from the various samples are pooled and processed together, e.g., on a single microarray or within a single NGS run. Information from each sample can be read against the background of the others because the assay is designed with no overlap of SNPs or other polymorphism targets of interest between the samples, and, for microarray detection, each product MIP probe has a separate array location for detection. In certain embodiments, e.g., microbiome assays or other assays where multiple organisms are at issue, particular SNPs are unique to particular samples (e.g., organism), thus identifying the presence of the organism on the array. In other embodiments, the same species may be at issue in the samples, but different polymorphisms are at issue because, e.g., the samples come from different generations of animals or plants (such as in animal and plant breeding programs).

This approach can be used to genotype multiple samples on a single array where each set of samples has a unique, non-overlapping set of SNPs of interest. Where there are X samples and sample requires genotyping of a unique set of Y SNPs, the total number of SNPs to be genotyped would be X * Y. Each sample is used individually to prepare amplified DNA target (e.g., circularized MIP product) from the set of SNPs unique for that sample. Amplified target is then pooled and hybridized to a single array where the array contains probes to enable a genotype call for each SNP. Since the list of SNPs is sample-unique, there is no need for sample de-convolution on the array. DNA target for this method can be prepared in a number of optional ways as long as different specific markers are amplified in each sample.

For example, the mixed sample input for multiplexed array analysis can be the product of a multiplex PCR where a unique primer pool is used for each sample. Because each array location is unique to a particular sample, there is no confusion as to what sample contributed a detected amplicon. The presence and/or quantity of each target sequence of interest can be determined. The array of signals from a sample is a unique fingerprint identifying that sample. Further, the presence of a particular sample can be inferred by, e.g., the detection of any one of the unique SNPs from that sample.

Optionally, the separate samples can initially be interrogated by molecular inversion probes. A unique set of MIP probes is used for each sample, resulting in a unique set of circularized MIP probe products. The circularized MIP probes could be detected directly. However, in many embodiments the MIP probes would have universal PCR primer binding sites and could be amplified after linearization. The PCR amplification could occur for all samples in the same reaction. Finally, the probes, and/or their amplicons, could be analyzed on a single microarray to identify and/or quantitate the presence of one or more nucleic acid targets at once for one or more different samples.

Oligonucleotide ligation assays (OLA) can also be used as a source of unique target mixes between different samples, in the present methods. The OLA technique amplifies a target in a first step, then confirms sequence with a specific ligase reaction that also labels the amplified target for detection. Initially target sequences are interrogated by hybridization with PCR primer. The primers are designed to include either the normal or mutant nucleotide at the 3' end and a tail of different lengths to distinguish various PCR products based on size at the 5' end. The PCR reaction is performed. In the ligation reaction phase of the assay, a common primer contains a fluorescent dye marker at the 3' end and meets the first primer right over the nucleotide position (e.g., SNP) that is under investigation for an altered allele. If the 3' end of the first primer matches perfectly with the target DNA, it will be brought into close enough proximity to the second oligonucleotide that both primers can be ligated together. No ligation occurs when there is a mismatch between the 3' end of the first primer and the target DNA. The OLA product, e.g., from reactions with several different samples) can then be subjected to analysis on a capture array to identify, e.g., what sequences are present, e.g., in what amounts. The information can indicate the identity of the samples the quantity or character of their genetic makeup.

In each case discussed above, the array for the read-out could use, e.g., a perfect match probe design (a design that is perfectly complementary), or could use a design that combines perfect match and one or more mismatch probes (with the mismatch probes having one or more base mismatches compared to the perfect match probes) in which probes for both the A allele and B allele are used.

### Example 8 - Elongated Gap-Fill Reactions

As discussed herein, the MIP the gap-fill reaction can fill a gap of one or more nucleotides between HR1 and HR2 on a target nucleic acid. Elongated fill regions can be useful to add informative and functional sequences to a MIP reaction product.

The complementary target sequences for the MIP HR1 and HR2 can be separated by a target nucleotide sequence of more than one nucleotide, so that specific MIP binding and polymerization provides an extended MIP gap fill product of two or more nucleotides. Gap-fill reactions can span from one nucleotide to more than 100 nucleotides, from 2 nucleotides to 50 nucleotides, from 3 nucleotides to 40 nucleotides, 5 from nucleotides to 35 nucleotides, from 10 nucleotides to 30 nucleotides, or from 15 nucleotides to 50 nucleotides.

Extended gap-fill products can be configured to function in a variety of sequence detection methods. For example, the extended region between HRs can present junctions, bar codes, and/or capture target structures functioning in various detection schemes such as, e.g., array capture assays, bDNA assays, PCR assays, multiplexed detections, and/or the like.

An extended fill MIP product can have a length and sequence suitable for an array capture assay method. For example, AXIOM^{®} Microarrays (Affymetrix, Inc., Santa Clara, California) typically have capture probes of about 30 nucleotides on an array. In addition, a labeling oligo (ligated to the capture probe in the presence of target) is typically about 9 nucleotides. So, a specific MIP product should present a target sequence of about 39 bases to the array. The HRs of the MIP are generally about 15 to 25 nucleotides each. Therefore, where the HRs are to be part of the captured sequence, it can be useful to have an elongated gap fill of at least, e.g., 9 nucleotides. If the key sequence to be interrogated is in the gap between the HRs, the MIP can be designed to provide a gap of, e.g., about 39 nucleotides. In still other cases, all or part of one of more HR can be included in the capture target sequence, with the length of the gap appropriately adjusted.

The gap between HRs in a MIP gap fill reaction can be adjusted to suit any number of assays, e.g., based on the teachings herein. The gap fill could be longer or shorter to comply with other assays. For example, BEADCHIP^{®} Microarrays (Illumina, Inc., San Diego, California) typically have longer capture probes of 79 bases, of which 50 are designed to hybridize with a target. In this context, the gap fill can be adjusted to provide an appropriately longer compatible MIP product.

### EXAMPLE 9 - METHOD FOR MULTIPLEXED GENOTYPING

This example describes several methods for genotyping SNPs using molecular inversion probes. The techniques allow for multiplexed SNP genotyping of a single sample or a set of samples.

Figure 22 depicts a structure of a molecular inversion probe before and after binding to DNA and inversion. The 3' end of the probe anneals to genomic DNA one base upstream of a polymorphism of interest to be interrogated. The assay includes a single-base extension step coupled with a DNA ligation event. The circular probe is then opened to form the inverted structure via an UDG/heat reaction.

Figures 23 and 24 depict three distinct samples each with a different genotype: the three samples represent a A/C heterozygous genotype sample (#1), a C/C homozygous genotype sample (#2), and a A/A homozygous genotype sample (#3). Tags 1, 3, and 5 encode the C allele for a single SNP (SNP #1) and for 3 different samples. Tags 2, 4, and 6 encode for the A allele for a single SNP (SNP #1) and for 3 different samples. All of these 6 MIP probes use the same homology region arms but each has a completely different tag sequence as indicated by the hatching patterns.

Each sample is separately interrogated with two pairs of MIPs. Each MIP in the pair is allele-specific, and has a different tag sequence. Thus, in order to genotype "N samples" at a level of "Y SNP genotypes per sample" the total number of MIPs that must be synthesized is N x Y x 2. For example, genotyping 100 samples at a multiplex level of 1000 SNPs per sample would require 200,000 unique MIPs.

The MIP reactions are carried out on individual samples and then can be pooled at some step after the probe inversion. For example, the circularized MIP products can be pooled after the exonuclease cocktail digestion step, the UDG/heat step, or the PCR step.

There are a variety of ways to read-out the MIP probes. For example, after linearization, the MIPs can be read out using next generation sequencing (NGS). The universal primers may be modified to accommodate specific needs of different NGS platforms. The entire linearized MIP probe product could be sequenced. Alternately, the probe tag could be released via cleavage and only the tag would need to be sequenced to identify the result since the tag encodes the locus under interrogation, the allele, and the individual sample. This approach differs from the use of MIPs for target enrichment since in this embodiment the MIP is needed to discriminate SNP alleles and NGS is used only for detection of the tag.

Alternately, the linearized MIPs can be read out on a universal tag array, e.g., with each tag encoding the locus being assayed, the allele at the polymorphic base, and the specific sample. A prepositioned cleavage site can be used to separate the tag from the homology region.

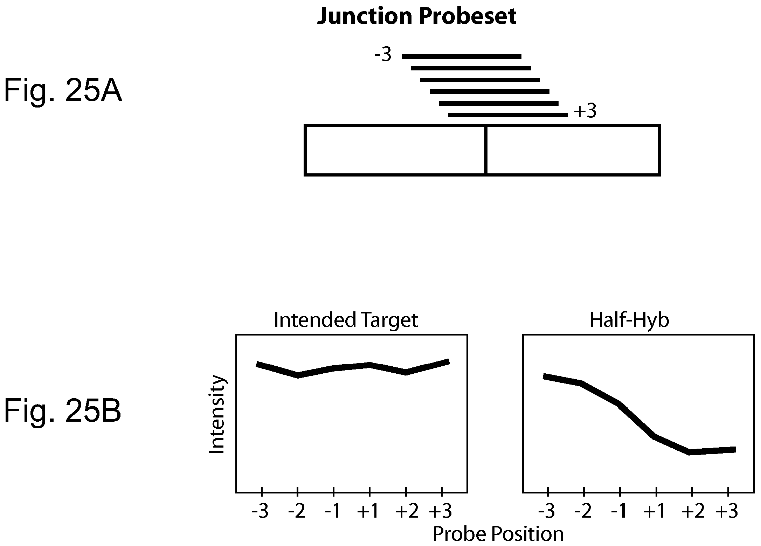

The linearized MIPs can also be read out using an array in which junction of a tag sequence and a homology region is interrogated in a tiling fashion with multiple probes on the array, thus obviating the need for a prepositioned cleavage site. The linearized MIP would be amplified and products hybridized to an array containing tag-genomic homology junction probes. The signal from these junction probes would encode the specific individual or sample along with the genotyped allele, providing an alternative to standard universal tag-based probe. However, there is limited flexibility in probe selection and there is a potential for undesirable cross-hybridization. This "half-hyb" problem can be mitigated by use of junction probeset as shown in Figure 25A. Half of the probe can be designed to detect the tag-genomic homology junction with a perfect match to the genomic homology sequence of the other alternative allele MIP. The junction probeset can be designed so that a full length intended target is strongly bound by all probes, while a half-hybrid target provides diminishing signal with advancement to the absent target segment, as shown in Figure 25B. Detection of full intended target will identify the presence of a particular SNP from a particular sample of origin.

In another method of multiplex SNP genotyping, circularized MIP products from complex mixes of samples can be deconvoluted by separate detection of SNP/sample information encoded into the MIP product. In this way, multiplexed SNP genotyping can be carried out on a large set of different samples.

In general, a SNP barcode (referred to as SBC) and an animal barcode (referred to ABC, although it should be noted that the "ABC" can refer to a specific plant or other non-animal sample source) are added at different steps of the protocol and then are finally brought together such that these segments become adjacent to one another. The SBC/ABC proximity in the DNA target sequences enables probes to be synthesized on an array to detect signals specific to various animals in the same detection step. Each animal sample can be interrogated by two MIP probes, e.g., to distinguish known alternate SNP bases at a position. Each MIP probe is allele-specific and has a different SBC sequence, thus in order to genotype 'N samples' at a level of 'Y SNP genotypes per sample' the total number of MIP probes that must be synthesized is Y x 2. For example, genotyping 100 samples at a multiplex level of 1000 SNPs per sample requires 200,000 unique MIPs. The present procedure has the advantage that it is also possible for the animal ID barcodes to be added after the MIP anneal and gap-fill reaction (or alternative to the gap fill reaction as discussed herein) and thus can use universal primers. The MIP reactions are carried out on individual samples and then are pooled at some step after the PCR amplification step wherein the ABC's are added as part of the PCR primer probes.

An exemplary assay can start with a MIP probe having first and second homology regions, a SBC tag separated from a homology region by a first cleavage site, and with PCR primer complements separated by a second cleavage site. See, e.g., Figures 32A to D. The SNP bar code (SBC) tag **320** can be encoded by a specific sequence, e.g., in the order of 8-10 bases or longer depending on the number of SNPs or other polymorphisms being interrogated. Following specific target hybridization, annealing and gap fill or alternative allelic determination reactions, the linearized (cut at second cleavage site) MIP product **321** is used as a template for PCR in which the animal specific barcode (ABC) **322** is introduced through the forward primer **323** in the PCR reaction (this forward primer can contain a 5' phosphate group; the ABC is, e.g., on the order of 8-10 bases, but again dependent on the number of different samples at issue). The ABC and SBC labeled PCR product is digested (at the first cleavage site **324**) the with, e.g., HaeIII restriction enzyme to uncouple the ABC/SBC tag from the genomic homology arms. The free linear ABC/SBC tag **325** is denatured to become substrate for a CircLigase or other enzymes capable of intramolecular ligation which forms a single strand circle **326** with the ABC and SBC tags abutting each other at a junction. Non-circular products are removed with an exonuclease cocktail. The circular products are opened with DNase I, then labeled using terminal deoxynucleotidyl transferase (TdT) DNA labeling reagent (DLR) before hybridizing to an array capturing the labeled ABC/SNP strands at specific locations. To mitigate false positive signals based on half-hybridization a probe tiling approach can be utilized, e.g., as described above and illustrated at Figures 25 A and B.

In other examples of the general concept, alternate cleavage strategies can be used. Again, following specific target hybridization, annealing and gap fill or alternative allelic determination reactions, the linearized (cut at second cleavage site) MIP product is used as a template for PCR in which the animal specific barcode is introduced through the forward primer in the PCR reaction. The first cleavage site is a HaeIII endonuclease substrate. The forward primer can contain a 5' phosphate group and several phosphorothioate (PS) linkages at the 5'end. The double stranded PCR product is digested with T7 exonuclease, which will digest the strand containing the 5' end that does not contain phosphorothioate linkages. This leaves remaining a single stranded ~120 base (or less) molecule in the order ABC/PCR primer/SBC/cleavage site/ homology regions/ PCR primer. A short synthetic oligomer is annealed to the single strand MIP product such that the duplexed segment re-generates the HaeIII restriction enzyme site. This partially duplexed molecule is digested with HaeIII to separate the ABC-UPS-SBC segment from the homology segment. The recognition sequence of HaeIII is GGCC so the oligomer used to recreate the double stranded structure may be NNNGGCCNNN, for example. Once the HaeIII digestion is complete, CircLigase is used to create an intramolecular circular molecule in which ABC is now adjacent to SBC. The circular molecule can be further processed, as described above, to specifically identify what animals have what SNPs, e.g., in a multiplexed detection format.

An alternative method to prepare single strand PCR product is to design the forward PCR primer with a 5'phosphate group and several PS-linkages and then digest the PCR product with T7 Exonuclease I to degrade the strand that is not resistant via phosphorothioate linkages.

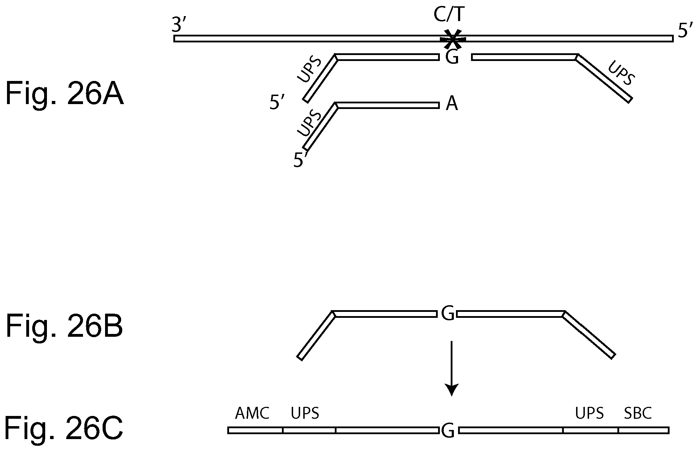

In still another similar approach, the ABC and SBC can be abutted using an oligomer ligation assay (OLA) rather than using MIP technology. For example, as shown in Figure 26A, OLA probes having universal PCR probe sites will hybridize to target, but only the probe complementary at the SNP site of interest will ligate. The resultant ligation product (Figure 26B) can be used as a PCR template in which animal barcodes and SNP or locus-specific barcodes are introduced (see Figure 26C). The PCR product can be denatured and then the single strands can be circularized using CircLigase enzyme, after a 5' phosphate group is added using ATP + T4 polynucleotide kinase or using a PCR primer containing a 5' phosphate group. Circularization of the PCR product brings ABC adjacent to SBC and this can be detected using a hybrid ABC-SBC probe on an array. Multiple probes on the array can be tiled through the ABC-SBC junction in order to mitigate potential half-hybridization issues, as discussed above.

### EXAMPLE 10 - INCREASING THE YIELD OF SITE-SPECIFIC MIP PROBES

During the gap fill reaction using MIP probes for genotyping, it is possible for the DNA polymerase to incorporate more than one nucleotide and cause polymerase mediated strand displacement which decreases the yield of MIP probes available for subsequent ligation. This would occur primarily in homopolymer repeats but under certain conditions could even occur in non-homopolymer regions of the template DNA.

We have found that using a modified NTP that bears a polymerase inhibiting function at the C5 or C7 position of the pyrimidine or purine (respectively) nucleobase will limit polymerase mediated gap fill to the incorporation of one and only one nucleotide. The structure of the modified nucleotides is similar but not exactly described in US 8,071,755. The primary difference between the modified nucleotides of this example and those described in US 8,071,755 is that the modified nucleotides of this example do not require that the purine or pyrimidine dNTP carry a fluorescent label. The modified nucleotides after incorporation into the MIP probe may or may not require a chemical treatment to remove the polymerase inhibiting portion of the purine or pyrimidine modification depending upon whether or not the polymerase inhibitor also inhibits the post gap-fill ligation mediated circularization of the MIP probe.

### EXAMPLE 11 - TARGET DETECTION BYCAPTURE OF RELEASED MIP ENDS

Detection of a successful MIP hybridization to target can be by detection of an associated free MIP removable region. In above described techniques, there is often a fragment released as a result of a specific cleavage event, e.g., RNase of a specifically hybridized ribonucleotide, enzymatic cleavage of a specific mismatch, cleavage at an unnatural base, specific chemical cleavage at a mismatch, and/or the like. Detection of the specific cleavage event is often by analysis for a resultant circularized MIP. However, detection of specific hybridization can be by identification of released removable groups.

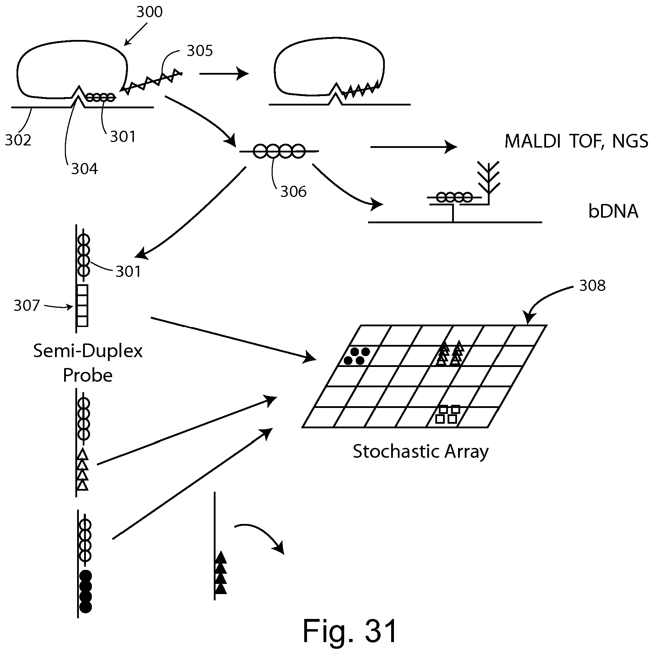

As shown in Figure 31, a MIP **300** having removable region **301** can hybridize to a target nucleic acid **302** generating a substrate **304** for a specific chemical or enzymatic cleavage. Departure of the removable region can be hastened by competition with a stronger binding MIP homology region **305.** Released removable region can be detected to confirm there has been a specific hybridization to the target nucleic acid.

Released removable region **306** can be modified to make it readily amplified and/or detected. For example, one or more PCR primer binding sites can be attached to the ends of the released removable region for ready PCR amplification and detection. Adaptors can be previously designed into the removable region or attached (e.g., by ligation) to make it readily sequenced, e.g., by NGS.

Alternately, the released removable region can be labeled, isolated, and/or detected using a semi-duplex probe. A semi-duplex probe can have a double stranded utility segment and a single stranded capture segment. The semi-duplex probe **307** can have a sequence complementary to the removable region, functioning to capture the released removable region. The utility segment can have various functions useful in the amplification, capture, and/or labeling of the captured removable region. For example, the captured removable region can be ligated to one strand of the semi-duplex probe, creating a junction detectable as described herein, e.g., by bDNA, NGS, and/or using junction probe set. In certain embodiments, the semi-duplex probes can be configured as stochastic probe sets useful in counting the number of target sequence copies in a sample, as described below.

### EXAMPLE 12 -TARGET NUMERATION USING STOCHASTIC PROBES

The number of target nucleic acids in a sample having a particular sequence can be determined using stochastic detections (see, e.g., US application 2013/0116130), e.g., wherein one or more copies of a MIP process product is randomly labeled with a specifically detectable reporter.

For example, for a given released removable region **301** (associated with a target sequence of interest) a set of 10 different stochastic probes can be provided. Each stochastic probe can have a single stranded segment to specifically hybridize and capture a copy of the released removable region. If the sample tested contained three copies of the target sequence of interest, three copies of the removable region would be released during the MIP process. Of the ten different stochastic probes three different probes would hybridize to the three released removable regions. Determination of the number of hybridized stochastic probes will provide the number of released removable regions , and the number of the target sequence in the original sample.

As shown in Figure 31, the semi-duplex probes can be generated in a set with the same single stranded capture segment, but with detectably different utility segments (e.g., having different tag ID sequences). Detection of three different hybridized stochastic probes indicates there were three released removable regions present.

Hybridized stochastic probes can be detected by any appropriate technique. For example, hybridized probes can be processed to make them more sensitively, quantitatively, and/or qualitatively detectable by any number of analysis techniques. For example, amplification primer sites or NGS adaptors can be incorporated or attached.

In one aspect, the hybridized stochastic probes can be quantitatively amplified (e.g., by qPCR) and captured on a solid support array **308.** Each different hybridized stochastic probe can be captured at a different location on the array. The number of locations whereon probes are detected will reflect the number of released removable region copies (and original sample nucleic acid target of interest copies). If two copies of the released removable region each were captured by a stochastic probe with the same label the total number of copies could still be determined, e.g., by noting array positions with signals twice as strong as others.

In alternate strategies, circularized MIP product, e.g., from any MIP process described herein, can be quantitated using stochastic probes in a fashion similar to the process described for detection and quantitation of released removable regions. For example, circularized product can be cleaved (as described herein) and hybridized to a set of stochastic probes, for detection and quantitation, as described above.

### EXAMPLE 13 - NGS MULTIPLEXED MIP ASSAYS

In this example, the basic idea is to use NGS to read out MIP results instead of using an array detection format.

As shown in Figure 27, NGS detection and quantitation of multiple mutants or SNPs can be carried out after initial MIP interrogation. Initial steps of the MIP assay (e.g., the OncoScan^{®} FFPE Assay) technique are completed, e.g., anneal **270,** gap-fill and ligation **271,** exonuclease clearing of non-circular nucleic acids **272,** cleavage **273,** and PCR amplification **274.** However, the product at this stage is subjected to next generation nucleic acid sequencing **276,** optionally after addition of any required NGS adaptors **275.**

In many cases it can be beneficial to run a second PCR amplification using with primers allowing NGS sequencing. However, it is possible to provide all necessary primers for both first PCR amplification and NGS sequencing in a single amplification. For example, as shown in Figure 28, the first stage PCR primers can have, e.g., both sequences priming the universal primer sequence of the linearized MIP product and a further tail useful for either a second round of PCR amplifications or configured as NGS functional sequences. The primer probes can include a typical (e.g., 17-mer) primer sequence complementary to the universal target designed into the MIP probe system. Further, the remaining sections of the, e.g., 60 to 80-mer primer can include sequences necessary to any number of NGS sequencing protocols. Optionally, the primers can include an overlap region functioning as a target for a second PCR amplification scheme.

One-step PCR for incorporating NGS primers could include pairs of 60-80mer primers including all NGS-needed sequences. However, there is a risk of poorly-functioning primers, e.g., due to complications with hairpins, steric inhibitions, enzyme interference, and the like. Optionally, primers could be nested through process steps. A first amplification can employ shorter common primers, e.g., with 5'-NGS platform sequences and 3'-OSv3 tails. Then an outer NGS primer set to amplify inner-step PCR. One side of the second primer set can contain barcode sequences, if there will be two or more samples per run. Some assay processes could even have a three-step PCR based on shorter primers.

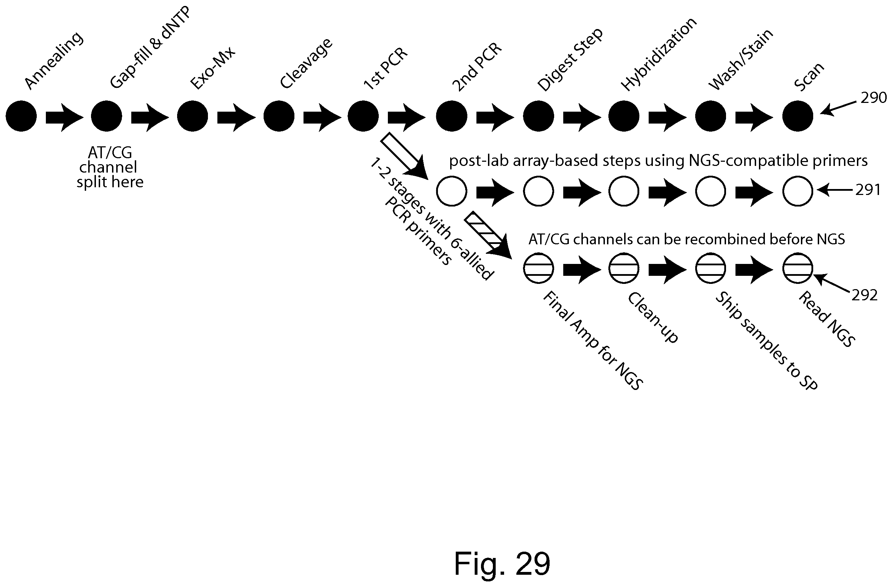

There are many options on process configuration for assays combining MIP technology with NGS readout. As shown in Figure 29, amplification and reading can branch from the first amplification step to provide the amplification and/or structural modification adjustments necessary to successfully cooperate with any of various NGS schemes. For example, the first PCR of the linearized MIP product can take place with standard PCR primers or with primers having NGS compatible tails. The MIP product can go a second standard PCR and on to a NGS scan **290,** e.g., if the NGS technique requires a higher copy number of genomic fragment for sequencing. Alternately, the MIP product can have a standard first PCR amplification followed by a second amplification introducing 5' sequences configured for a NGS reading **291.** Optionally, the MIP product can experience multiple amplifications and tailing before NGS reading **292.**

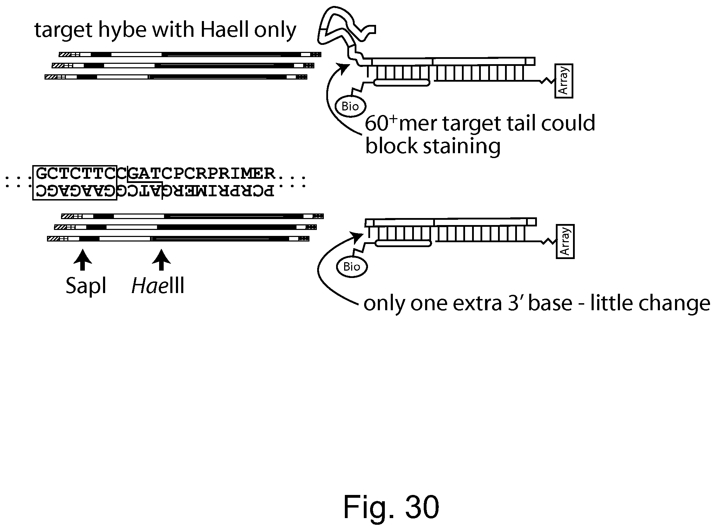

To avoid some problems that may arise when using long tailed PCR primers, such as with a subsequent array readout, the primers may be designed to include a cleavage site to remove excess sequences, once they are no longer needed. For example, in Figure 30 a restriction endonuclease site **300** has been included in a MIP probe so that the PCR primer, and other upstream primer, tail and cleavage sequences can be removed before steps where they may interfere.

In many of the NGS techniques, it can be useful to incorporate additional information into a MIP reaction product. In many schemes, the nucleic acid for detection can be provided with multiple tags or other informative sequences. As discussed below in Example 14, it is possible to include a tag between segments of an HR sequence. Such an arrangement can provide multiple relevant encoded data in a very compact MIP product or fragment thereof. For example, two samples can be separately probed with different MIPs, each of the MIP sets having a different sample ID tag within an HR sequence, but probing the same population of SNP targets. After the initial target binding and circularization, the circularized MIPs from both samples can be mixed and detected in the same NGS assay. The compact and connected nature of the tagged HR makes it well suited to interrogation by many NGS techniques. Regardless of the relationship between multiple information encoding sequences, they can be useful in matrix type analyses. Providing multiple informative sequences in the product for analysis can allow mixed MIP reaction products to be analyzed together in NGS (or other parallel assays) so that a detected signal can be assigned to, e.g., a sample, target, SNP ID, sample collection time, and/or the like.

The field of next generation sequencing has recently expanded dramatically. The products of MIPs assays can complement any of these techniques, e.g., with appropriate provision of capture sequences, primer sequences, and/or the like. Three commonly utilized NGS techniques are the MiSeq, MiSeqDx, NextSeq, and HiSeq platforms by Illumina, Ion PGM and Ion Proton platforms by Thermo Fisher Scientific, or the PacBio RS II platform by Pacific Biosciences.

In the Ion PGM and Ion Proton platforms, nucleic acid fragments are modified with specific adaptor sequences and captured and amplified using emulsion PCR on the surface of micro beads. The beads are introduced into pH-sensitive wells of a semiconductor. Sequencing progresses as different NTPs are introduced one at a time and pH drops are detected only in wells wherein the NTP is added to the captured nucleic acid. The signals are tracked for each well and the data can be compiled to determine exactly what NTP bases were added in what sequence at each well. This technology is compatible with the MIP processes described above. For example, the amplification steps can be configured to incorporate or attach appropriate chemical or sequence targets for capture by the microbeads of the assay.

The Pacific Biosciences single molecule real time (SMRT) sequencing system is also compatible with MIP products described herein. In the SMRT system, DNA polymerase enzyme is attached to the bottom of a zero-mode waveguide well. The system may include many wells for massively parallel processing. A single molecule of a nucleic acid of interest is introduced into a well along with a fluorescently labeled nucleotide. If the polymerase incorporates the fluorescent nucleotide while replicating the nucleic acid, it is subject to excitation. Detection of an associated emission in the well signals the incorporation of the base in the well. Again, the data can be evaluated for each well to determine the order of NTP incorporation. The MIP products are compatible with this technology. The identity of the MIP product source can be read, e.g., based on a bar code element made unique to the genomic target during MIP processing.

The MiSeq, MiSeqDx, NextSeq, and HiSeq platforms by Illumina use a variant of the sequencing by synthesis concept. Surface capture adaptors and adapter sequences are incorporated onto the ends of nucleic acid fragments of interest. The adapted fragments are diluted and captured randomly distributed on a substrate including closely spaced capture elements corresponding to the two capture adaptors on each nucleic acid. A nucleic acid captured by the first capture adaptor and amplified by bridge amplification on the surface to produce amplicons which are captured on the other end by a second capture adaptor, forming a bridge across the surface between capture elements. Over the course of several amplification cycles the synthesized strands bridge and walk across the substrate, forming a cluster of target nucleic acid copies. To read the sequence of the nucleic acid, polymerase synthesis takes place with stepwise introduction of certain fluorescent NTPs to the substrate. If the NTP is incorporated at a particular cluster, a fluorescent signal is detected, e.g., using photoimaging equipment. Data is collected on what NTPs resulted in signals from what clusters in what order. The MIP products discussed herein are compatible with the Illumina sequencing by synthesis approach. Using techniques discussed above, appropriate capture adaptor, and bar code sequences can be incorporated into MIP products for sequencing by synthesis sequence determination.

Next Generation Sequencing technologies, in combination with MIP products, can provide adequate data for sequencing of multiple sequences of interest in a single (e.g., clinical patient or agricultural) sample or multiple samples within a single sequencing run. Further, the combination can provide adequate quantity and quality of information to determine copy numbers of multiple sequences (e.g., genes, mRNA, cDNA) of interest.

### Example 14 - TAGS WITHIN HOMOLOGY REGIONS

In a typical MIP assay a barcode tag can be included in the inversion probe backbone. The bar code can provide any desired information, e.g., sample identification, intended SNP target, and/or the like. Probes circularized at the target are protected from nucleases and can be linearized for detection, e.g., on a capture array. The linearized probe can be captured, e.g., at the tag sequence to present a detectable signal at an array location identifying the sample sequence, e.g., a SNP.

However, there are assay schemes (e.g., matrix detections of multiple samples and multiple targets) wherein it is useful to have multiple unique sequences. For example, two samples can be separately probed with different MIPs, each of the MIP sets having a different sample ID tag, but probing the same population of SNP targets. After the initial target binding and circularization, the circularized MIPs from both samples can be mixed and detected on the same array. Optionally, the mixed circularized MIPs can be linearized and bound to locations on the array associated with certain SNP sequences. Two different detection probes with different detectable signals can be hybridized to the array to bind the sample identification tags. At the detection step, different SNP array locations might exhibit reporter signals, e.g., of different colors depending on what SNPs were in what samples. One can see it is useful in matrix assays to retain sample and target information on the probe, throughout the hybridization and detection steps.

In some processes described herein, the entire circularized or linearized MIP does not make it to the final detection step. Further, a full length linearized MIP may have undesirable kinetics or be too large for some detection techniques. For example, certain NGS methodologies, in PCR detection of the linearized MIP, or in schemes wherein the linearized probe is to act as a PCR primer, the full length MIP product may not be suitable.

In cases where it is desirable to retain two types of MIP information close together in a short length of nucleic acid, we envision that the tag sequence can be incorporated between two sections of homology region sequences. For example, a sample ID tag can be inserted with a first and/or second genomic homology region. When the MIP hybridizes to the target sequence, the 3' and 5' segments of the homology region can bind with the target, with the tag sequence looping out from the specifically hybridized sequences. The 5' and 3' ends of the first and second homology regions can still align (e.g., with or without a gap between) on the target to be extended (as necessary) and ligated. An advantage can be that such configurations can have fewer false positive binding events at the sample target nucleic acid due to greater specificity as non-specific hybridization to a nucleic acid other than the intended target sequence is more likely to disrupt the formation of a stable nucleic acid duplex prior to extension (as necessary) and ligation, as compared to MIPs with homology regions designed to hybridize across an continuous region of a target nucleic acid.

Cleavage sites can be located to provide modified MIP products of shorter length with higher information concentration. For example cleavage sites can be engineered into the ends of the homology regions and/or within the MIP backbone (but abutting or within a small number of bases to the ends of the homology regions) to remove all sequences but the homology regions with their integrated tags. For example, a pair of homology regions and a tag may have a length of less than about 25 bases to more than 100 bases, from 40 bases to 75 bases, or about 65 bases, including the tag. The tags can include enough bases to encode the desired information. Typically the tags range in length from more than about 40 bases to less than about 4 bases, from 25 bases to 10 bases, or about 12 bases. If a cleavage site were located within or between the ligated homology regions, a fragment of about 20 bases could be generated including the homology region (or at least the portion thereof that included the base(s) interrogator nucleotide(s)) with an integrated tag. Taking it further, if a cleavage site were located between the tag and one segment of the associated homology region, the construct containing both tag and homology region (e.g., retaining SNP information) could be present in a fragment of about 15 bases. Such short fragments would be suitable, and highly information laden, substrates for certain next generation sequencing schemes, e.g., in highly multiplexed analyses. For example, the sequencing readout of such MIP assays would include both tag sequence(s) and also the interrogation of the genomic target, such as the SNP, indel, etc. that was encompassed by the gap fill or is otherwise interrogated by the MIP assay if no gap fill is utilized. Additionally, incorporation of a tag within both homology regions can easily facilitate additional encoded information while adding relatively minimal length (e.g., 10, 12, 15, 20 bases) to the end product to be sequenced or otherwise read out and detected.

The tag can be inserted anywhere within the HR. In most cases, the tag is inserted at about the middle of the HR. However, the tag can be inserted at a point 50% along the HR, 60%, 75%, 90%, or 95% along the length of an HR, e.g., starting from the 3' or 5' end of the HR.

In another aspect, MIP products having a tag inserted within a homology region sequence can act as unique PCR primer target sequences. A signal based on a PCR primer complementary to the homology region and tag could confirm both sample identification and sample target sequence. If an MIP product had the same or different tag sequence inserted into each of the two homology regions, this could provide two PCR primer target sequences. Such unique PCR primer targets could represent the two ends of a sequence to be amplified by PCR. Alternately, the two PCR primer targets could be separated to different fragments for amplification.

In another variation, additional tags can be incorporated within a PCR amplification. While one or more tags can be incorporated within genomic homology regions, tags can also be added during the PCR amplification step. This can result in two (or three or more) total tags. For example, if the two tags within the genomic homology regions are for biomarker (e.g., SNP) and sample, the PCR primer added tag could be for time point (e.g., if there are multiple samples for a particular person, e.g., one sample taken before treatment and a second sample taken after treatment). This way, the same MIPs could be used, but different primer tags to differentiate time points.

### Example 15 - MIP SINGLE REACTION V. SUBPANEL DETECTION

As described above, an AXIOM^{®} array is capable of detecting thousands of nucleic acid fragments of different sequences. Further, nucleic acid fragments from multiple samples can be detected and distinguished on the same array, e.g., if they are ultimately labeled with a different reporter fluorophore. There are, however, circumstances where it is desirable to assay one sample against one probeset, and other samples against another relevant probeset. That is, instead of assaying each sample for the same complete set of targets (e.g., in a MIP assay, using the same panel of MIPs with each sample), it would be desirable to react each sample only with those relevant probes or probe panels of interest.

We envision scenarios where only interrogating certain samples for certain biomarkers can provide all of the data of interest without needing to analyze all targets, which in turn allows greater multiplexing by allowing different samples to be pooled and analyzed together at an appropriate step within the assay at issue. If different species are being analyzed within the multiplexed samples, irrelevant assays for target nucleic acids that will not be present or that will not provide useful information can be avoided. For example, in an agricultural biology context, various species of livestock or crops can be probed only with MIPs of interest, but detected in the same PCR, array, or NGS pool. Similar benefits can be realized in the context of clinical diagnostic sequences, human microbiome pattern evaluations, screening multiple clinical samples of a patient for various microbe species, and/or the like.

To test a proof of concept, a set of samples were assayed against a single large set of MIPs and compared to the results for the same set of samples assayed against the same MIPs but reacted in 10 different MIP subpanels:
1) 48 DNA samples were processed in 3000-plex (pure) of MIPs. Those MIPs circularized by hybridization to target were detected on an Axiom^{®} array with 46 of 48 (95.8%) passing developmental QC as releasable results.
2) The same 48 DNA samples were processed across ten 300-plex (pooled before reading) MIP subpanels with all 48 samples being processed across each subpanel. The circularized MIPs were detected on the same type of Axiom^{®} array with 38 of 48 (79.2%) passing developmental QC.

The comparison of pure 3000-plex MIP reaction detection to the ten 300-plex reaction pool detection was carried out, as follows.
I. Annealing - Add 6.60 µL genomic DNA to 96 well MIP reaction plates (79.2 ng at 12.0 ng/uL).
Anneal Master Mix was prepared on ice; vortex between additions:
Prepare 3 subpanel plates on 2/5/2015.
Prepare 2 subpanel + full panel plates on 2/9/2015.
Add 3.4 uL Anneal Master Mix to each sample well for a 10 µL volume per sample.
Add from a strip-tube layout of 4 wells X 2 rows for each subpanel half-plate.
Fill the anneal wells column-wise in pairs with 24-channel P20 (pipette six times from left to right).
Seal the plates and vortex at maximum speed for 4 seconds. Spin down the plates at 2400 rpm for 60 seconds.
Place the plates on thermal cyclers and stagger-start the anneal program 98°C, 3 min; 85°C, 30 min; 60°C, 60 min, then 56°C, 16 hr.

II. Gap-Fill - Thaw Buffer A, Betaine, NAD, dNTPs, Cleavage Tube, and Amp Mix required.
Prepare Gap-Fill Master Mix on ice according to the table below. Vortex between additions:

| **Gap-Fill Master Mix** | | | | | | | |
|---|---|---|---|---|---|---|---|
| **Part Number** | **Supplier** | **Reagent** | **[Stock]** | **[MM]** | **[In Rxn]** | **Units** | **1 Sample (µL)** |
| 902253 | Affymetrix | Water | - | - | - | - | 4.47 |
| A57-40775 | Epicentre | 10X Ampligase Buffer | 10 | 1.00 | 0.50 | X | 1.00 |
| 77507 | USB | Betaine | 5 | 0.75 | 0.375 | M | 1.50 |
| B90075 | NEB | NAD+ | 50 | 2.00 | 1.00 | mM | 0.40 |
| 77212 | USB | dNTPs (each of 4 nucleotides in mix) | 10000 | 30.00 | 15.00 | uM | 0.03 |
| A32750 | Epicentre | Ampligase (5 U/uL) | 5 | 0.50 | 0.25 | u/µL | 1.00 |
| M0530L | NEB | Phusion Polymerase (2 U/uL) | 2 | 0.32 | 0.16 | u/µL | 1.60 |
| **Total** | | | | | | | **10.00** |

| **Volume Per Tube for 24-tube Strip** | | | | | | | |
|---|---|---|---|---|---|---|---|
| | | | | | | | |

Remove the Anneal plate from the thermal cycler and chill it on the cold block for 1 minute. Start Gap Fill program and pause it at 56°C.
Spin down the Anneal plate at 2400 rpm for 30 seconds.
Add 10 µL of Gap-Fill Master Mix per well to the chilled Anneal plate. Mix by pipette 3x. Volume per sample now is 20.0 µL.
Seal the plate and vortex at max speed for 4 seconds. Spin down the plate at 2400 rpm for 60 seconds.
Start the Gap Fill program, which had been paused at 56°C. Set a timer for 80 minutes. Cycle - 56°C, 60 min; 72°C, 20 min; 37°C, pause at the start of the exonuclease step.

III. Exo-Mix Addition - Prepare Exo-Mix on ice. Vortex between additions.
Remove the 1st Stage PCR plate after 20 min at 72°C, place it on the cold block for 1 minute.
Spin down the 1st PCR plate at 2400 rpm for 30 seconds.
Allow the thermal cycler to ramp down to the start of the 37 °C incubation. Pause thermal cycler.
Add 5 µL of Exo Master Mix per well to the chilled 1st Stage PCR plate. Mix by pipette 3x. Current volume per sample: 25.0 µL
Seal the plate and vortex at max speed for 4 seconds. Spin down the plate at 2400 rpm for 60 seconds.
Resume the Gap Fill program, which had been paused at 37 °C (set a timer for 65 minutes)- 37°C, 45 min; 80°C, 20 min; 37°C pause at the start of the cleavage step.

IV. Cleavage Mix Addition - Prepare Cleavage Master Mix on ice according to the table below. Vortex between additions.

| **Part Number** | **Supplier** | **Reagent** | **[Stock]** | **[MM]** | **[In Rxn]** | **Units** | **1 Sample (µL)** |
|---|---|---|---|---|---|---|---|
| 902257 | Affymetrix | Cleavage Buffer | 1 | 0.99 | 0.45 | X | 19.00 |
| 902258 | Affymetrix | Cleavage Enzyme | 2 | 0.02 | 0.00952 | U/µL | 0.20 |
| **Total** | | | | | | | **19.20** |

Pause the thermal cycler at the start of second 37 °C incubation (after the completion of Exo-Mix denaturation step at 80 °C for 20 minutes).
Remove the 1st PCR plate and place it on the cold block for 1 minute.
Spin down the 1st PCR plate at 2400 rpm for 30 seconds.
Add 19.0 µL of Cleavage Mix per well to the wells in the assay plate. Mix by pipette 3x. Volume per sample now 44.0 µL.
Seal the plate and vortex at maximum speed for 4 seconds. Spin down the plate at 2400 rpm for 60 seconds.
Resume the Gap Fill program, which had been paused at 37 °C. Set a timer for 25 minutes - 37°C, 15 min; 95°C, 15 min; 4°C hold.
Chill the post-cleavage plate 60 seconds. Vortex at max speed for 4 seconds. Spin down the plate at 2400 rpm for 60 seconds.
Freeze all cleavage products and store at -20°C until all plates have reached this stage.

V. Post Cleavage Pipetting - For the 300-Plex rows, pipette 48 wells x 3 uL from 10 cleavage half-plates to a new PCR plate
For the 3000-Plex plate, transfer 48 wells x 30 uL of post-cleavage reaction into rows on the same PCR plate.
Save all the Cleavage Plates in the freezer.

VI. Amplification Mix Addition - Prepare PCR Master Mix on ice according to the table below. Vortex between additions.

| **Shendure PCR Master Mix for 30 cycle PCR (15-Dec-2014)** | | | | | | | |
|---|---|---|---|---|---|---|---|
| **Part Number** | **Supplier** | **Reagent** | **[Stock]** | **[MM]** | **[In Rxn]** | **Units** | **1 Sample (µL)** |
| 902253 | Affymetrix | Water | - | - | - | - | 21.03 |
| B05185 | NEB | Phusion HF Buffer | 5 | 1.80 | 1.00 | X | 13.50 |
| 77212 | USB | dNTPs (each of 4 nucleotides in mix) | 10000 | 360.00 | 200.00 | uM | 1.35 |
| M0530L | NEB | Phusion HS-II Polymerase (2 U/uL) | 2 | 0.07 | 0.04 | U/µl | 1.35 |
| | IDT | OncoScan PCR Capture Primers (both F&R) | 125 | 0.90 | 0.50 | uM | 0.27 |
| **Total** | | | | | | | **37.50** |

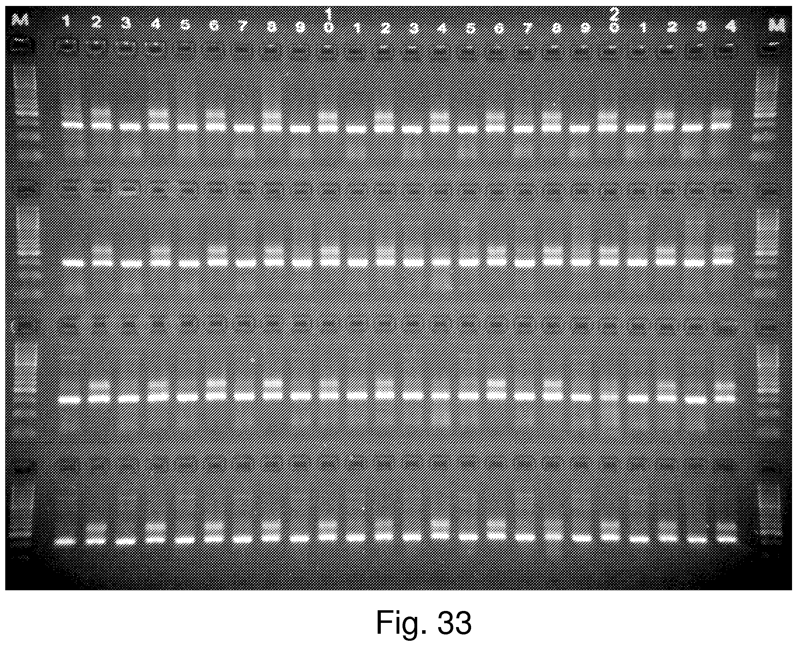

Add 37.5 uL master mix to all 88 (post-cleavage pipetted) wells with 30 uL Cleavage Product. Mix well.
Seal, gently vortex & spin down. Run program (Shendure-26c-veriflex) on the Veriti TC.
Shedure PCR (original) 95°C, 2 min; 98°C, 15 sec; 60°C, 15 sec; 72°C, 45 sec; repeat for 26 cycles.
Bring to 72°C for 5 min, and hold at 4°C.
After the completion of the PCR remove the plate from the thermal cycler.
Prepare a PCR QC Gel plate (2 µL Gel Loading Dye, 8 µL of sample from the PCR plate)
Run the QC 3% Agarose gel for the PCR products.
ODD wells = 300-plex mixed samples from rows A-D
EVEN wells = 3000-plex original samples (33 Hybrid + 15 Test-Lines) from rows E-H (See Figure 33).

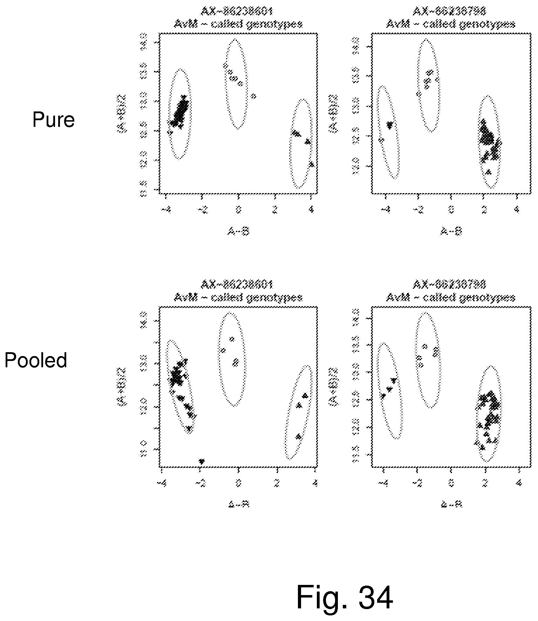

Figure 34 shows called SNP genotype readouts for a couple of the samples that were reacted in a master panel of 3034 probes versus the same samples in 10 separate reactions with subpanels of the same probes in MIP groups of about 300. Note that resolution and detection are similar whether the samples were subjected to the MIP reaction in the master panel or in separate sub panels before detection.

While the foregoing invention has been described in some detail for purposes of clarity and understanding, it will be clear to one skilled in the art from a reading of this disclosure that various changes in form and detail can be made without departing from the true scope of the invention. For example, all the techniques and apparatus described above can be used in various combinations.

## Claims

1. A composition comprising:
a molecular inversion probe (MIP) comprising
a first homology region (HR1) sequence at a 5' end wherein the HR1 comprises a sequence complementary to a target nucleic acid and
a second homology region 2 (HR2) sequence at the 3' end, wherein the HR2 comprises a sequence complementary to the target nucleic acid, and
wherein a target sequence complementary to HR1 is located 5' and separated at least 5 nucleotides from a target sequence complementary to HR2; and,
one or more oligomer insert nucleic acid(s) having a sequence at least 80% complementary to the at least 5 nucleotides separating the HR1 and HR2 regions,
wherein the oligomer insert nucleic acid(s) are designed to hybridize in the gap between the homology regions to the target nucleic acid such that the HR1 and HR2 ends each abut the oligomer.

2. The composition of claim **1,** wherein the oligomer insert is 100% complementary to the allele occurring most frequently in a selected population (wild type) and the oligomer insert mismatches at least one nucleotide at the position of a putative SNP or variant allele, and/or wherein the oligomer insert ranges in length from 5 nucleotides to 15 nucleotides.

3. The composition of any one of claims **1 to 2,** wherein the oligomer insert mismatches with the target nucleic acid by from one to three nucleotides.

4. The composition of claim 1 to **3,** wherein the MIP comprises additional nucleic acid regions selected from the group consisting of: a tag ID, a first cleavage site, a second cleavage site, a forward (e.g. PCR) primer binding site, and a reverse (e.g. PCR) primer binding site.

5. The composition of claim 1 to 4, wherein the oligomer insert is designed to hybridize with the target in the gap with stringency adapted to not bind significantly in the presence of a mismatch.

6. A method of identifying a target sequence of interest, the method comprising:
providing a composition according to any one of claims 1 to5;
providing a putative target nucleic acid comprising a sequence complementary to the HR1, and comprising a sequence complementary to the HR2, wherein the sequence complementary to HR1 is located 5' and separated at least 5 bases from the target nucleic acid sequence complementary to HR2;
hybridizing the MIP and oligomer insert to the putative target nucleic acid;
contacting the hybridized nucleic acids with a ligase;
determining whether the MIP has been ligated to the oligomer insert; and,
confirming the putative target sequence is the target sequence of interest if the MIP is determined to be ligated to the oligomer insert.

7. The method of claim 6, further comprising, determining the MIP has not been ligated to the oligomer insert; thereby confirming the putative target sequence comprises at least one mismatch with the oligomer insert.

8. The methods of claims 6 or 7, wherein the MIP comprises a first cleavage site, a forward PCR primer binding site, and a reverse PCR primer binding site.

9. The method of claim 8, wherein said determining ligation comprises:
cleavage of a circularized MIP between PCR primer binding sites; and
amplification of the cleaved MIP by PCR.

## Patentansprüche

1. Zusammensetzung, umfassend:
eine molekulare Inversionssonde (MIP), umfassend
eine Sequenz der ersten Homologieregion (HR1) an einem 5'-Ende, wobei die HR1 eine Sequenz umfasst, die zur Zielnukleinsäure komplementär ist, und
eine Sequenz der zweiten Homologieregion (HR2) am 3'-Ende, wobei die HR2 eine Sequenz umfasst, die zur Zielnukleinsäure komplementär ist und wobei eine zu HR1 komplementäre Zielsequenz 5'-seitig angeordnet und um mindestens 5 Nukleotide von einer zu HR2 komplementären Zielsequenz getrennt ist; und
eine oder mehrere Oligomer-Insert-Nukleinsäure(n), aufweisend eine Sequenz, die zu mindestens 80 % komplementär zu den mindestens 5 Nukleotiden ist, die die HR1- und HR2-Regionen trennen, wobei die Oligomer-Insert-Nukleinsäure(n) so ausgelegt ist/sind, dass sie in der Lücke zwischen den Homologieregionen mit der Zielnukleinsäure hybridisieren, sodass die HR1- und HR2-Enden jeweils an das Oligomer stoßen.

2. Zusammensetzung nach Anspruch 1, wobei das Oligomer-Insert zu 100 % komplementär zu dem Allel ist, das am häufigsten in einer ausgewählten Population (Wildtyp) auftritt und das Oligomer-Insert zumindest eine Fehlpaarung an einem Nukleotid an der Stelle eines putativen SNPs oder Variantenallels aufweist und/oder wobei das Oligomer-Insert eine Länge im Bereich von 5 bis 15 Nukleotiden aufweist.

3. Zusammensetzung nach einem der Ansprüche 1 bis 2, wobei das Oligomer-Insert mit der Zielnukleinsäure an einem bis drei Nukleotiden eine Fehlpaarung aufweist.

4. Zusammensetzung nach einem der Ansprüche 1 bis 3, wobei die MIP zusätzliche Nukleinsäureregionen umfasst, ausgewählt aus der Gruppe bestehend aus: einem ID-Tag, einer ersten Spaltungsstelle, einer zweiten Spaltungsstelle, einer Vorwärts-Primerbindungsstelle (z. B. PCR) und einer Rückwärts-Primerbindungsstelle (z. B. PCR).

5. Zusammensetzung nach einem der Ansprüche 1 bis 4, wobei das Oligomer-Insert so ausgelegt ist, dass es mit dem Ziel in der Lücke mit einer Stringenz hybridisiert, die so angepasst ist, dass es bei Vorliegen einer Fehlpaarung nicht signifikant bindet.

6. Verfahren zur Identifizierung einer Zielsequenz von Interesse, wobei das
Verfahren Folgendes umfasst:
Bereitstellen einer Zusammensetzung nach einem der Ansprüche 1 bis 5;
Bereitstellen einer putativen Zielnukleinsäure, die eine Sequenz umfasst, die zu HR1 komplementär ist, und eine Sequenz umfasst, die zu HR2 komplementär ist, wobei die zu HR1 komplementäre Sequenz 5'-seitig angeordnet und mindestens 5 Basen von der zu HR2 komplementären Zielnukleinsäuresequenz getrennt ist;
Hybridisieren der MIP und des Oligomer-Inserts an die putative Zielnukleinsäure;
Inkontaktbringen der hybridisierten Nukleinsäuren mit einer Ligase;
Bestimmen, ob die MIP an das Oligomer-Insert ligiert wurde; und
Bestätigen, dass es sich bei der putativen Zielsequenz um die Zielsequenz von Interesse handelt, falls bestimmt wird, dass die MIP an das Oligomer-Insert ligiert ist.

7. Verfahren nach Anspruch 6, ferner umfassend: Bestimmen, dass die MIP nicht an das Oligomer-Insert ligiert wurde; wodurch bestätigt wird, dass die putative Zielsequenz mindestens eine Fehlpaarung mit dem Oligomer-Insert umfasst.

8. Verfahren nach Anspruch 6 oder 7, wobei die MIP eine erste Spaltungsstelle, eine Vorwärts-PCR-Primerbindungsstelle und eine Rückwärts-PCR-Primerbindungsstelle umfasst.

9. Verfahren nach Anspruch 8, wobei das Bestimmen der Ligation Folgendes umfasst:
Spaltung einer zirkularisierten MIP zwischen PCR-Primerbindungsstellen; und
Amplifikation der gespaltenen MIP durch PCR.

## Revendications

1. Composition comprenant :
une sonde d'inversion moléculaire (MIP) comprenant
une séquence d'une première région d'homologie (HR1) à une extrémité 5', dans laquelle la HR1 comprend une séquence complémentaire d'un acide nucléique cible ; et
une séquence d'une seconde région d'homologie 2 (HR2) à l'extrémité 3', dans laquelle la HR2 comprend une séquence complémentaire de l'acide nucléique cible, et dans laquelle une séquence cible complémentaire de la HR1 est située en 5' et séparée d'au moins 5 nucléotides d'une séquence cible complémentaire de la HR2 ; et,
un ou plusieurs acides nucléiques d'oligomère d'insertion, présentant une séquence au moins 80 % complémentaire des au moins 5 nucléotides séparant les régions HR1 et
HR2, dans laquelle les un ou plusieurs acides nucléiques d'oligomère d'insertion sont conçus pour s'hybrider dans l'intervalle entre les régions d'homologie et l'acide nucléique cible, de telle sorte que les extrémités des HR1 et HR2 jouxtent ainsi l'oligomère.

2. Composition selon la revendication 1, dans laquelle l'oligomère d'insertion est 100 % complémentaire de l'allèle apparaissant le plus fréquemment dans une population sélectionnée (type sauvage), et l'oligomère d'insertion présente au moins un mésappariement nucléotidique à la position d'un SNP putatif ou d'un allèle variant, et/ou dans laquelle la longueur de l'oligomère d'insertion varie de 5 à 15 nucléotides.

3. Composition comprenant l'une quelconque des revendications 1 à 2, dans laquelle l'oligomère d'insertion présente un mésappariement par rapport à l'acide nucléique cible de un à trois nucléotides.

4. Composition selon la revendication 1 à 3, dans laquelle la MIP comprend des régions d'acide nucléique supplémentaires choisies dans le groupe constitué par : un identifiant de marqueur, un premier site de clivage, un second site de clivage, un site de liaison pour l'amorce sens (par exemple, pour la PCR) et un site de liaison pour l'amorce antisens (par exemple, pour la PCR).

5. Composition selon les revendications 1 à 4, dans laquelle l'oligomère d'insertion est conçu pour s'hybrider avec la cible dans l'intervalle, avec une stringence adaptée de manière à ne pas se lier de manière significative en présence d'un mésappariement.

6. Procédé d'identification d'une séquence cible d'intérêt, ledit procédé comprenant les étapes consistant à :
fournir une composition selon l'une quelconque des revendications 1 à 5 ;
fournir un acide nucléique cible putatif comprenant une séquence complémentaire de HR1 et une séquence complémentaire de HR2, la séquence complémentaire de HR1 étant située en 5' et séparée d'au moins 5 bases de la séquence d'acide nucléique cible complémentaire de HR2 ;
hybrider la MIP et l'oligomère d'insertion à l'acide nucléique cible putatif ;
mettre en contact les acides nucléiques hybridés avec une ligase ;
déterminer si la MIP a été ligaturée à l'oligomère d'insertion ; et,
confirmer que la séquence cible putative est la séquence cible d'intérêt s'il est déterminé que la MIP est ligaturée à l'oligomère d'insertion.

7. Procédé selon la revendication 6, comprenant en outre la détermination que la MIP n'a pas été ligaturée à l'oligomère d'insertion ; en confirmant ainsi que la séquence cible putative comporte au moins un mésappariement avec l'oligomère d'insertion.

8. Procédés selon les revendications 6 ou 7, dans lesquels la MIP comprend un premier site de clivage, un site de liaison pour l'amorce de PCR sens et un site de liaison pour l'amorce de PCR antisens.

9. Procédé selon la revendication 8, dans lequel ladite étape de détermination de la ligature comprend les étapes consistant à :
cliver une MIP circularisée entre les sites de liaison d'amorce de PCR ; et
amplifier par PCR la MIP clivée.
